# EUROPÄISCHE PATENTSCHRIFT

(11) **EP 0 497 127 B1**
(45) Veröffentlichungstag und Bekanntmachung des Hinweises auf die Patenterteilung: **19.06.1996**
(21) Anmeldenummer: 92100419.8
(22) Anmeldetag: 13.01.1992
(51) Int. Cl.: C07D 207/38, A01N 43/40, A01N 43/36

(54) **Substituierte 3-Aryl-pyrrolidin-2,4-dione**
Substituted 3-aryl-pyrrolidine-2,4-diones
3-Aryl-pyrrolidine-2,4-diones substitués

(30) Priorität: 26.01.1991 DE 4102339
(43) Veröffentlichungstag der Anmeldung: 05.08.1992
(73) Patentinhaber: BAYER AG, 51368 Leverkusen (DE)
(72) Erfinder: Fischer, Reiner, Dr., W-4019 Monheim 2 (DE); Uhr, Hermann, Dr., W-5090 Leverkusen 3 (DE); Widdig, Arno, Dr., W-5068 Odenthal-Blecher (DE); Dutzmann, Stefan, Dr., W-4010 Hilden 1 (DE); Erdelen, Christoph, Dr., W-5653 Leichlingen 1 (DE); Wachendorff-Neumann, Ulrike, Dr., W-4019 Monheim (DE); Schaller, Klaus, Dr., W-5600 Wuppertal 1 (DE)

(56) Entgegenhaltungen:
- EP-A- 0 377 893
- EP-A- 0 442 077
- EP-A- 0 456 063
- US-A- 4 551 547

## Beschreibung

Die Erfindung betrifft neue substituierte 3-Aryl-pyrrolidin-2,4-dione, mehrere Verfahren zu ihrer Herstellung und ihre Verwendung als Insektizide, Akarizide, Fungizide und Antimykotika.

Von 3-Acyl-pyrrolidin-2,4-dionen sind pharmazeutische Eigenschaften vorbeschrieben (S. Suzuki et. al. Chem. Pharm. Bull. 15 1120 (1967)). Weiterhin wurden N-Phenyl-pyrrolidin-2,4-dione von R. Schmierer und H. Mildenberger Liebigs Ann. Chem. 1985 1095 synthetisiert. Eine biologische Wirksamkeit dieser Verbindungen wurde nicht beschrieben.

In EP-A 0 262 399 werden ähnlich strukturierte Verbindungen (3-Aryl-pyrrolidin-2,4-dione) offenbart, von denen jedoch keine insektizide oder akarizide Wirkung bekannt geworden ist.

Es wurden nun neue substituierte 3-Aryl-pyrrolidin-2,4-dione gefunden, die durch die Formel (I) dargestellt sind, 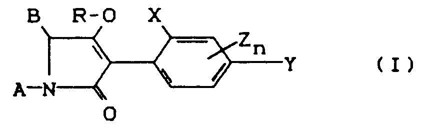
in welcher
- X: für Wasserstoff, Alkyl, Halogen, Alkoxy steht,
- Y: für Wasserstoff, Alkyl, Halogen, Alkoxy, Halogenalkyl steht,
- Z: für Alkyl, Halogen, Alkoxy steht,
- n: für eine Zahl von 0-3 steht,
- R: für Wasserstoff oder für die Gruppen
-CO-R¹, -CO-O-R²
steht, in welchen
- R¹: für gegebenenfalls durch Halogen substituiertes Alkyl, Alkenyl, Alkoxyalkyl, Alkylthioalkyl, Polyalkoxyalkyl und Cycloalkyl, das durch Heteroatome unterbrochen sein kann, gegebenenfalls subst. Phenyl, gegebenenfalls substituiertes Phenylalkyl, substituiertes Hetaryl, substituiertes Phenoxyalkyl und substituiertes Hetaryloxyalkyl steht und
- R²: für gegebenenfalls durch Halogen substituiertes Alkyl, Alkenyl, Alkoxyalkyl, Polyalkoxyalkyl und gegebenenfalls substituiertes Phenyl steht,
- A: für gegebenenfalls durch Halogen substituiertes Alkyl, Alkenyl, Alkinyl, Alkoxyalkyl, Polyalkoxyalkyl, Alkylthioalkyl, gegebenenfalls durch Heteroatome unterbrochenes Cycloalkyl oder gegebenenfalls durch Halogen-, Alkyl-, Halogenalkyl-, Alkoxy-, Nitro substituiertes Arylalkyl steht,
- B: für gegebenenfalls substituiertes Aryl oder gegebenenfalls substituiertes Benzyl steht
sowie die enantiomerenreinen Formen von Verbindungen der Formel (I).

Im folgenden seien die folgenden Untergruppen definiert:
- (Ia):: Verbindungen der Formel (I) worin R = Wasserstoff,
- (Ib):: Verbindungen der Formel (I) worin R = COR¹,
- (Ic):: Verbindungen der Formel (I) worin R = COOR².

Weiterhin wurde gefunden, daß man die neuen substituierten 3-Aryl-pyrrolidin-2,4-dione bzw. deren Enole der Formel (Ia) 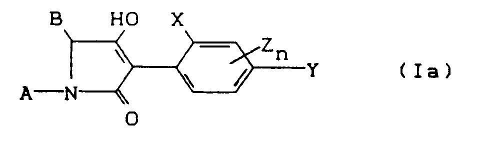
in welcher A, B, X, Y, Z und n die oben angegebene Bedeutung haben,
erhält, wenn man
(A)
   N-Acylaminosäureester der Formel (II) 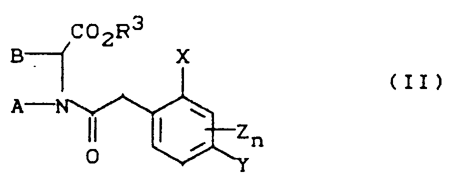 in welcher
   A, B, X, Y, Z und n die oben angegebene Bedeutung haben
   und
   - R³: für Alkyl steht,
   in Gegenwart eines Verdünnungsmittels und in Gegenwart einer Base intramolekular kondensiert.
(B)
   Außerdem wurde gefunden, daß man Verbindungen der Formel (Ib) 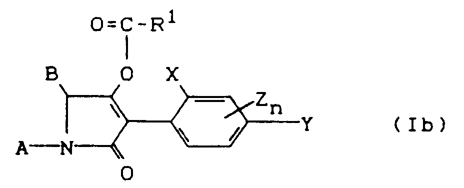 in welcher A, B, X, Y, Z, R¹ und n die oben angegebene Bedeutung haben,
   erhält, wenn man Verbindungen der Formel (Ia), 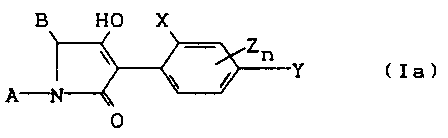 in welcher
   A, B, X, Y, Z und n die oben angegebene Bedeutung haben,
   - α): mit Säurehalogeniden der allgemeinen Formel (III) 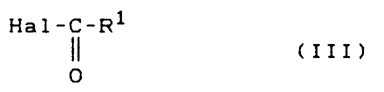 in welcher
   - R¹: die oben angegebene Bedeutung hat
   und
   - Hal: für Halogen, insbesondere Chlor und Brom steht,
   gegebenenfalls in Gegenwart eines Verdünnungsmittels und gegebenenfalls in Gegenwart eines Säurebindemittels,
   oder
   - β): mit Carbonsäureanhydriden der allgemeinen Formel (IV)

   R¹-CO-O-CO-R¹ (IV)

   in welcher
   - R¹: die oben angegebene Bedeutung hat,
   gegebenenfalls in Gegenwart eines Verdünnungsmittels und gegebenenfalls in Gegenwart eines Säurebindemittels,
   umsetzt.
(C)
   Ferner wurde gefunden, daß man Verbindungen der Formel (Ic) 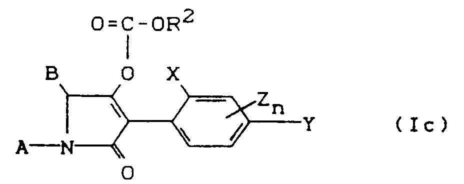 in welcher
   A, B, X, Y, Z, R² und n die oben angegebene Bedeutung haben,
   erhält, wenn man Verbindungen der Formel (Ia) 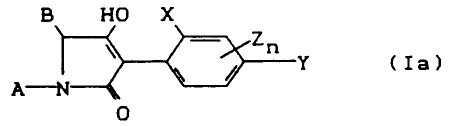 in welcher
   A, B, X, Y, Z und n die oben angegebene Bedeutung haben,
   mit Chlorameisensäureester der allgemeinen Formel (V)

   R²-O-CO-Cl (V)

   in welcher
   - R²: die oben angegebene Bedeutung hat,
   gegebenenfals in Gegenwart eines Verdünnungsmittels und gegebenenfalls in Gegenwart eines Säurebindemittels umsetzt.

Überraschenderweise wurde gefunden, daß die neuen substituierten 3-Arylpyrrolidin-2,4-dione der Formel (I) sich durch sehr gute insektizide, akarizide, fungizide und antimykotische Wirkungen auszeichnen.

Bevorzugt sind diejenigen substituierten 3-Aryl-pyrrolidin-2,4-dione der Formel (I), in welcher
- X: für Wasserstoff, C₁-C₆-Alkyl, Halogen, C₁-C₆-Alkoxy steht,
- Y: für Wasserstoff, C₁-C₆-Alkyl, Halogen, C₁-C₆-Alkoxy, C₁-C₃-Halogenalkyl steht,
- Z: für C₁-C₆-Alkyl, Halogen, C₁-C₆-Alkoxy steht,
- n: für eine Zahl von 0-3 steht,
- R: für Wasserstoff (Ia) oder für die Gruppen der Formel

-CO-R¹ (Ib) oder

-CO-O-R² (Ic)

steht, in welchen
- R¹: für gegebenenfalls durch Halogen substituiertes: C₁-C₂₀-Alkyl, C₂-C₂₀-Alkenyl, C₁-C₈-Alkoxy-C₂-C₈-alkyl, C₁-C₈-Alkylthio-C₂-C₈-alkyl, C₁-C₈-Polyalkoxy-C₂-C₈-alkyl und Cycloalkyl mit 3-8 Ringatomen, das durch Sauerstoff und/oder Schwefel unterbrochen sein kann, steht,
für gegebenenfalls durch Halogen-, Nitro-, C₁-C₆-Alkyl-, C₁-C₆-Alkoxy-, C₁-C₆-Halogenalkyl-, C₁-C₆-Halogenalkoxy-substituiertes Phenyl;
für gegebenenfalls durch Halogen-, C₁-C₆-Alkyl, C₁-C₆-Alkoxy-, C₁-C₆-Halogenalkyl-, C₁-C₆-Halogenalkoxy-substituiertes Phenyl-C₁-C₆-alkyl steht,
für gegebenenfalls durch Halogen- und C₁-C₆-Alkyl-substituiertes Hetaryl steht,
für gegebenenfalls durch Halogen- und C₁-C₆-Alkyl-substituiertes Phenoxy-C₁-C₆-alkyl steht,
für gegebenenfalls durch Halogen, Amino und C₁-C₆-Alkyl-substituiertes Hetaryloxy-C₁-C₆-Alkyl steht,
- R²: für gegebenenfalls durch Halogen substituiertes: C₁-C₂₀-Alkyl, C₂-C₂₀-Alkenyl, C₁-C₈-Alkoxy-C₂-C₈-alkyl, C₁-C₈-Polyalkoxy-C₂-C₈-alkyl steht,
für gegebenenfalls durch Halogen-, Nitro-, C₁-C₆-Alkyl-, C₁-C₆-Alkoxy-, C₁-C₆-Halogenalkyl-substituiertes Phenyl steht,
- A: für gegebenenfalls durch Halogen substituiertes geradkettiges oder verzweigtes C₁-C₁₂-Alkyl, C₃-C₈-Alkenyl, C₃-C₈-Alkinyl, C₁-C₁₀-Alkoxy-C₂-C₈-alkyl, C₁-C₈-Polyalkoxy-C₂-C₈-alkyl, C₁-C₁₀-Alkylthio-C₂-C₈-alkyl, Cycloalkyl mit 3-8 Ringatomen, das durch Sauerstoff und/oder Schwefel unterbrochen sein kann oder gegebenenfalls durch Halogen, C₁-C₆-Alkyl-C₁-C₆-Haloalkyl-, C₁-C₆-Alkoxy-, Nitro substituiertes Aryl-C₁-C₆-alkyl steht,
- B: für gegebenenfalls einfach oder dreifach, gleich oder verschieden durch Nitro, Halogen oder jeweils für gegebenenfalls durch Halogen substituiertes Alkyl oder Alkoxy substituiertes Phenyl oder Benzyl steht
sowie die enantiomerenreinen Formen von Verbindungen der Formel (I).

Besonders bevorzugt sind Verbindungen der Formel (I) in welcher
- X: für Wasserstoff, C₁-C₄-Alkyl, Halogen, C₁-C₄-Alkoxy steht,
- Y: für Wasserstoff, C₁-C₆-Alkyl, Halogen, C₁-C₄-Alkoxy, C₁-C₂-Halogenalkyl steht,
- Z: für C₁-C₄-Alkyl, Halogen, C₁-C₄-Alkoxy steht,
- n: für eine Zahl von 0-3 steht,
- R: für Wasserstoff (Ia) oder für die Gruppen der Formel

-CO-R¹ (Ib) oder

-CO-O-R² (Ic)

steht, in welchen
- R¹: für gegebenenfalls durch Halogen substituiertes: C₁-C₁₆-Alkyl, C₂-C₁₆-Alkenyl, C₁-C₆-Alkoxy-C₂-C₆-alkyl, C₁-C₆-Alkylthio-C₂-C₆-alkyl, C₁-C₆-Polyalkoxy-C₂-C₆-alkyl und Cycloalkyl mit 3-7 Ringatomen, das durch 1-2 Sauerstoff- und/oder Schwefelatome unterbrochen sein kann steht,
für gegebenenfalls durch Halogen-, Nitro-, C₁-C₄-Alkyl-, C₁-C₄-Alkoxy-, C₁-C₃-Halogenalkyl-, C₁-C₃-Halogenalkoxy-substituiertes Phenyl steht,
für gegebenenfalls durch Halogen-, C₁-C₄-Alkyl-, C₁-C₄-Alkoxy-, C₁-C₃-Halogenalkyl-, C₁-C₃-Halogenalxoxy-substituiertes Phenyl-C₁-C₄-alkyl steht,
für gegebenenfalls duch Halogen- und C₁-C₆-Alkyl-substituiertes Hetaryl steht,
gegebenenfalls für durch Halogen- und C₁-C₄-Alkyl-substituiertes Phenoxy-C₁-C₅-alkyl steht,
für gegebenfalls durch Halogen, Amino und C₁-C₄-Alkyl-substituiertes Hetaryloxy-C₁-C₅-alkyl steht,
- R²: für gegebenenfalls durch Halogen substituiertes: C₁-C₁₆-Alkyl, C₂-C₁₆-Alkenyl, C₁-C₁₆-Alkoxy-C₂-C₆-alkyl, C₁-C₆-Polyalkoxy-C₂-C₆-alkyl steht,
für gegebenenfalls durch Halogen, Nitro-, C₁-C₄-Alkyl, C₁-C₃-Alkoxy-, C₁-C₃-Halogenalkyl-substituiertes Phenyl steht,
- A: für gegebenenfalls durch Halogen substituiertes geradkettiges oder verzweigtes C₁-C₁₀-Alkyl, C₃-C₆-Alkenyl, C₃-C₆-Alkinyl, C₁-C₈-Alkoxy-C₂-C₆-alkyl, C₁-C₆-Polyalkoxy-C₂-C₆-alkyl, C₁-C₈-Alkylthio-C₂-C₆-alkyl, Cycloalkyl mit 3-7 Ringatomen, das durch 1-2 Sauerstoff- und/oder Schwefelatomen unterbrochen sein kann oder gegebenenfalls durch Halogen-, C₁-C₄-Alkyl-, C₁-C₄-Haloalkyl-C₁-C₄-Alkoxy-, Nitro substituiertes Aryl-C₁-C₄-alkyl steht,
- B: für gegebenenfalls einfach oder zweifach, gleich oder verschieden durch Nitro, Fluor, Chlor, Brom, gegebenenfalls durch Fluor oder Chlor substituiertes C₁-C₄-Alkyl oder C₁-C₄-Alkoxy substituiertes Phenyl oder Benzyl steht
sowie die enantiomerenreinen Formen von Verbindungen der Formel (I).

Ganz besonders bevorzugt sind Verbindungen der Formel (I) in welcher
- X: für Wasserstoff, Methyl, Ethyl, Propyl, i-Propyl, Fluor, Chlor, Brom, Methoxy und Ethoxy steht,
- Y: für Wasserstoff, Methyl, Ethyl, Propyl, i-Propyl, Butyl, i-Butyl, tert.-Butyl, Fluor, Chlor, Brom, Methoxy, Ethoxy und Trifluormethyl steht,
- Z: für Methyl, Ethyl, i-Propyl, Butyl, i-Butyl, tert.-Butyl, Fluor, Chlor, Brom, Methoxy und Ethoxy steht,
- n: für eine Zahl von 0-3 steht,
- R: für Wasserstoff (Ia) oder für die Gruppen der Formel

-CO-R¹ (Ib) oder

-CO-O-R² (Ic)

steht, in welcher
- R¹: für gegebenenfalls durch Fluor oder Chlor substituiertes: C₁-C₁₄-Alkyl, C₂-C₁₄-Alkenyl, C₁-C₄-Alkoxy-C₂-C₆-alkyl, C₁-C₄-Alkylthio-C₂-C₆-alkyl, C₁-C₄-Polyalkoxyl-C₂-C₄-alkyl und Cycloalkyl mit 3-6 Ringatomen, das durch 1-2 Sauerstoff- und/oder Schwefelatome unterbrochen sein kann steht,
für gegebenenfalls durch Fluor-, Chlor, Brom-, Methyl-, Ethyl-, Propyl, i-Propyl, Methoxy, Ethoxy, Trifluormethyl-, Trifluormethoxy-, Nitro- substituiertes Phenyl steht,
für gegebenenfalls durch Fluor-, Chlor-, Brom-, Methyl-, Ethyl-, Propyl-, i-Propyl, Methoxy, Ethoxy, Trifluormethyl, Trifluormethoxy- substituiertes Phenyl-C₁-C₃-alkyl steht,
für gegebenenfalls durch Fluor-, Chlor-, Brom-, Methyl-, Ethyl-substituiertes Pyridyl, Pyrimidyl, Thiazolyl und Pyrazolyl steht,
für gegebenenfalls durch Fluor-, Chlor-, Methyl-, Ethyl-substituiertes Phenoxy-C₁-C₄-alkyl steht,
für gegebenenfalls durch Fluor-, Chlor-, Amino-, Methyl-, Ethyl-, substituiertes Pyridyloxy-C₁-C₄-alkyl, Pyrimidyloxy-C₁-C₄-alkyl und Thiazolyloxy-C₁-C₄-alkyl steht,
- R²: für gegebenenfalls durch Fluor oder Chlor substituiertes C₁-C₁₄-Alkyl, C₂-C₁₄-Alkenyl, C₁-C₄-Alkoxy-C₂-C₆-alkyl, C₁-C₄-Polyalkoxy-C₂-C₆-alkyl steht,
oder für gegebenenfalls durch Fluor, Chlor, Nitro, Methyl, Ethyl, Propyl, i-Propyl, Methoxy, Ethoxy, Trifluormethyl substituiertes Phenyl steht,
- A: für gegebenenfalls durch Halogen substituiertes geradkettiges oder verzweigtes C₁-C₈-Alkyl, C₃-C₄-Alkenyl, C₃-C₄-Alkinyl, C₁-C₆-Alkoxy-C₂-C₄-alkyl, C₁-C₄-Polyalkoxy-C₂-C₄-alkyl, C₁-C₆-Alkylthio-C₂-C₄-alkyl, Cycloalkyl mit 3-6 Ringatomen, das durch 1-2 Sauerstoff- und/oder Schwefelatomen unterbrochen sein kann oder gegebenenfalls durch Fluor-, Chlor-, Brom-, Methyl-, Ethyl-, Propyl-, iso-Propyl-, Methoxy-, Ethoxy-, Trifluormethyl-, Nitro susbtituiertes Aryl-C₁-C₃-alkyl steht,
- B: für unsubstituiertes Phenyl oder Benzyl oder für durch Nitro, Fluor, Chlor, gegebenenfalls durch Fluor oder Chlor substituiertes C₁-C₂-Alkyl oder C₁-C₂-Alkoxy substituiertes Phenyl oder Benzyl steht
sowie die enantiomerenreinen Formen von Verbindungen der Formel I.

Im einzelnen seien außer den bei den Herstellungsbeispielen genannten Verbindungen die folgenden substituierten 3-Aryl-pyrrolidin-2,4-dione der allgemeinen Formel (I) genannt:

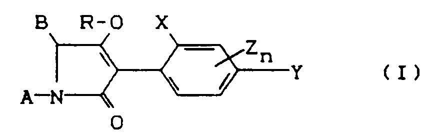

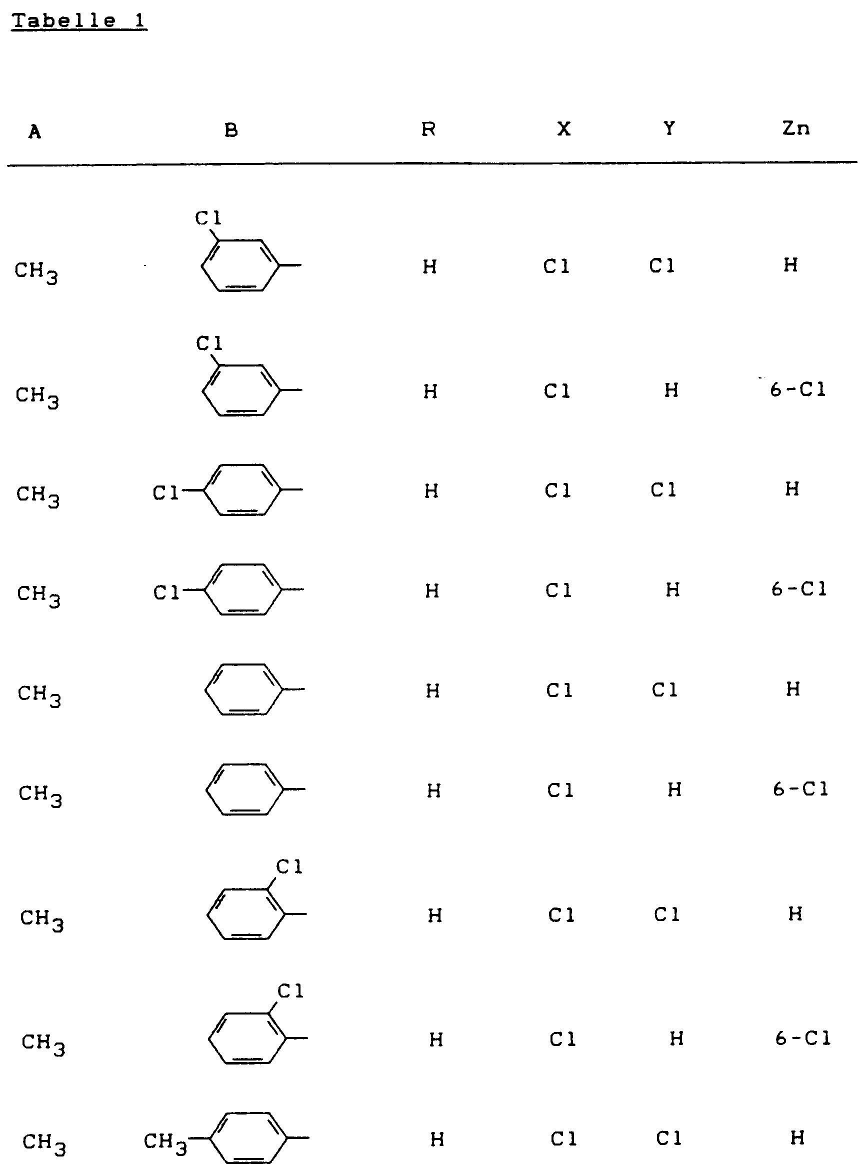

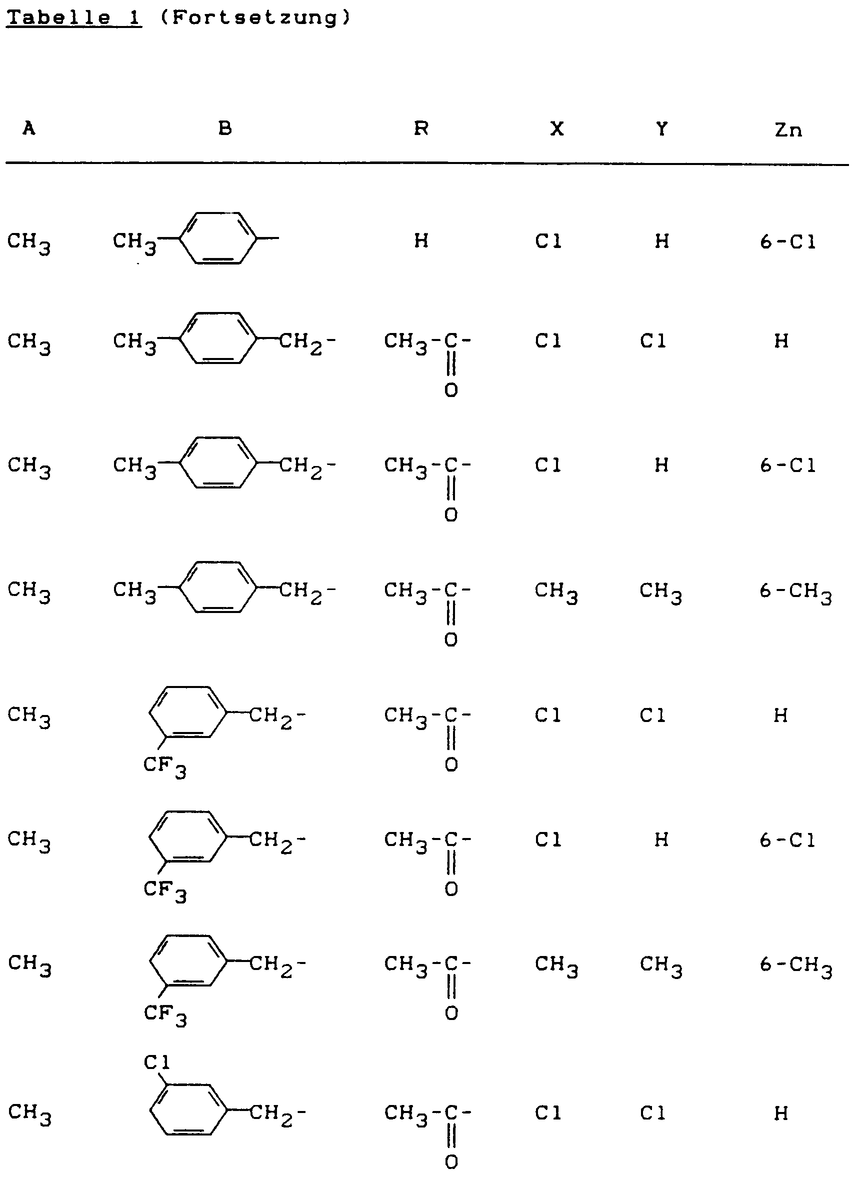

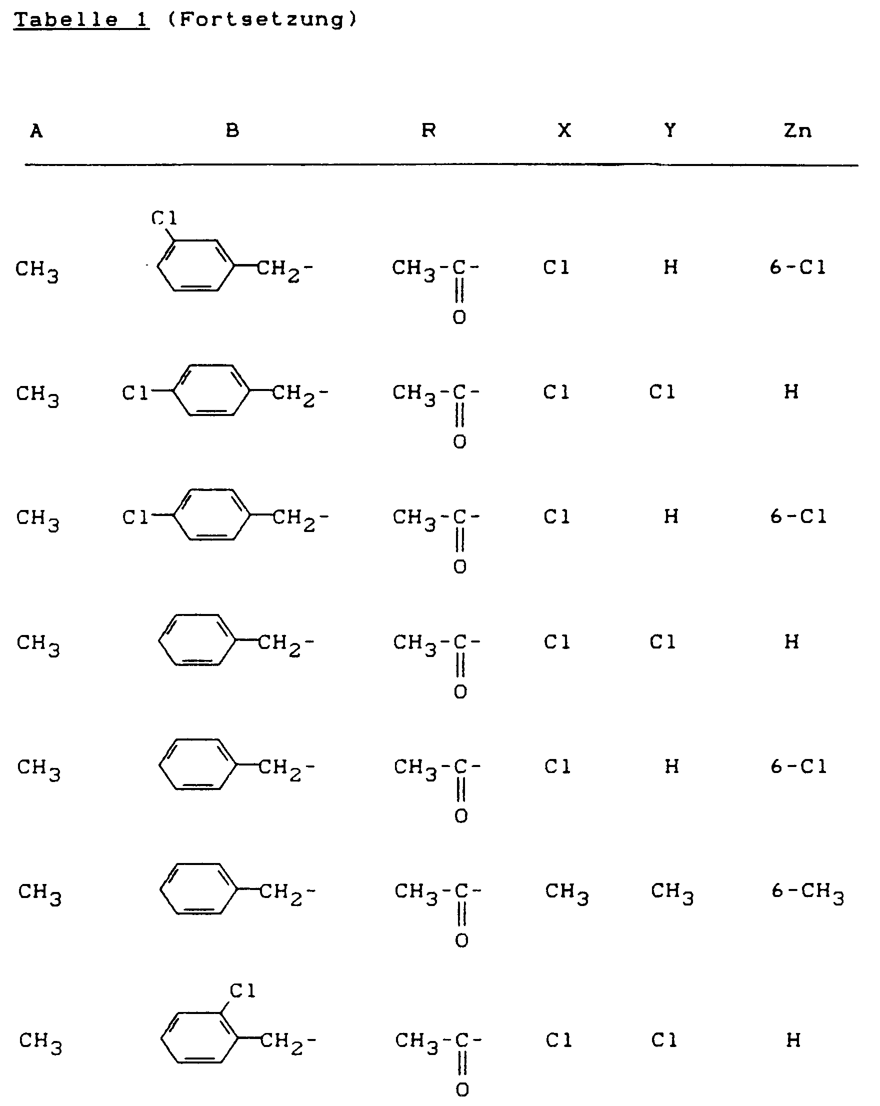

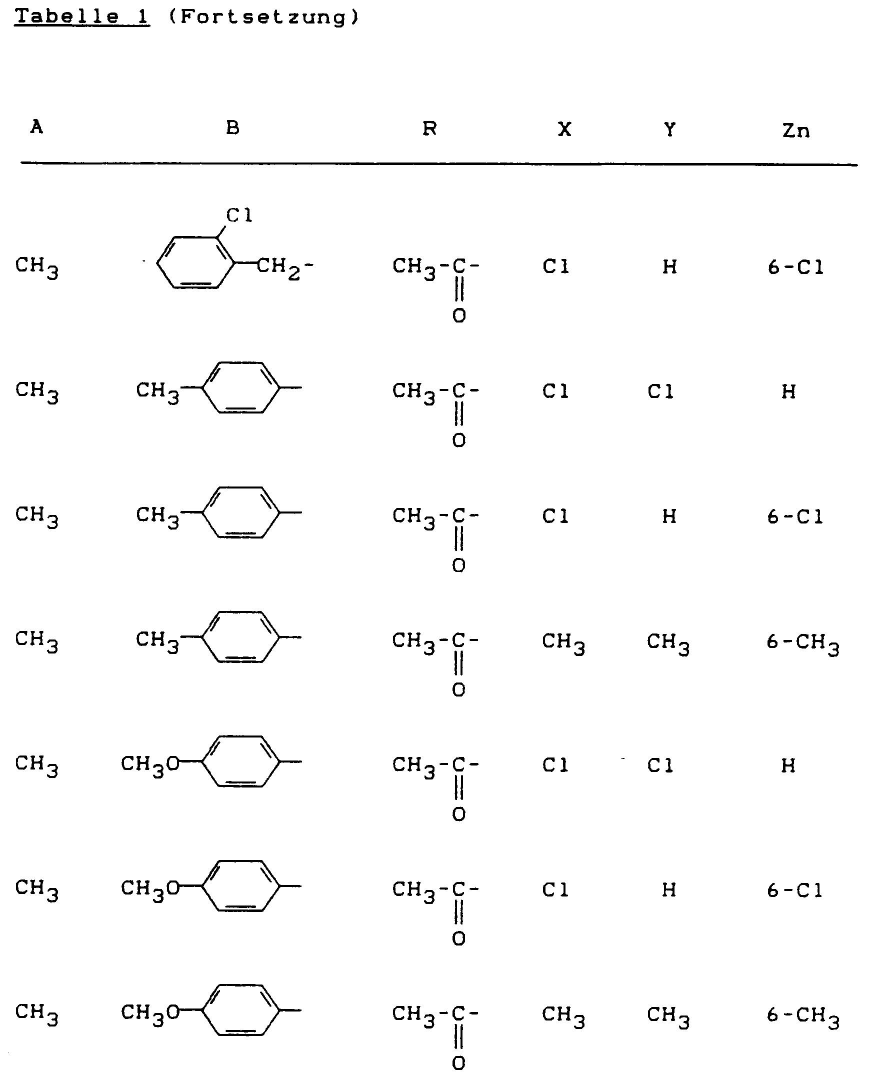

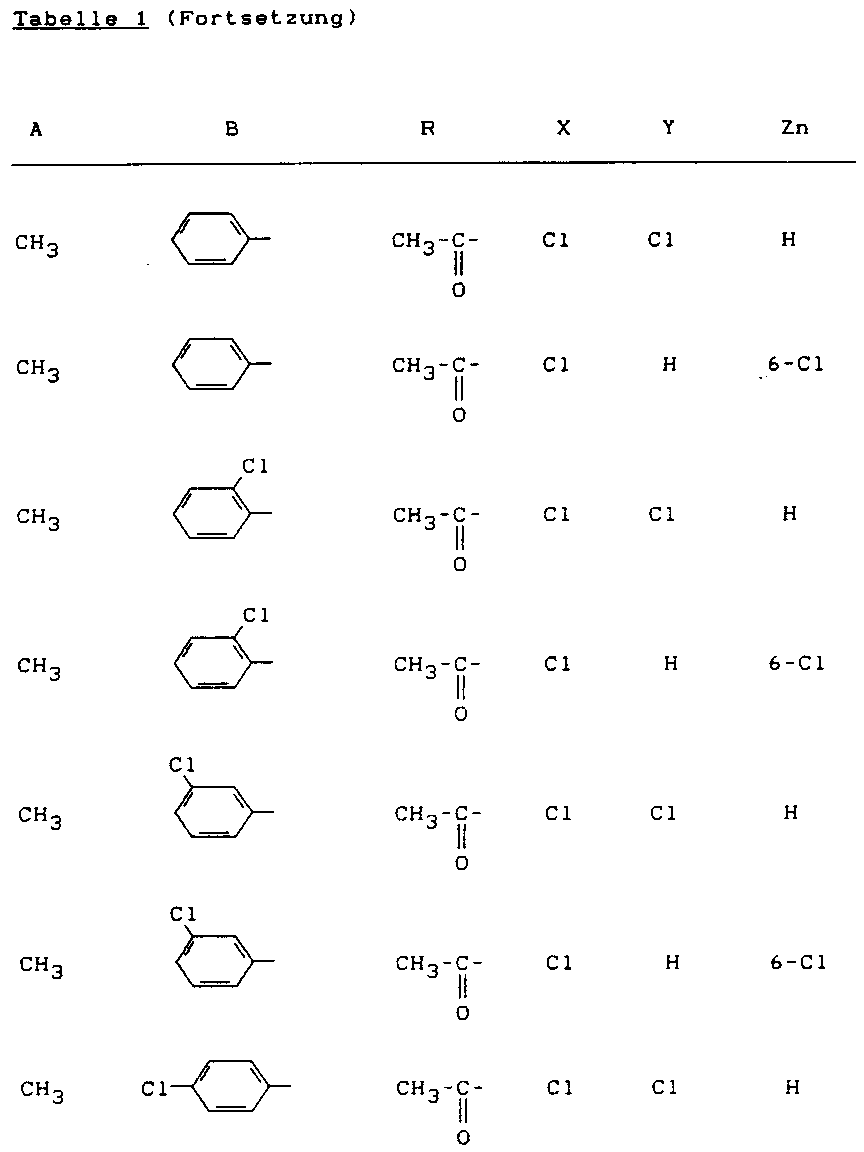

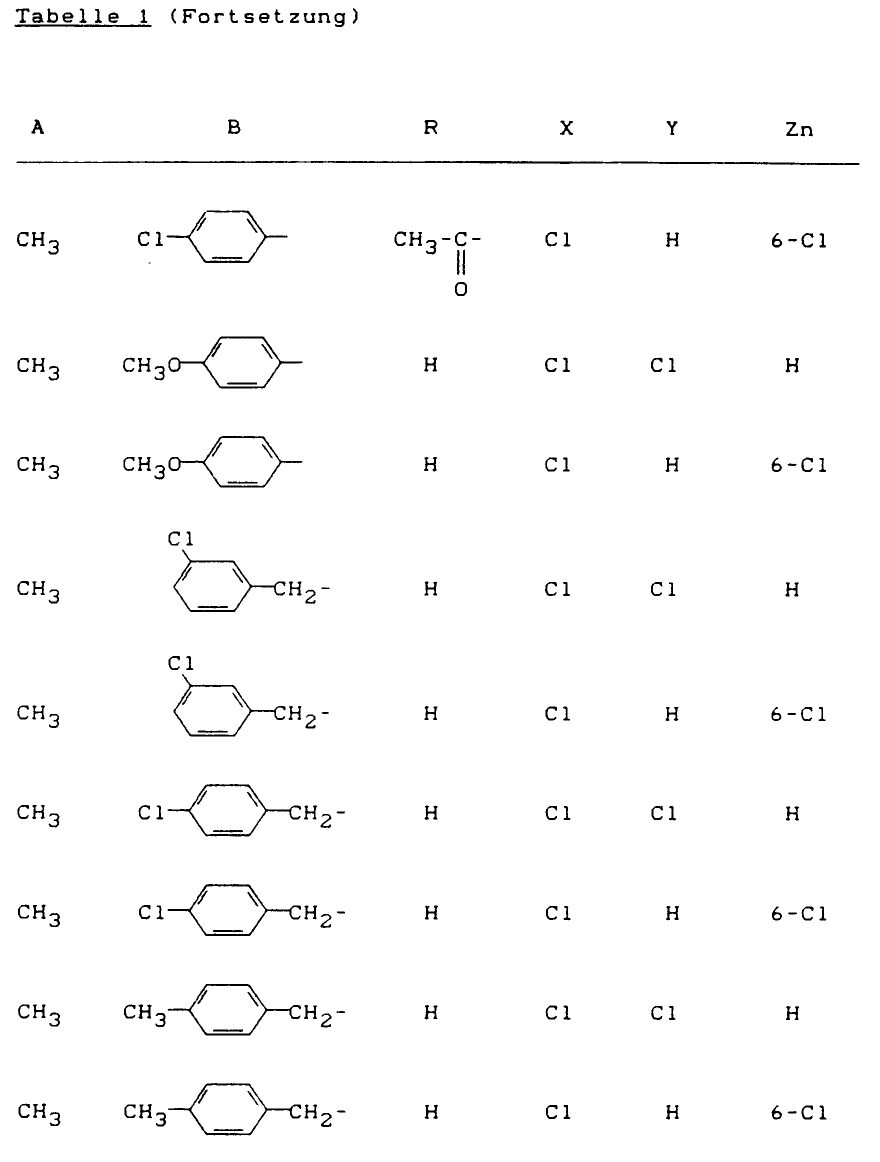

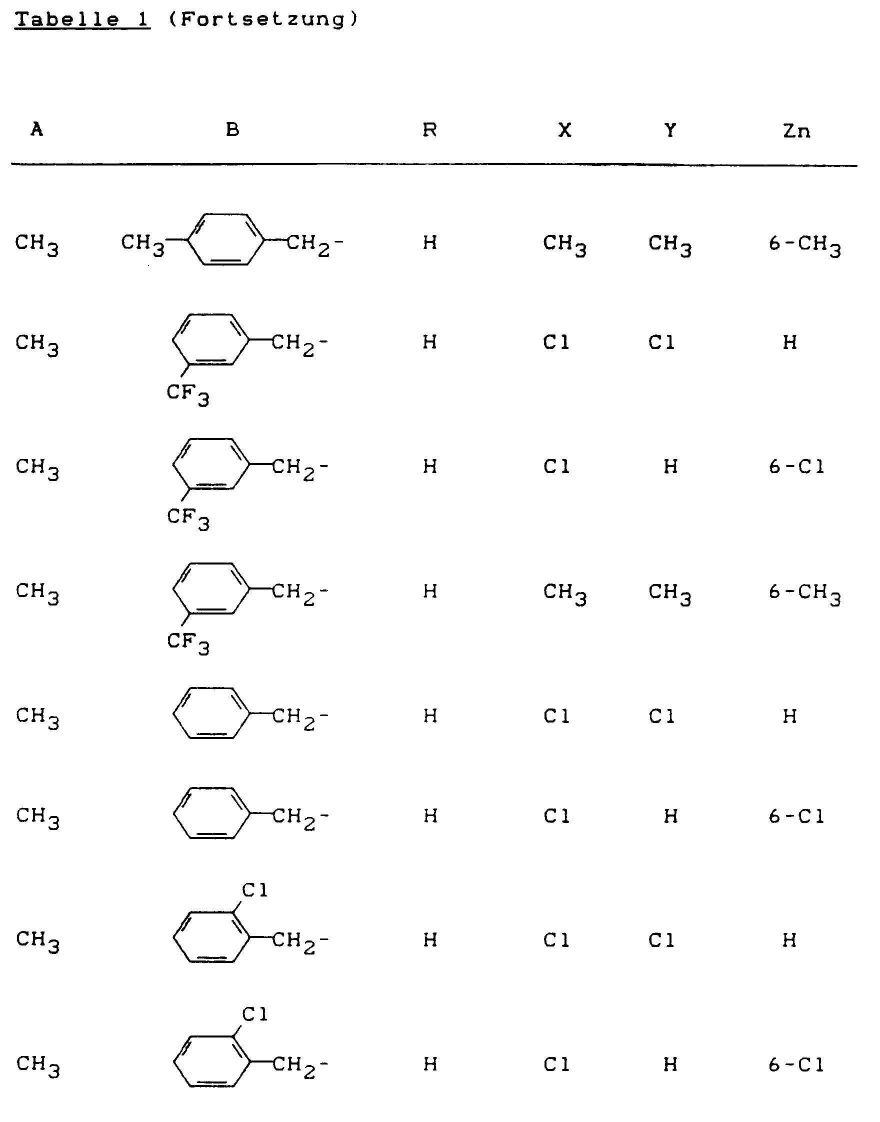

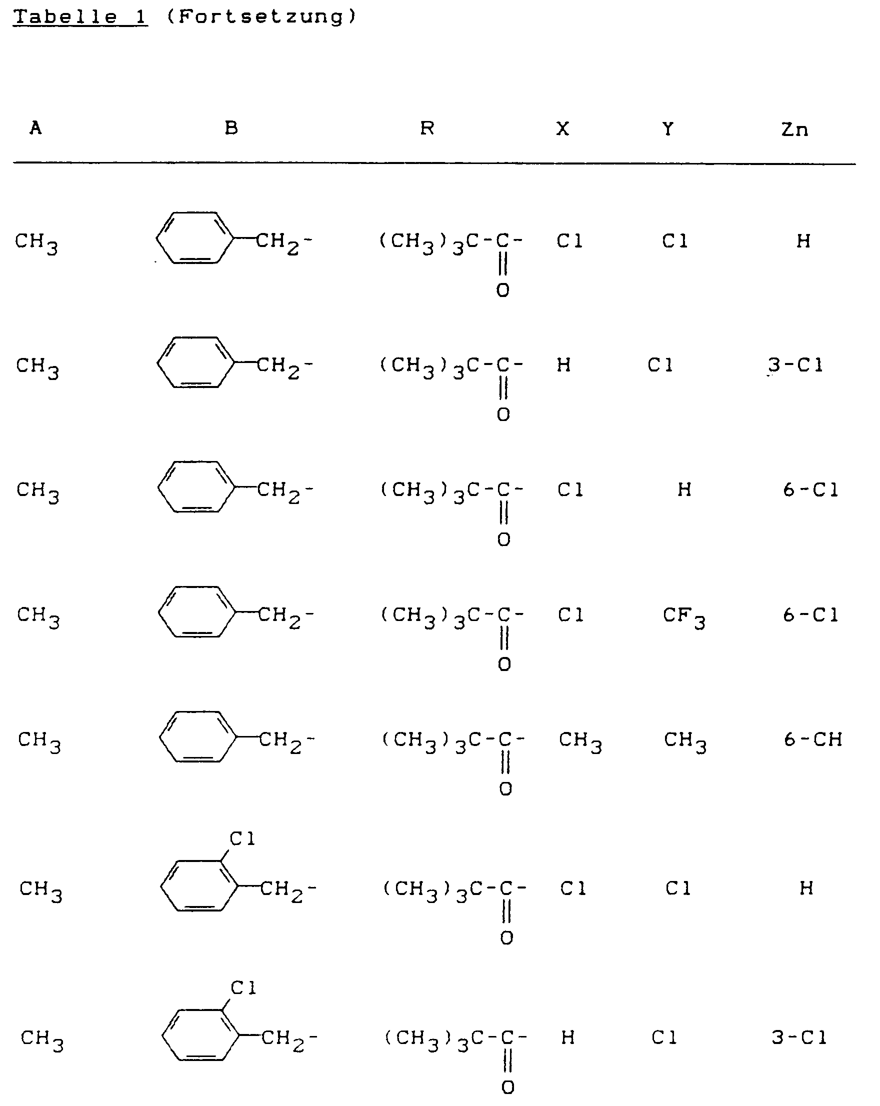

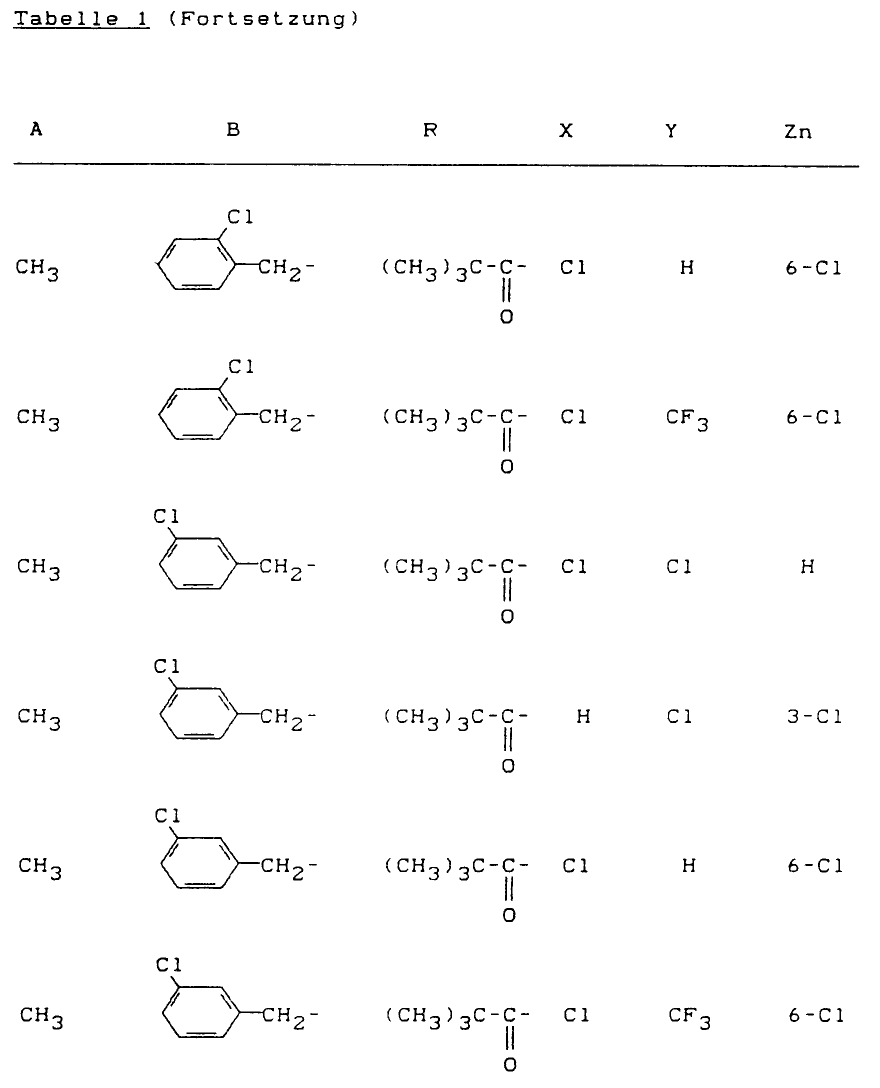

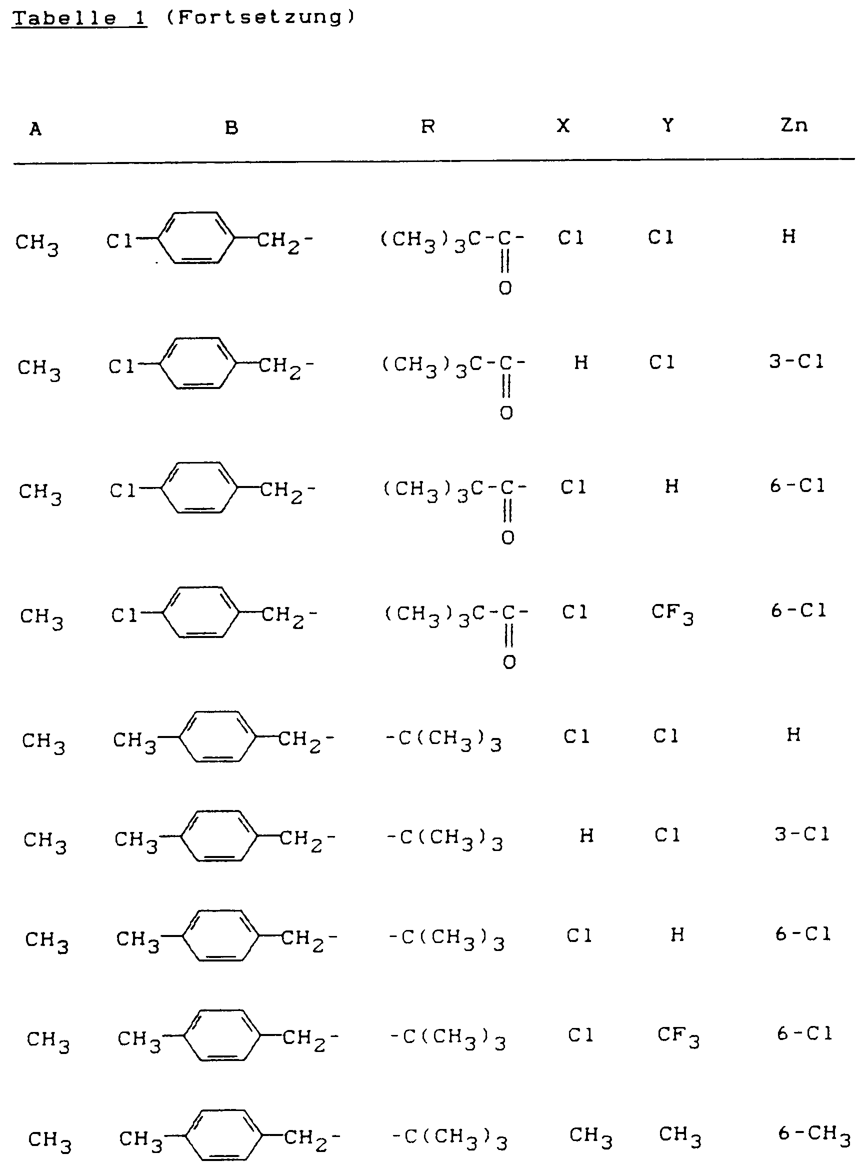

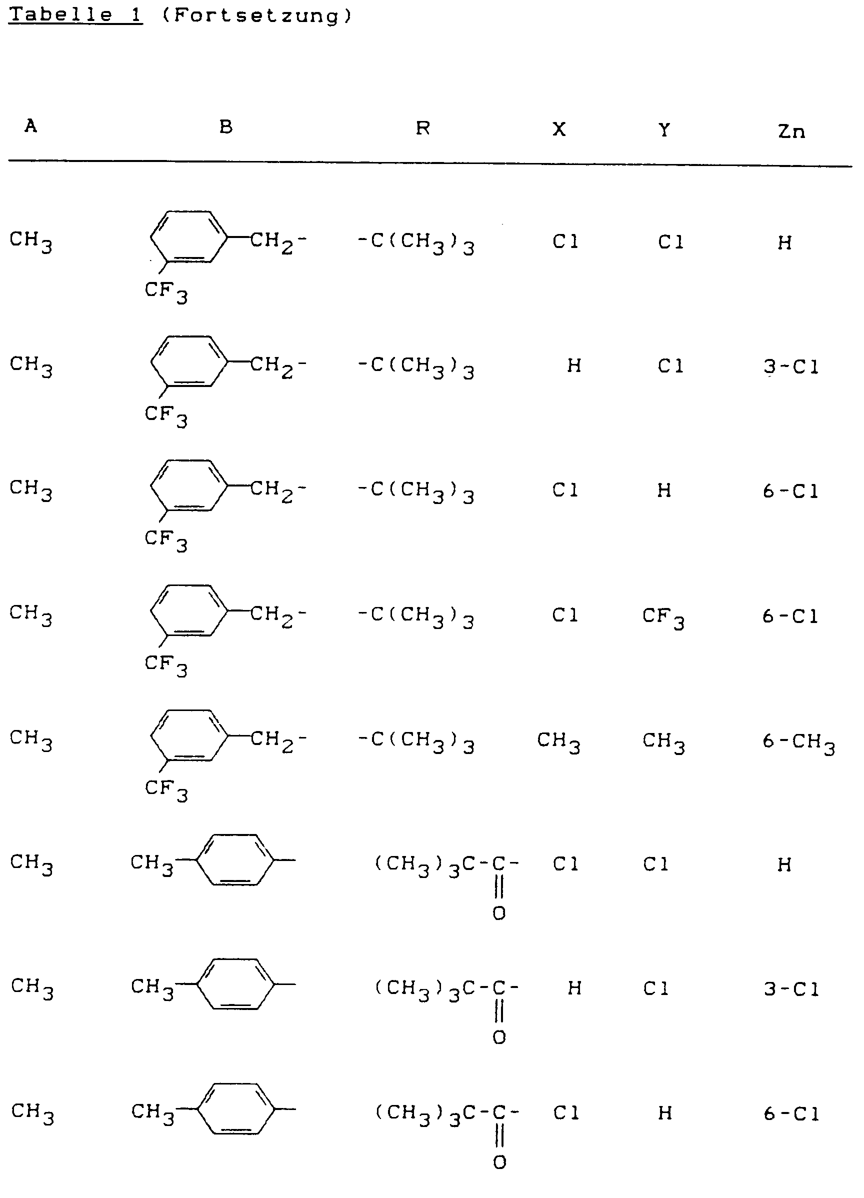

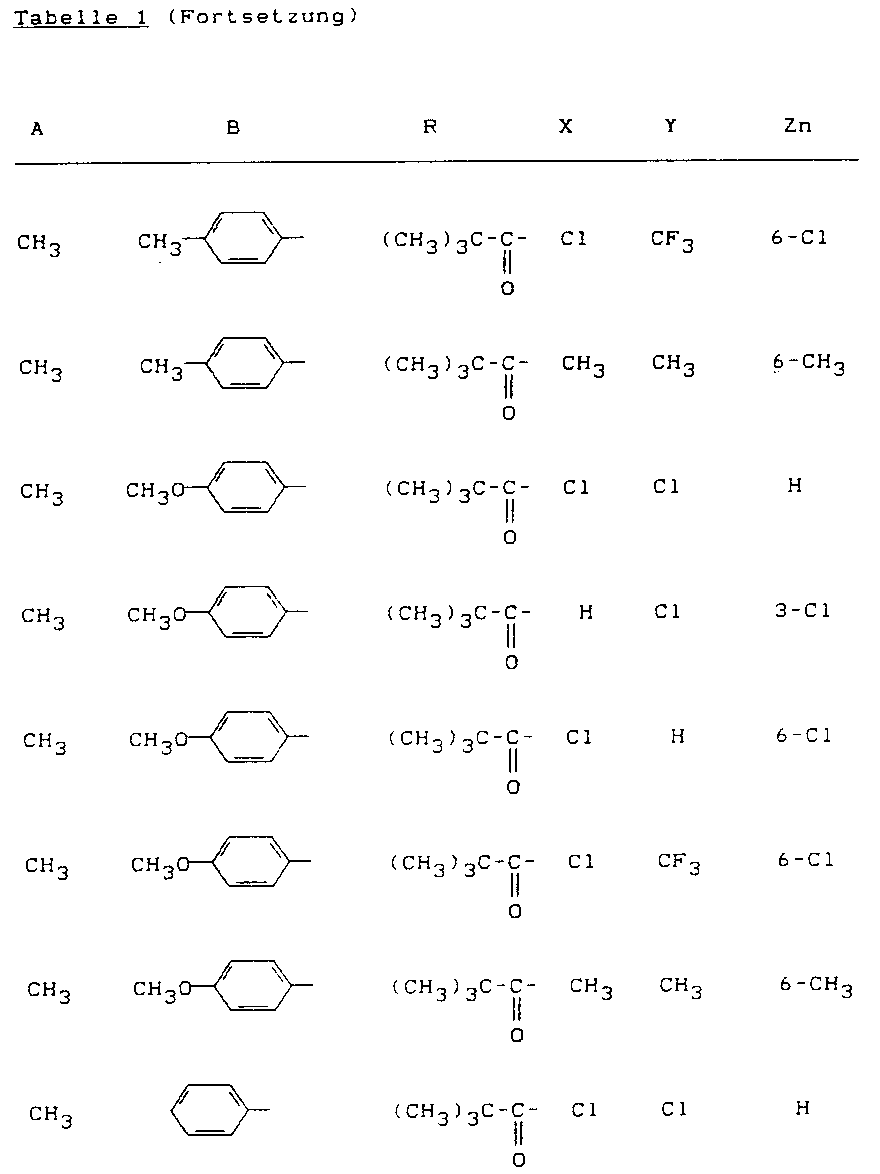

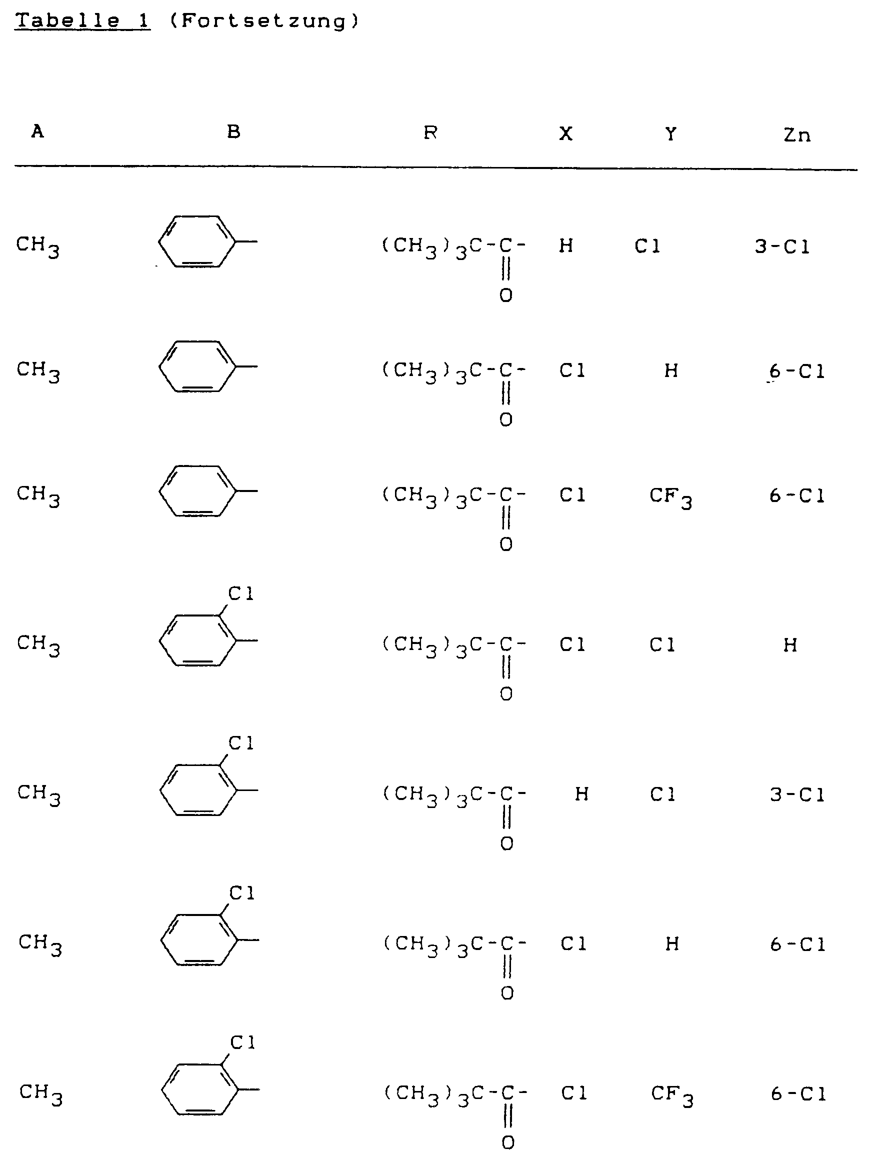

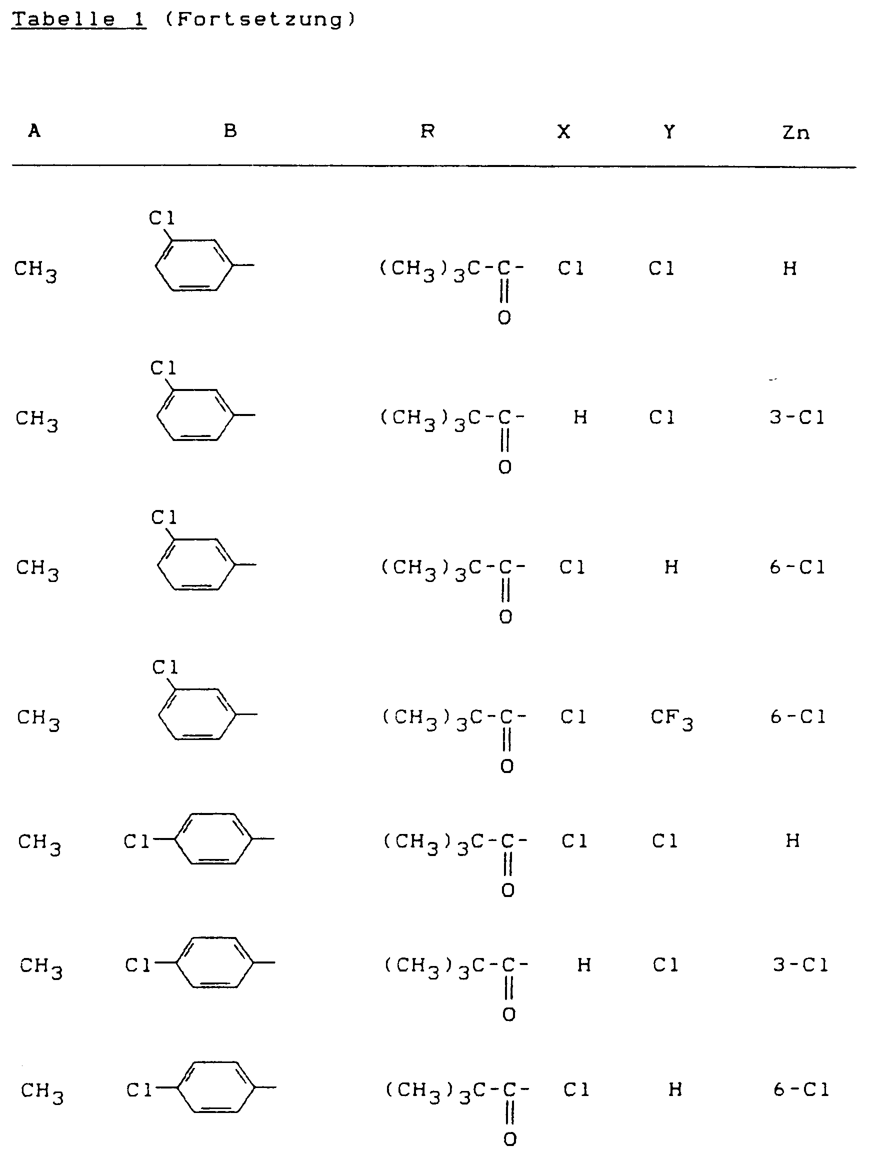

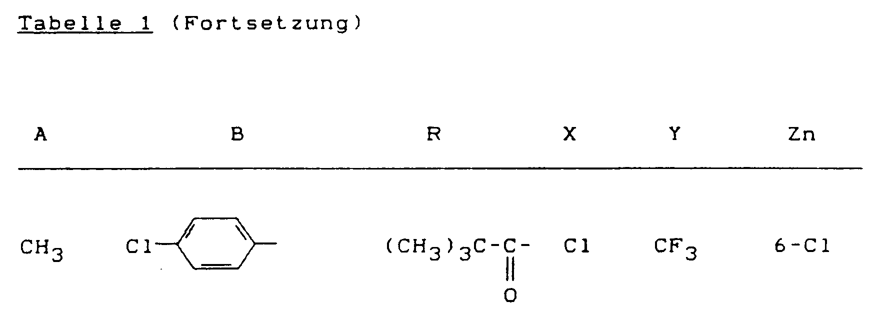

Verwendet man gemäß Verfahren (A) N-2,6-Dichlorphenylacetyl-N-methyl-phenylalaninethylester, so kann der Verlauf des erfindungsgemäßen Verfahrens durch folgendes Reaktionsschema wiedergegeben werden: 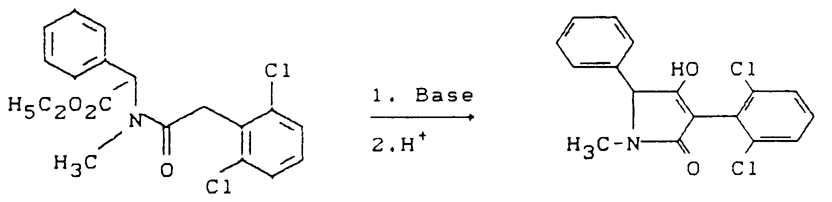
Verwendet man gemäß Verfahren (B) (Variante α) 3-(2,4,6-Trimethylphenyl)-1-isopropyl-5-benzyl-pyrrolidin-2,4-dion und Pivaloylchlorid als Ausgangsstoff, so kann der Verlauf des erfindungsgemäßen Verfahrens durch folgendes Reaktionsschema wiedergegeben werden. 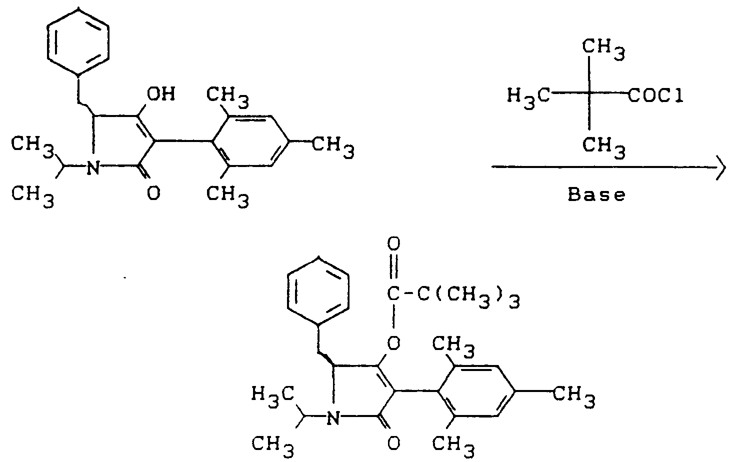
Verwendet man gemäß Verfahren B (Variante β) 3-(2,4,6-Trimethylphenyl)-1-cyclopentyl-5-(4-chlorphenyl)-pyrrolidin-2,4-dion und Acetanhydrid, so kann der Verlauf des erfindungsgemäßen Verfahrens durch folgendes Reaktionsschema wiedergegeben werden. 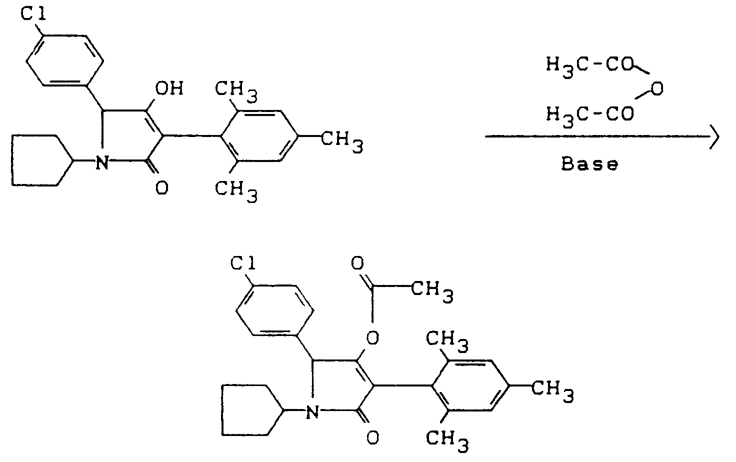
Verwendet man gemäß Verfahren C 3-(2,4-6-Trimethylphenyl)-1-methoxyethyl-5-phenyl-pyrrolidin-2,4-dion und Chlorameisensäureethoxyethylester, so kann der Verlauf des erfindungsgemäßen Verfahrens durch folgendes Reaktionsschema wiedergegeben werden. 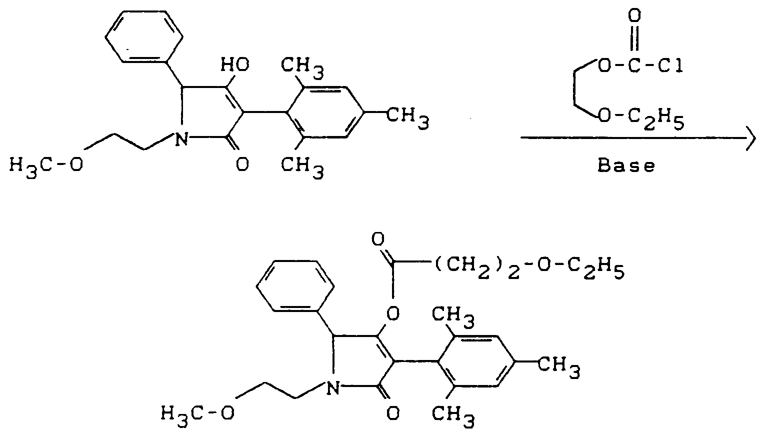
Die bei dem obigen Verfahren (A) als Ausgangsstoffe benötigten Verbindungen der Formel (II) 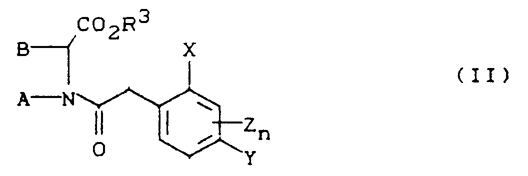
in welcher
A, B, X, Y, Z, n und R³ die oben angegebene Bedeutung haben, sind teilweise bekannt oder lassen sich nach im Prinzip bekannten Methoden in einfacher Weise herstellen. So erhält man z.B. Acyl-aminosäureester der Formel (II), wenn man
a) Aminosäureester der Formel (VI), 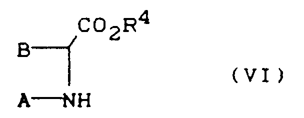 in welcher
   - R⁴: für Wasserstoff (VIa) und Alkyl (VIb) steht
   und
   A und B die oben angegebene Bedeutung haben,
   mit Phenylessigsäurehalogeniden der Formel (VII) 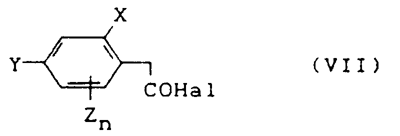 in welcher
   X, Y, Z und n die oben angegebene Bedeutung haben und
   - Hal: für Chlor oder Brom steht,
   acyliert (Chem. Reviews 52 237-416 (1953));
   oder wenn man Acylaminosäuren der Formel (IIa), 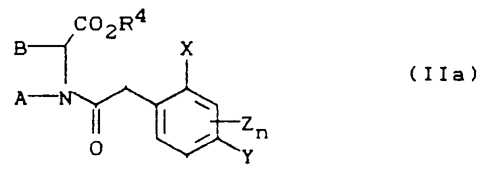 in welcher
   A, B, X, Y, Z und n die oben angegebene Bedeutung haben
   und
   - R⁴: für Wasserstoff steht,
   verestert (Chem. Ind. (London) 1568 (1968)).
   Verbindungen der Formel (IIa) sind beispielsweise aus den Phenylessigsäurehalogeniden der Formel (VII) und Aminosäuren der Formel (VIa) nach Schotten-Baumann (Organikum 9. Auflage 446 (1970) VEB Deutscher Verlag der Wissenschaften, Berlin) erhältlich.
   Verbindungen der Formel VI, in welchen A, B und R⁴ die oben angegebene Bedeutung haben, sind nach literaturbekannten Verfahren beispielsweise aus α-Halogencarbonsäuren bzw. -estern und Aminen (Advanced Organic Chemistry, J. March S. 377, Mc Graw-Hill Inc. 1977), oder durch Umsetzung von Aldehyden mit Cyanid und Aminen nach der Streckersynthese erhältich (vergleiche z. B. Org. Synth. 22 24 (1942) und Org. Synth. Coll. Vol. III, 705).

Beispielhaft seien folgende Verbindungen der Formel (II) genannt: 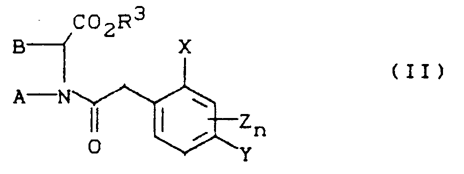
2-Phenyl-N-(2,4-dichlorphenyl-acetyl)-sarkosin-ethylester
2-Phenyl-N-(3,4-dichlorphenyl-acetyl)-sarkosin-ethylester
2-Phenyl-N-(2,6-dichlorphenyl-acetyl)-sarkosin-ethylester
2-(2-Chlorphenyl)-N-(2,4-dichlorphenyl-acetyl)-sarkosin-ethylester
2-(2-Chlorphenyl)-N-(3,4-dichlorphenyl-acetyl)-sarkosin-ethylester
2-(2-Chlorphenyl)-N-(2,6-dichlorphenyl-acetyl)-sarkosin-ethylester
2-(3-Chlorphenyl)-N-(2,4-dichlorphenyl-acetyl)-sarkosin-ethylester
2-(3-Chlorphenyl)-N-(3,4-dichlorphenyl-acetyl)-sarkosin-ethylester
2-(3-Chlorphenyl)-N-(2,6-dichlorphenyl-acetyl)-sarkosin-ethylester
2-Phenyl-N-(2,4,6-trimethylphenyl-acetyl)-sarkosin-ethylester
2-(2-Chlorphenyl)-N-(2,4,6-trimethylphenyl-acetyl)-sarkosin-ethylester
2-(3-Chlorphenyl)-N-(2,4,6-trimethylphenyl-acetyl)-sarkosin-ethylester
2-(4-Chlorphenyl)-N-(2,4,6-trimethylphenyl-acetyl)-sarkosin-ethylester
2-(4-Methylphenyl)-N-(2,4,6-trimethylphenyl-acetyl)-sarkosin-ethylester
2-(4-Chlorphenyl)-N-(2,4-dichlorphenyl-acetyl)-sarkosin-ethylester
2-(4-Chlorphenyl)-N-(3,4-dichlorphenyl-acetyl)-sarkosin-ethylester
2-(4-Chlorphenyl)-N-(2,6-dichlorphenyl-acetyl)-sarkosin-ethylester
2-(4-Methylphenyl)-N-(2,4-dichlorphenyl-acetyl)-sarkosin-ethylester
2-(4-Methylphenyl)-N-(3,4-dichlorphenyl-acetyl)-sarkosin-ethylester
2-(4-Methylphenyl)-N-(2,6-dichlorphenyl-acetyl)-sarkosin-ethylester
2-(4-Methoxyphenyl)-N-(2,4-dichlorphenyl-acetyl)-sarkosin-ethylester
2-(4-Methoxyphenyl)-N-(3,4-dichlorphenyl-acetyl)-sarkosin-ethylester
2-(4-Methoxyphenyl)-N-(2,6-dichlorphenyl-acetyl)-sarkosin-ethylester
2-(4-Methoxyphenyl)-N-(2,4,6-trimethylphenyl-acetyl)-sarkosin-ethylester
2-(4-Chlorphenyl)-N-(2,6-dichlorphenyl-4-trifluormethyl-acetyl)-sarkosin-ethylester
2-(4-Methylphenyl)-N-(2,6-dichlorphenyl-4-trifluormethyl-acetyl)-sarkosin-ethylester
2-(4-Methoxyphenyl)-N-(2,6-dichlorphenyl-4-trifluormethyl-acetyl)-sarkosin-ethylester
2-Phenyl-N-(2,6-dichlorphenyl-4-trifluormethyl-acetyl)-sarkosin-ethylester
2-(2-Chlorphenyl)-N-(2,6-dichlorphenyl-4-trifluormethyl-acetyl)-sarkosin-ethylester
2-(3-Chlorphenyl)-N-(2,6-dichlorphenyl-4-trifluormethyl-acetyl)-sarkosin-ethylester
2-Benzyl-N-(2,4-dichlorphenyl-acetyl)-sarkosin-ethylester
2-Benzyl-N-(3,4-dichlorphenyl-acetyl)-sarkosin-ethylester
2-Benzyl-N-(2,6-dichlorphenyl-acetyl)-sarkosin-ethylester
2-(2-Chlorbenzyl)-N-(2,4-dichlorphenyl-acetyl)-sarkosin-ethylester
2-(2-Chlorbenzyl)-N-(3,4-dichlorphenyl-acetyl)-sarkosin-ethylester
2-(2-Chlorbenzyl)-N-(2,6-dichlorphenyl-acetyl)-sarkosin-ethylester
2-(3-Chlorbenzyl)-N-(2,4-dichlorphenyl-acetyl)-sarkosin-ethylester
2-(3-Chlorbenzyl)-N-(3,4-dichlorphenyl-acetyl)-sarkosin-ethylester
2-(3-Chlorbenzyl)-N-(2,6-dichlorphenyl-acetyl)-sarkosin-ethylester
2-Benzyl-N-(2,4,6-trimethylphenyl-acetyl)-sarkosin-ethylester
2-(2-Chlorbenzyl)-N-(2,4,6-trimethylphenyl-acetyl)-sarkosin-ethylester
2-(3-Chlorbenzyl)-N-(2,4,6-trimethylphenyl-acetyl)-sarkosin-ethylester
2-(4-Chlorbenzyl)-N-(2,4,6-trimethylphenyl-acetyl)-sarkosin-ethylester
2-(4-Methylbenzyl)-N-(2,4,6-trimethylphenyl-acetyl)-sarkosin-ethylester
2-(4-Chlorbenzyl)-N-(2,4-dichlorphenyl-acetyl)-sarkosin-ethylester
2-(4-Chlorbenzyl)-N-(3,4-dichlorphenyl-acetyl)-sarkosin-ethylester
2-(4-Chlorbenzyl)-N-(2,6-dichlorphenyl-acetyl)-sarkosin-ethylester
2-(4-Methylbenzyl)-N-(2,4-dichlorphenyl-acetyl)-sarkosin-ethylester
2-(4-Methylbenzyl)-N-(3,4-dichlorphenyl-acetyl)-sarkosin-ethylester
2-(4-Methylbenzyl)-N-(2,6-dichlorphenyl-acetyl)-sarkosin-ethylester
2-(3-Trifluormethylbenzyl)-N-(2,4-dichlorphenyl-acetyl)-sarkosin-ethylester
2-(3-Trifluormethylbenzyl)-N-(3,4-dichlorphenyl-acetyl)-sarkosin-ethylester
2-(3-Trifluormethylbenzyl)-N-(2,6-dichlorphenyl-acetyl)-sarkosin-ethylester
2-Benzyl-N-(2,6-dichlorphenyl-4-trifluormethyl-acetyl)-sarkosin-ethylester
2-(2-Chlorbenzyl)-N-(2,6-dichlorphenyl-4-trifluormethyl-acetyl)-sarkosin-ethylester
2-(3-Chlorbenzyl)-N-(2,6-dichlorphenyl-4-trifluormethyl-acetyl)-sarkosin-ethylester
2-(4-Chlorbenzyl)-N-(2,6-dichlorphenyl-4-trifluormethyl-acetyl)-sarkosin-ethylester
2-(4-Methylbenzyl)-N-(2,6-dichlorphenyl-4-trifluormethyl-acetyl)-sarkosin-ethylester
5-(3-Trifluormethylbenzyl)-N-(2,6-dichlorphenyl-4-trifluormethyl-acetyl)-sarkosin-ethylester
Das Verfahren (A) ist dadurch gekennzeichnet, daß Verbindungen der Formel (II) in welcher A, B, X, Y, Z, n und R³ die oben angegebene Bedeutung haben in Gegenwart von Basen einer intramolekularen Kondensation unterwirft.

Als Verdünnungsmittel können bei dem erfindungsgemäßen Verfahren (A) alle üblichen inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glylkoldimethylether und Diglykoldimethylether, außerdem polare Lösungsmittel, wie Dimethylsulfoxid, Sulfolan, Dimethylformamid und N-Methyl-pyrrolidon.

Als Deprotonierungsmittel können bei der Durchführung des erfindungsgemäßen Verfahrens (A) alle üblichen Protonenakzeptoren eingesetzt werden. Vorzugsweise verwendbar sind Alkalimetall- und Erdalkalimetall-oxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 oder TDA 1 eingesetzt werden können. Ferner sind

Alkalimetall- und Erdalkalimetallamide und -hydride, wie Natriumamid, Natriumhydrid und Calciumhydrid, und außerdem auch Alkalimetall-alkoholate, wie Natrium-methylat, Natriummethylat und Kalium-tert.-butylat einsetzbar.

Die Reaktionstemperaturen können bei der Durchführung des erfindungsgemäßen Verfahrens (A) innerhalb eines größeren Bereiches variiert werden. Im allgemeinen arbeitet man bei Temperaturen zwischen 0°C und 250°C, vorzugsweise zwischen 50°C und 150°C.

Das erfindungsgemäße Verfahren (A) wird im allgemeinen unter Normaldruck durchgeführt.

Bei der Durchführung des erfindungsgemäßen Verfahrens (A) setzt man die Reaktionskomponenten der Formeln (II) und die deprotonierenden Basen im allgemeinen in etwa äquimolaren Mengen ein. Es ist jedoch auch möglich, die eine oder andere Komponente in einem größeren Überschuß (bis zu 3 Mol) zu verwenden.

Das Verfahren (Bα) ist dadurch gekennzeichnet, daß man Verbindungen der Formel (Ia) mit Carbonsäurehalogeniden der Formel (III) umsetzt.
Adogen 464 = Methyltrialkyl(C₈-C₁₀)ammoniumchlorid
TDA 1 = Tris-(methoxyethoxylethyl)-amin

Als Verdünnungsmittel können bei dem erfindungsgemäßen Verfahren (Bα) bei Verwendung der Säurehalogenide alle gegenüber diesen Verbindungen inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan. Wenn die Hydrolysestabilität des Säurehalogenids es zuläßt, kann die Umsetzung auch in Gegenwart von Wasser durchgeführt werden.

Verwendet man die entsprechenden Carbonsäurehalogenide so kommen als Säurebindemittel bei der Umsetzung nach dem erfindungsgemäßen Verfahren (Bα) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat.

Die Reaktionstemperaturen können auch bei dem erfindungsgemäßen Verfahren (Bα) auch bei der Verwendung von Carbonsäurehalogeniden innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (Bα) werden die Ausgangsstoffe der Formel (Ia) und das Carbonsäurehalogenid der Formel (III) im allgemeinen in angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Das Verfahren (Bβ) ist dadurch gekennzeichnet, daß man Verbindungen der Formel (Ia) mit Carbonsäurehydriden der Formel (IV) umsetzt.

Verwendet man bei dem erfindungsgemäßen Verfahren (Bβ) als Reaktionskomponente der Formel (IV) Carbonsäureanhydride, so können als Verdünnungsmittel vorzugsweise diejenigen Verdünnungsmittel verwendet werden, die auch bei der Verwendung von Säurehalogeniden vorzugsweise in Betracht kommen. Im übrigen kann auch ein im Überschuß eingesetztes Carbonsäurehydrid gleichzeitig als Verdünnungsmittel fungieren.

Die Reaktionstemperaturen können auch bei dem erfindungsgemäßen Verfahren (Bβ) auch bei der Verwendung von Carbonsäureanhydriden innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens werden die Ausgangsstoffe der Formel (Ia) und das Carbonsäureanhydrid der Formel (IV) im allgemeinen in angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Im allgemeinen geht man so vor, daß man Verdünnungsmittel und im Überschuß vorhandenes Carbonsäureanhydrid sowie die entstehende Carbonsäure durch Destillation oder durch Waschen mit einem organischen Lösungsmittel oder mit Wasser entfernt.

Das Verfahren (C) ist dadurch gekennzeichnet, daß man Verbindungen der Formel (Ia) mit Chlorameisensäureestern der Formel (V) umsetzt.

Verwendet man die entsprechenden Chlorameisensäureester so kommen als Säurebindemittel bei der Umsetzung nach dem erfindungsgemäßen Verfahren (C) alle üblichen Säureakzeptoren in Betracht. Vorzugsweise verwendbar sind tertiäre Amine, wie Triethylamin, Pyridin, DABCO, DBC, DBA, Hünig-Base und N,N-Dimethyl-anilin, ferner Erdalkalimetalloxide, wie Magnesium- und Calcium-oxid, außerdem Alkali- und Erdalkali-metall-carbonate, wie Natriumcarbonat, Kaliumcarbonat und Calciumcarbonat.

Als Verdünnungsmittel können bei dem erfindungsgemäßen Verfahren (C) bei Verwendung der Chlorameisensäureester alle gegenüber diesen Verbindungen inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan.

Bei Verwendung der Chlorameisensäureester als Carbonsäure-Derivate der Formel (V) können die Reaktionstemperaturen bei der Durchführung des erfindungsgemäßen Verfahrens (C) innerhalb eines größeren Bereiches variiert werden. Arbeitet man in Gegenwart eines Verdünnungsmittels und eines Säurebindemittels, so liegen die Reaktionstemperaturen im allgemeinen zwischen -20°C und +100°C, vorzugsweise zwischen 0°C und 50°C.

Das erfindungsgemäße Verfahren (C) wird im allgemeinen unter Normaldruck durchgeführt.

Bei der Durchführung des erfindungsgemäßen Verfahrens (C) werden die Ausgangsstoffe der Formel (Ia) und der entsprechende (Chlorameisensäureester der Formel (V) im allgemeinen in angenähert äquivalenten Mengen verwendet. Es ist jedoch auch möglich, die eine oder andere Komponente in einem größeren Überschuß (bis zu 2 Mol) einzusetzen. Die Aufarbeitung erfolgt dann nach üblichen Methoden. Im allgemeinen geht man so vor, daß man ausgefallene Salze entfernt und das verbleibende Reaktionsgemisch durch Abziehen des Verdünnungsmittels einengt.

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel insbesondere als Insektizide, Akarizide und Fungizide geeignet.

Die erfindungsgemäßen Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.

Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Globedera ssp., Meloidogyne spp., Aphelenchoides spp., Longidorus spp., Xiphinema spp., Trichodorus spp..

Die erfindungsgemäßen Wirkstoffe zeichnen sich insbesondere durch eine hohe insektizide und akarizide Wirksamkeit aus.

Sie lassen sich mit besonders gutem Erfolg zur Bekämpfung von pflanzenschädigenden Insekten, wie beispielsweise gegen die Raupen der Kohlschabe (Plutella maculipennis) oder gegen pflanzenschädigende Milben, wie beispielsweise gegen die gemeine Spinnmilbe oder die Bohnenspinnmilbe (Tetranychus urticae) einsetzen.

Desweiteren eignen sich die erfindungsgemäßen Wirkstoffe als fungizide Mittel im Pflanzenschutz. Sie werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur protektiven Bekämpfung von Erysiphegerste-Arten einsetzen.

Darüberhinaus zeigen die erfindungsgemäßen Wirkstoffe außerdem eine fungizide Wirkung gegen Pyricularia oryzae an Reis.

Die Verbindungen der Formel (I) zeigen ferner in gewissem Umfang ebenfalls herbizide Wirkung.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z,B, durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden, Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen 0,1 bis 95 Gewichtsprozent Wirkstoff, vorzugsweise 0,5 bis 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe konnen als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Die erfindungsgemäßen Verbindungen der Formel (I) weisen antimikrobielle, insbesondere starke antibakterielle und antimykotische Wirkungen auf. Sie besitzen ein sehr breites antimykotisches Wirkungsspektrum, insbesondere gegen Dermatophyten und Sproßpilze sowie biphasische Pilze, z.B. gegen Candida-Arten wie Candida albicans, Epidermophyton-Arten wie Epidermophyton floccosum, Aspergillus-Arten wie Aspergillus niger und Aspergillus fumigatus, Trichophyton-Arten wie Trichophyton mentagrophytes, Microsporon-Arten wie Microsporon felineum sowie Torulopsis-Arten wie Torulopsis glabrata. Die Aufzählung dieser Mikroorganismen stellt keinesfalls eine Beschränkung der bekämpfbaren Keime dar, sondern hat nur erläuternden Charakter.

Als Indikationsbeispiele in der Humanmedizin können beispielsweise genannt werden:
Dermatomykosen und Systemmykosen durch Trichophyton mentagrophytes und andere Trichophytonarten, Microsporonarten sowie Epidermophyton floccosum, Sproßpilze und biphasische Pilze sowie Schimmelpilze hervorgerufen.

Als Indikationsgebiet in der Tiermedizin können beispielsweise aufgeführt werden:
Alle Dermatomykosen und Systemmykosen, insbesondere solche, die durch die obengenannten Erreger hervorgerufen werden.

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullenvorliegen, deren Wirkstoffgehalt einen Bruchteil oder einem Vielfachen einer Einzeldosis entspricht. Die Dosierungseinheiten können z.B. 1,2,3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe oder Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder oder Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glucose, Mannit und Kieselsäure re, (b) Bindemittel, z.B. Carboxymethylcellulose, Alginate, Gelantine, Polyvinylpyrrolidon, (c) Feuchthaltemittel, z.B. Glycerin, (d) Sprengmittel, z.B. Agar-Agar, Calciumcarbonat und Natriumbicarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen gegebenenfalls Opakisierungsmittel enthaltenden Überzügen und Hüllen versehen sein und so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. C₁₄-Alkohol mit C₁₆-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe, Sprays können zusätzlich die üblichen Treibmittel z.B. Chlorfluorkohlenwasserstoffe enthalten.

Lösungen und Emulsionen können neben dem oder den Wirkstoffen die üblichen Trägerstoffe wie Lösungsmittel, Lösungsverzögerer und Emulgatoren, z.B. Wasser, Ethylalkohol, Isopropylalkohol, Ethylcarbonat,Ethylacetat, Benzylalkohol, Benzylbenzoat, Propylenglykol, 1,3-Buty lenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe, wie flüssige Verdünnungsmittel, z.B. Wasser, Ethylalkohol, Propylalkohol, Suspendiermittel, z.B. ethoxylierte Isostearylalkohole, Polyoxyethylensorbit- und -sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccharin enthalten.

Die therapeutisch wirksamen Verbindungen sollen in den oben angeführten pharmazeutischen Zubereitungen vorzugsweise in einer Konzentration von etwa 0,1 bis 99,5, vorzugsweise von 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der erfindungsgemäßen Wirkstoffe, sowie von pharmazeutischen Zubereitungen, die einen oder mehrere erfindungsgemäße Wirkstoffe enthalten, in der Human- und Veterinärmedizin zur Verhütung, Besserung und/oder Heilung der oben aufgeführten Erkrankungen.

Die Wirkstoffe oder die pharmazeutischen Zubereitungen können lokal, oral, parenteral, intraperitoneal und/oder rektal, vorzugsweise parenteral, insbesondere intravenös appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 2,5 bis etwa 200, vorzugsweise von 5 bis 150 mg/kg Körpergewicht je 24 Stunden, gegebenenfalls in Form mehrerer Einzelgaben zur Erzielung der gewünschten Ergebnisse zu verabreichen.

Bei oralen Applikationen werden die erfindungsgemäßen Wirkstoffe in Gesamtmengen von etwa 2,5 bis etwa 200, vorzugsweise von 5 bis 150 mg/kg Körpergewicht je 24 Stunden und bei parenteraler Applikation in Gesamtmengen von etwa 2,5 bis etwa 50, vorzugsweise von 1 bis 25 mg/kg Körpergewicht je 24 Stunden verabreicht.

Es kann jedoch erforderlich sein, von den genannten Dosierungen abzuweichenund zwar in Abhängigkeit von der Art und dem Körpergewicht des zu behandelnden Objektes, der Art und Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

### Herstellungsbeispiele:

### Beispiel 1

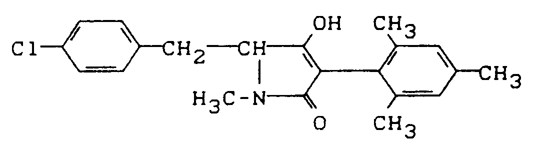

### (Verfahren A)

Zu einer siedenden Lösung von 120 ml absolutem Toluol und 7,5 g (0,25 Mol) Natriumhydrid werden 79,9 g (0,199 Mol) 2-(4-Chlorbenzyl)-N-(2,4,6-trimethylphenyl-acetyl)-sarkosin-ethylester in 200 ml absolutem Toluol getropft und der Ansatz unter dünnschichtchromatographischer Kontrolle unter Rückfluß erhitzt. Nach Beendigung der Reaktion wird unter Eisbadkühlung Ethanol zugetropft, bis kein Wasserstoff mehr entweicht. Nach dem Eindampfen unter vermindertem Druck wird der Rückstand in Wasser aufgenommen und bei 0°C bis 20°C mit konzentrierter Salzsäure angesäuert. Der Niederschlag wird abgesaugt und aus Chloroform / n-Hexan umkristallisiert.

Man erhält 48,4 g (68,4 % der Theorie) 5-(4-Chlorbenzyl)-1-methyl-3-(2,4,6-trimethylphenyl)-pyrrolidin-2,4-dion mit einem Schmelzpunkt > 230°C.

### Beispiel 2

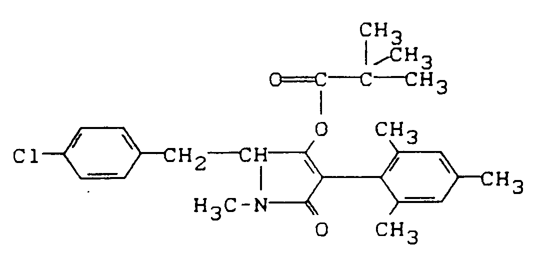

### (Verfahren Bα)

Zu einer Lösung von 7,12 g (0,02 Mol) 5-(4-Chlorbenzyl)-1-methyl-3-(2,4,6-trimethylphenyl)-pyrrolidin-2,4-dion in 70 ml Methyl-t-butyl-ether und 3,4 ml Ethyl-diisopropylamin werden bei 0°C bis 10°C 2,52 ml Pivaloylchlorid in 5 ml Methyl-t-butyl-ether zugetropft und der Ansatz bei Raumtemperatur gerührt. Nach Beendigung der Reaktion (dünnschicht chromatographische Kontrolle) wird der ausgefallene Niederschlag abgesaugt, mit Methyl-t-butyl-ether nachgewaschen und das Filtrat eingeengt. Die säulenchromatographische Reinigung (Laufmittel: Cyclohexan / Essigester 1:1 an Kieselgel) ergibt 7,4 g (84 % der Theorie) 4-t-butyryl-5-(4-chlorbenzyl)-1-methyl-3-(2,4,6-trimethylphenyl)-pyrrolidin-2,4-dion als Öl.

In analoger Weise zu Beispiel 1 und 2 und unter Berücksichtigung der Angaben in der Beschreibung zu den erfindungsgemäßen Verfahren, werden die nachfolgend in Tabelle 2 aufgeführten Endprodukte der Formel (I) 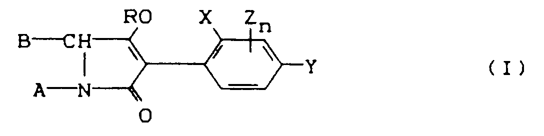
erhalten:

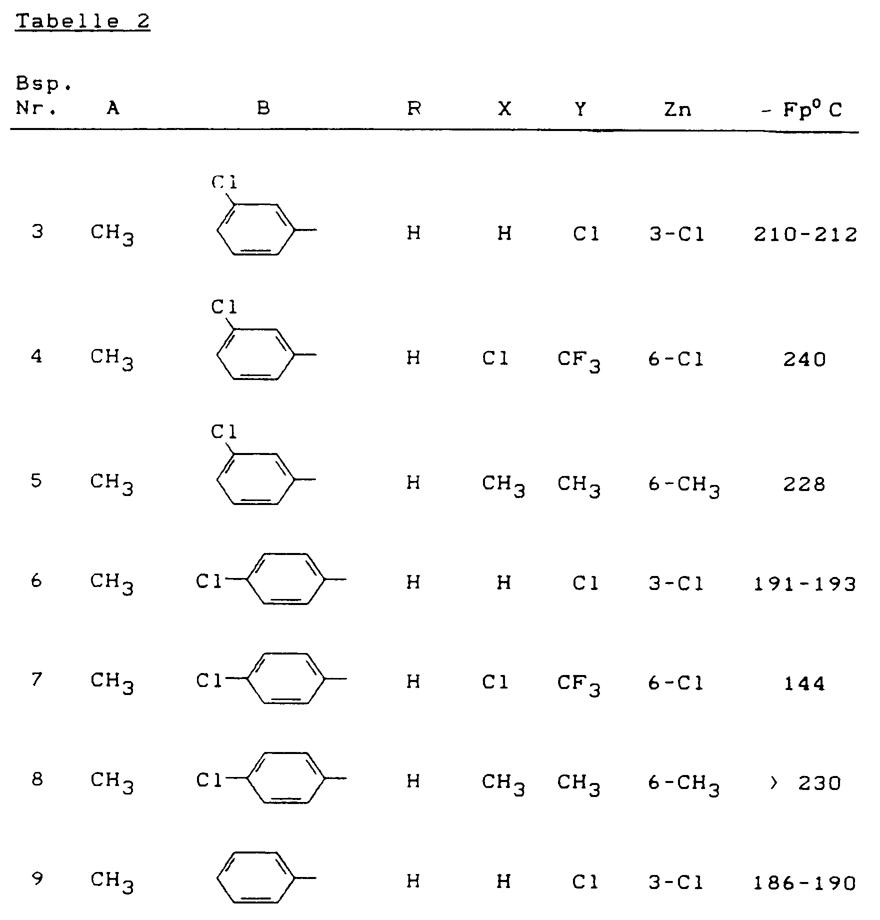

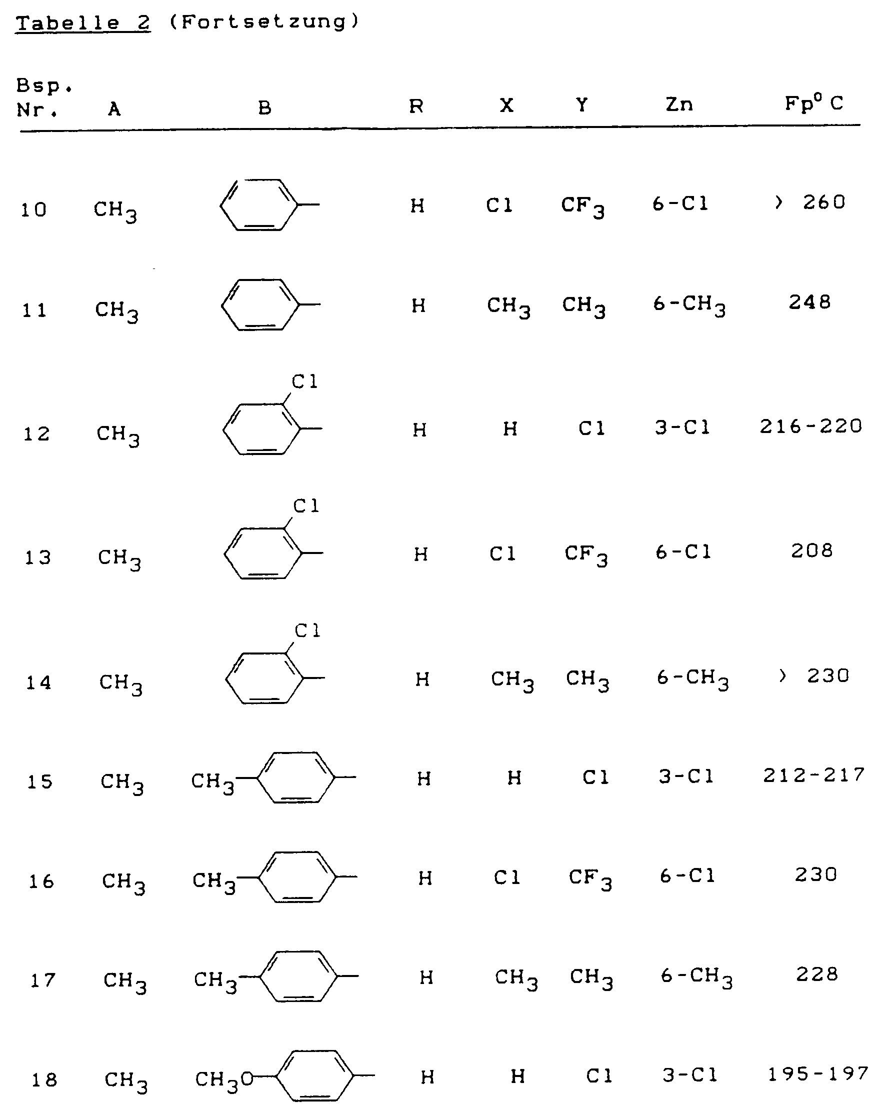

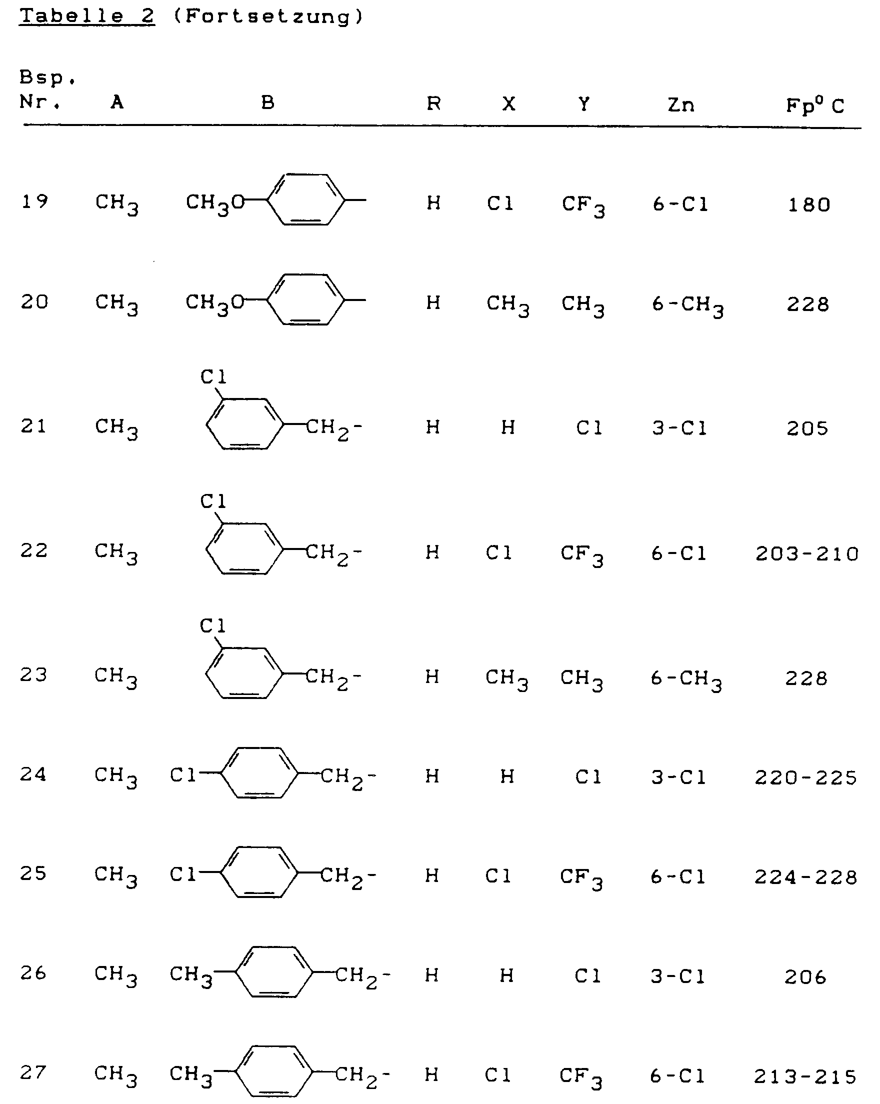

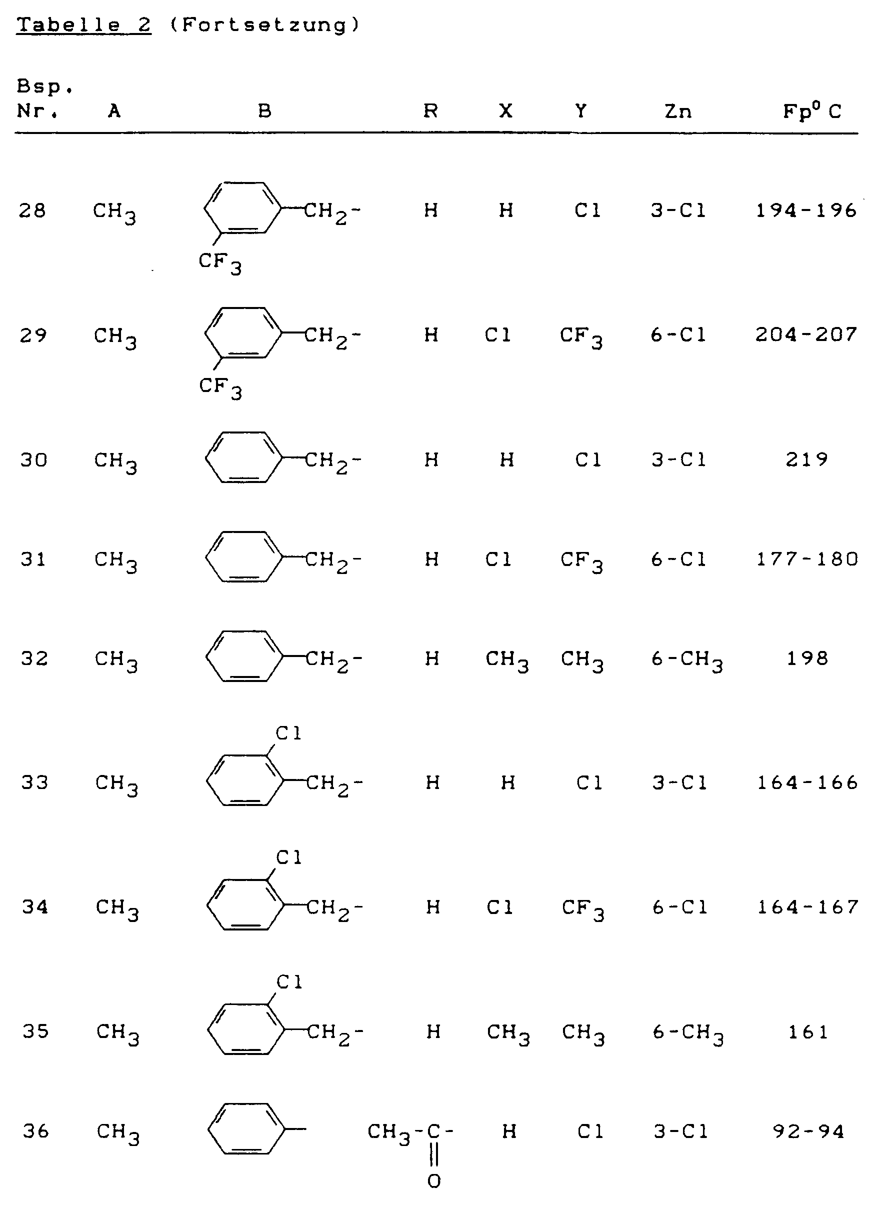

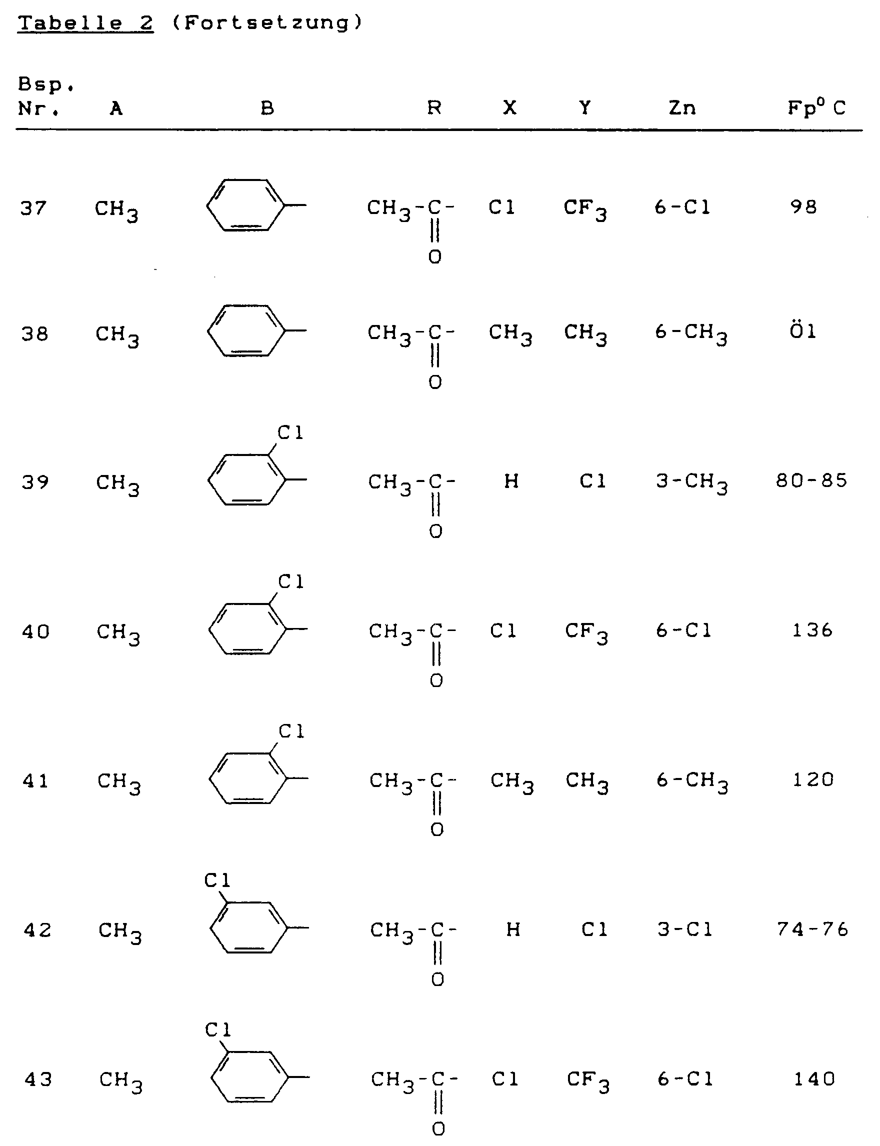

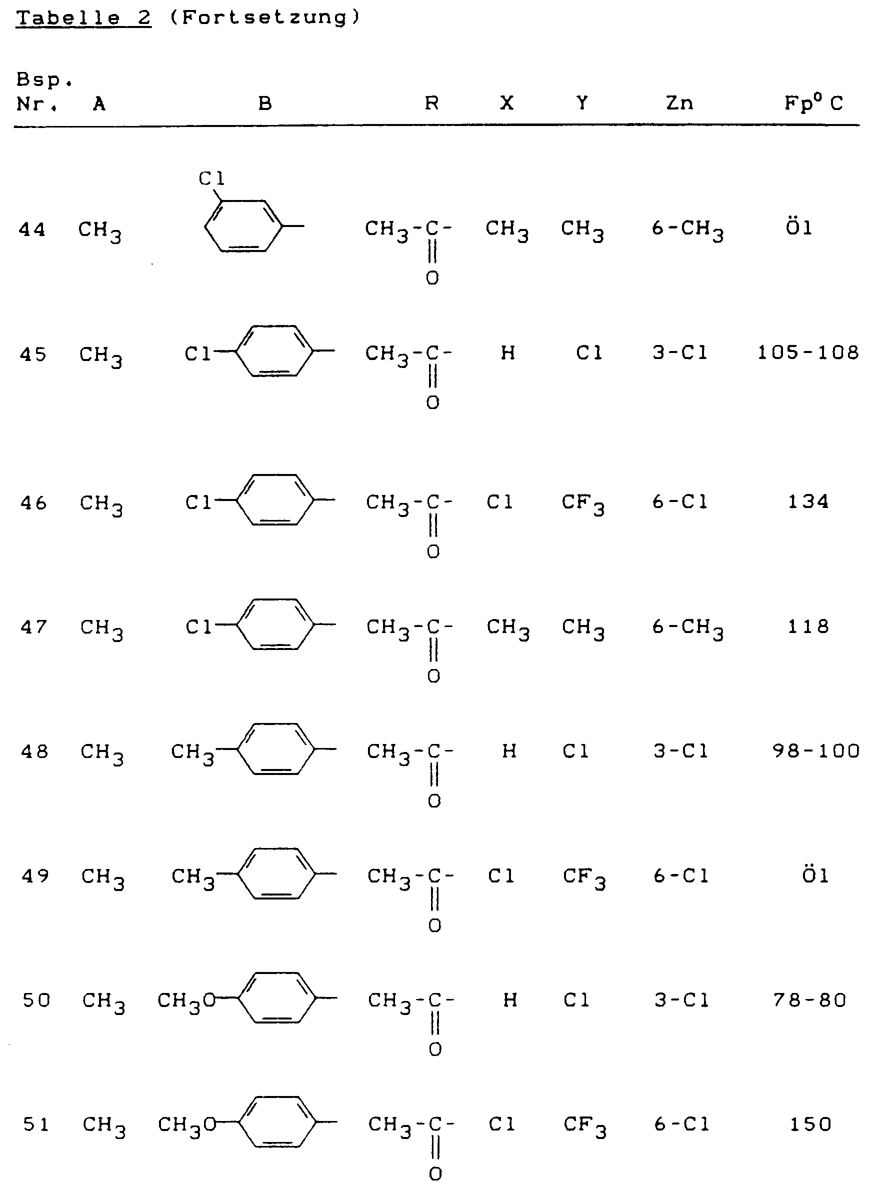

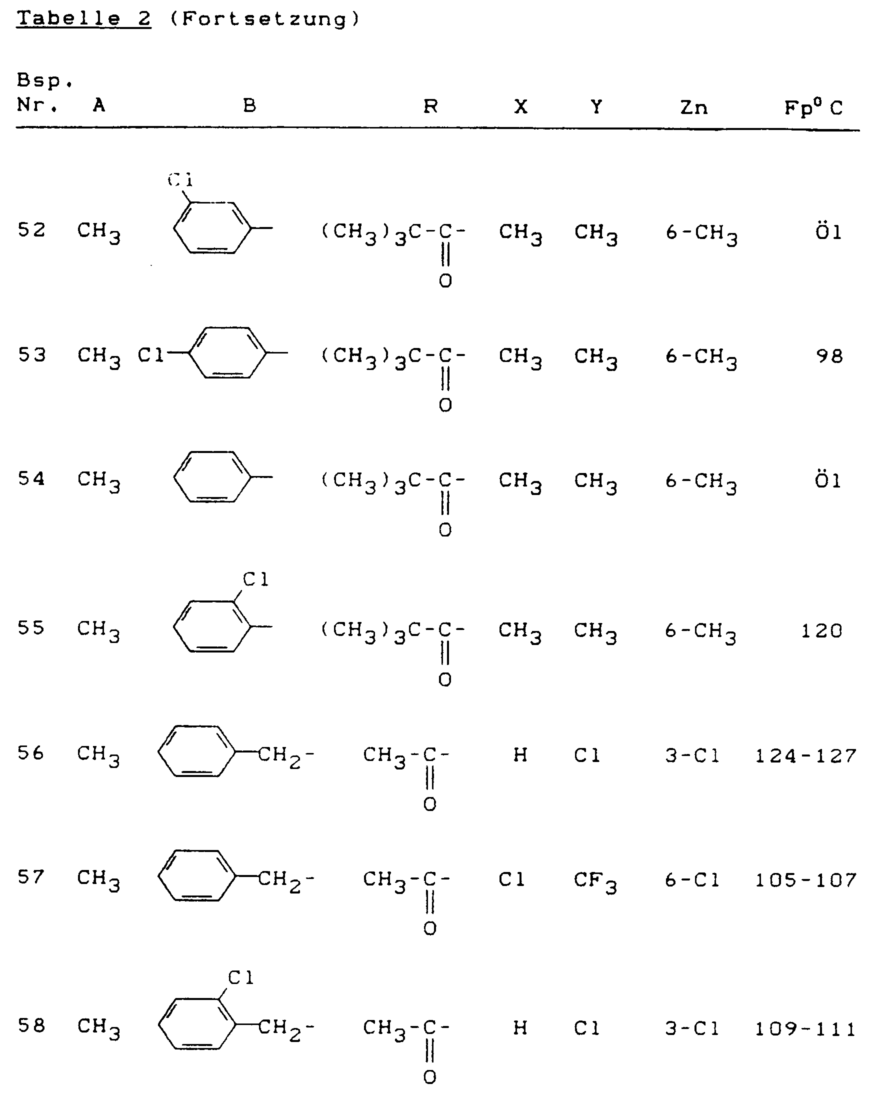

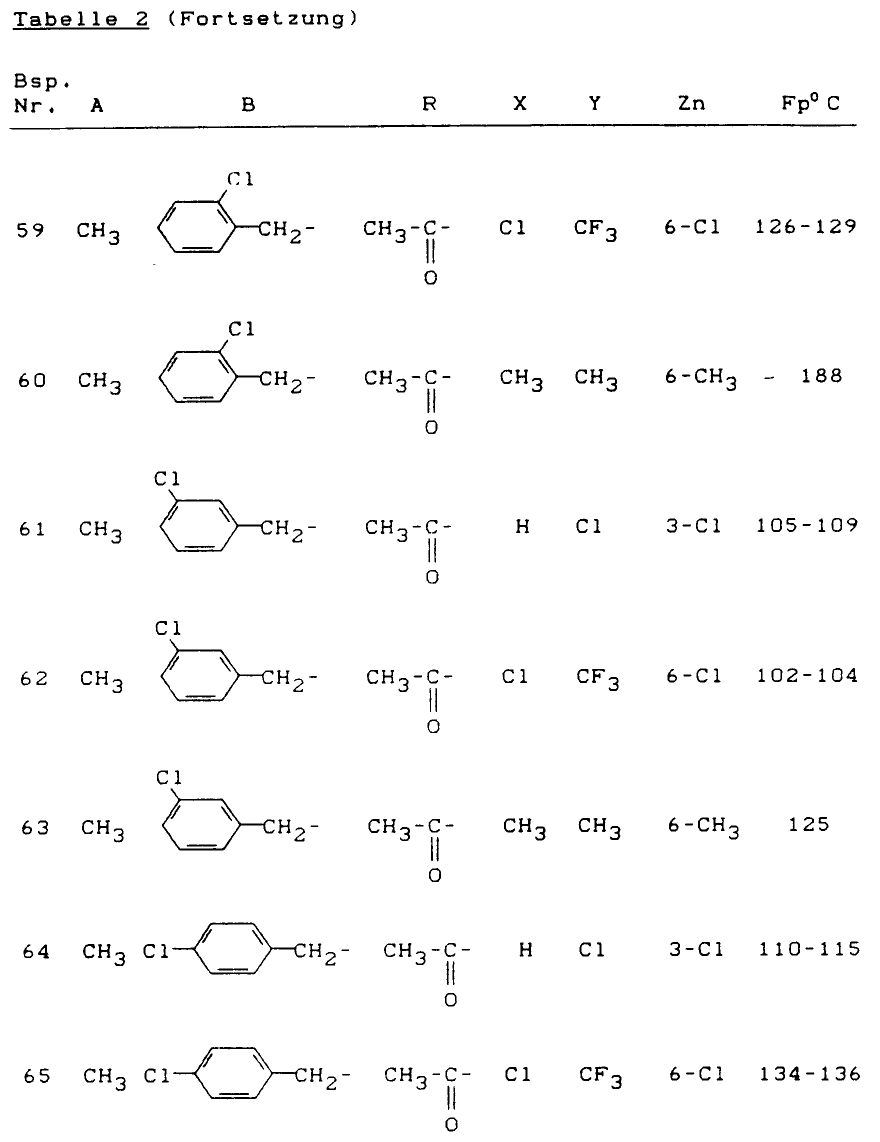

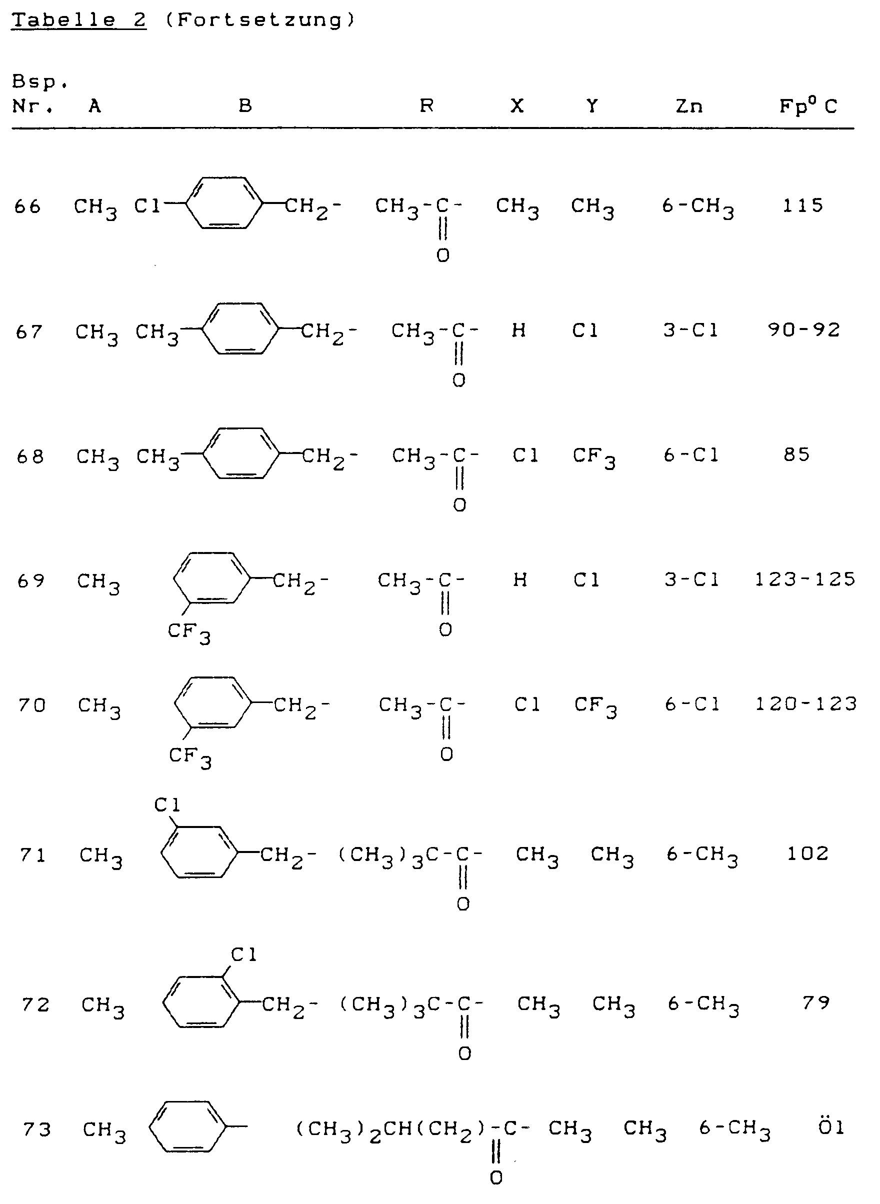

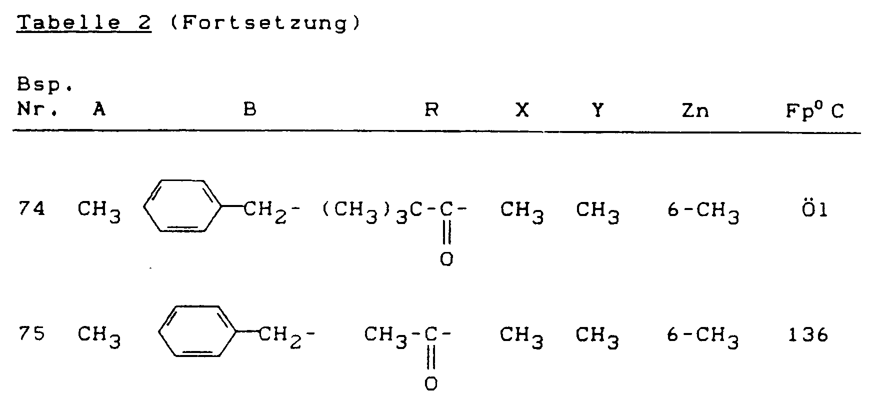

### Herstellung der Vorprodukte

### Beispiel (II-1)

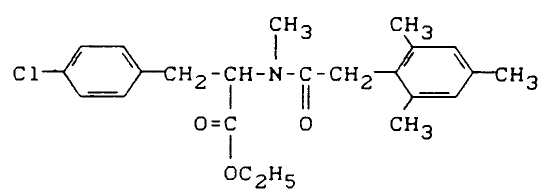

Zu einer Lösung von 48,3 g (0,20 Mol) 4-Chlorphenyl-N-methyl-alanin-ethylester in 300 ml absolutem Tetrahydrofuran und 28 ml Triethylamin werden bei 0°C bis 10°C 39,4 g (0,20 Mol) Mesitylenessigsäurechlorid in 40 ml absolutem Tetrahydrofuran getropft und der Ansatz bei Raumtemperatur gerührt. Das Ende der Reaktion wird durch Dünnschichtchromatographie ermittelt. Danach wird Ansatz in 1,2 l Eiswasser und 200 ml 1 N Salzsäure eingerührt und mit Dichlormethan extrahiert. Die organische Phase wird getrocknet und das Lösungsmittel unter vermindertem Druck abdestilliert.

Man erhält 79,91 g (99,4 % der Theorie) 2-(4-Chlorbenzyl)-N-(2,4,6-trimethylphenylacetyl)-sarkosinethylester als Öl.

In analoger Weise zu Beispiel (II-1) werden die nachfolgend in Tabelle 3 aufgeführten Zwischenprodukte der Formel (II) 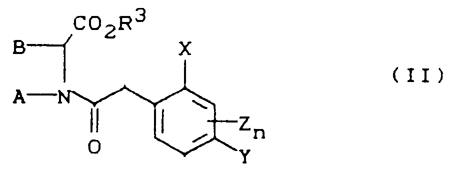
erhalten.

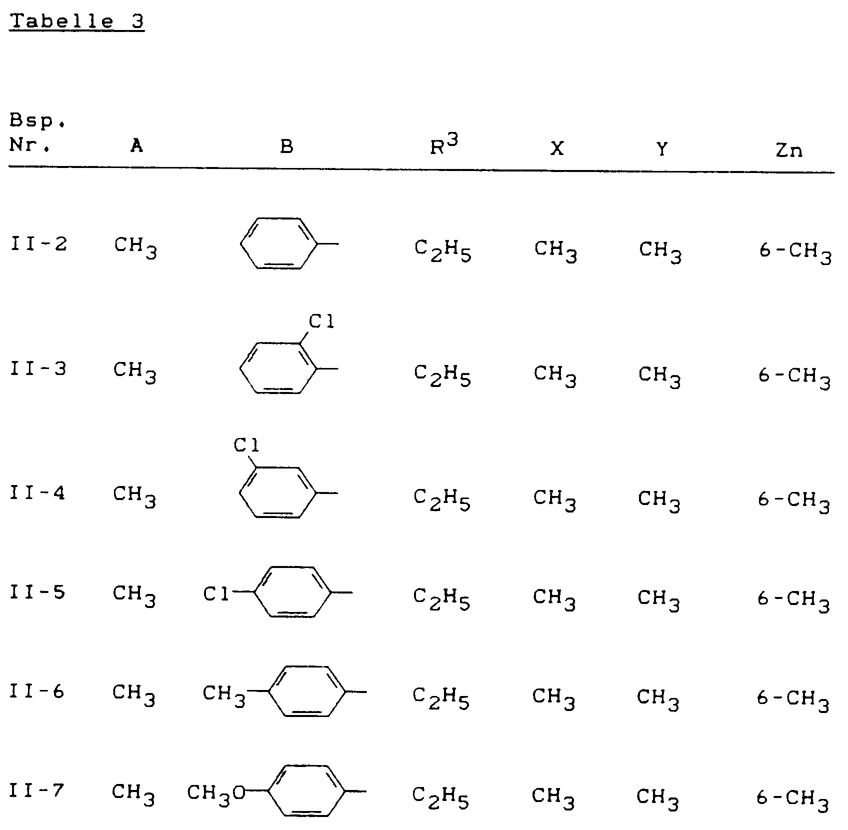

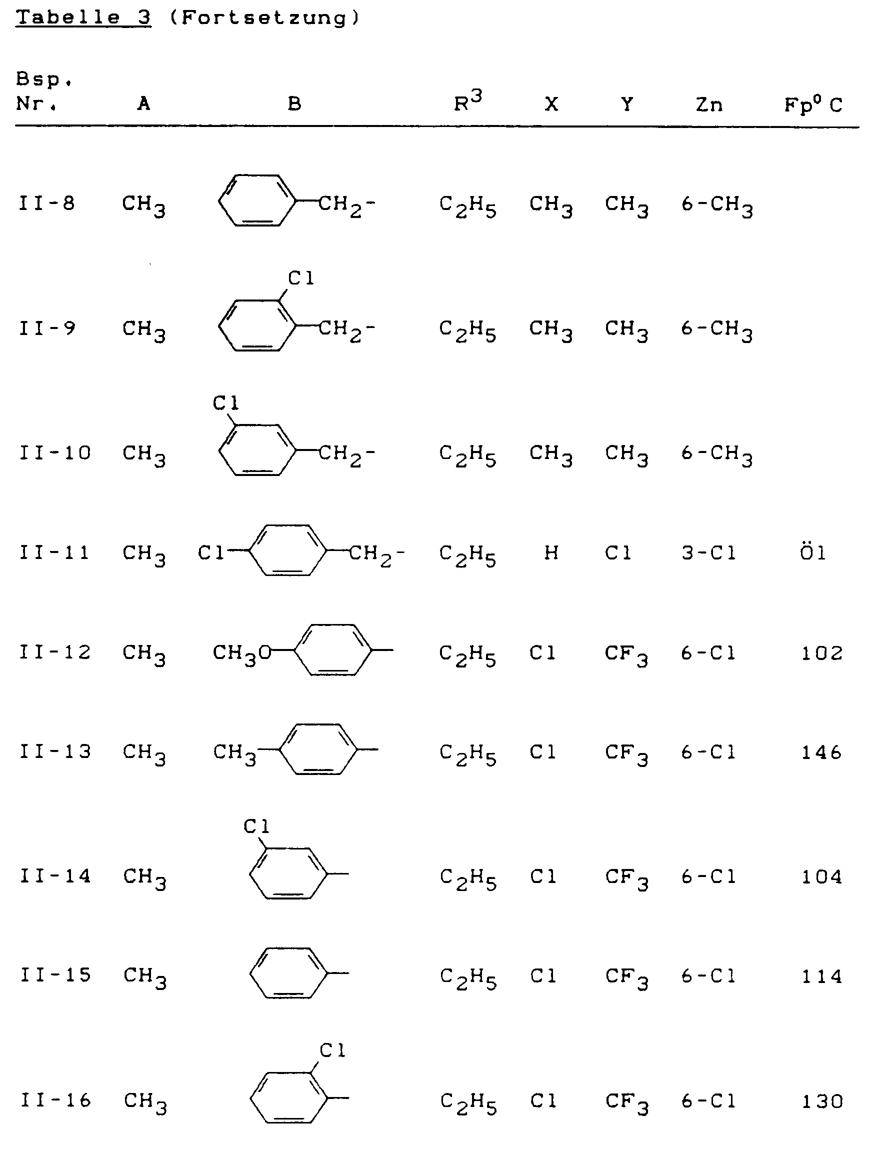

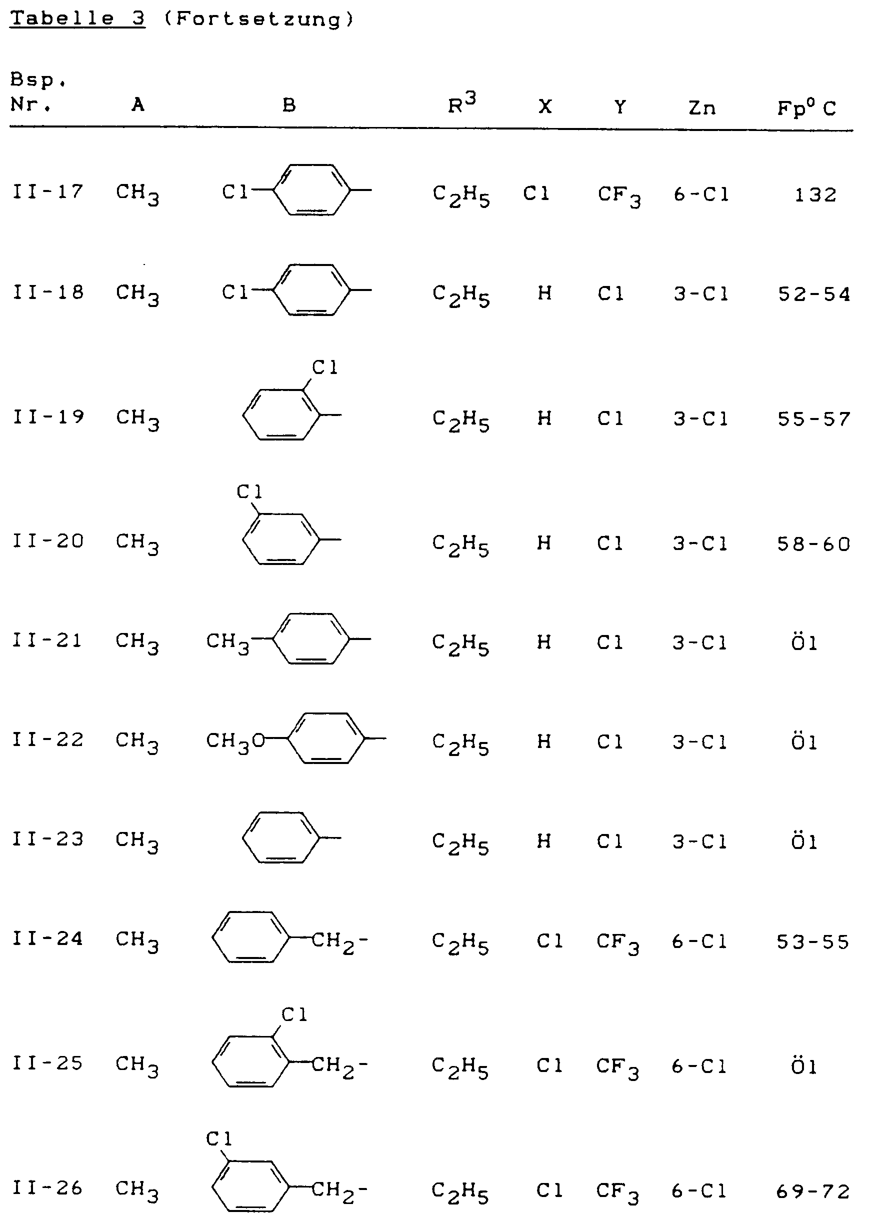

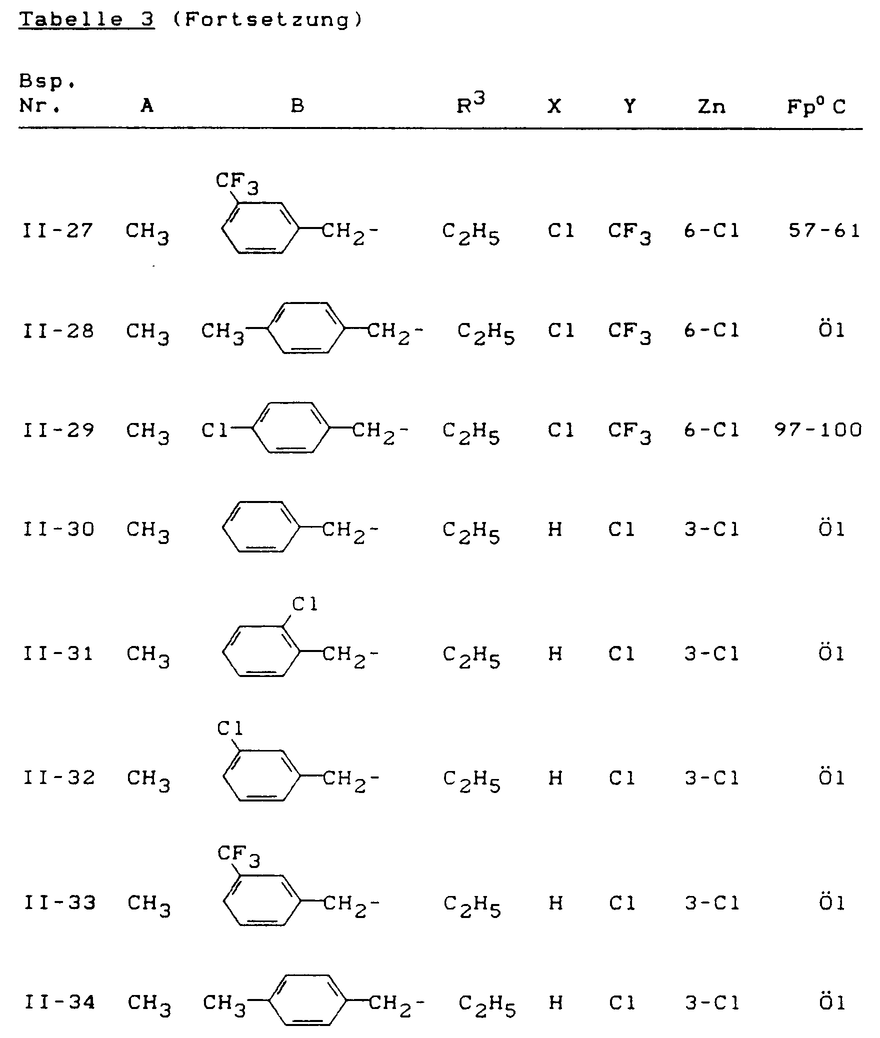

### Anwendungsbeispiele:

In den folgenden Anwendungsbeispielen wurde die nachstehend aufgeführte Verbindung als Vergleichssubstanz eingesetzt: 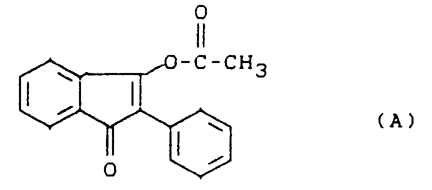
3-(Acetyloxy)-2-phenyl-1H-inden-1-on (bekannt aus US 410 40 43)

### Beispiel A

### Plutella-Test

- Lösungsmittel:: 3 Gewichtsteile Dimethylformamid
- Emulgator:: 1 Gewichtsteil Alkylarylpolyglykolether
Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt, Dabei bedeutet 100 %, daß alle Raupen abgetötet wurden; 0 % bedeutet, daß keine Raupen abgetötet wurden.

Bei diesem Test zeigen z. B. die folgenden Verbindungen der Herstellungsbeispiele überlegene Wirksamkeit gegenüber dem Stand der Technik: 1, 5, 8, 17, 23, 32, 35, 44, 52, 53 und 73.

### Beispiel B

### Tetranychus-Test (resistent)

- Lösungsmittel:: 3 Gewichtsteile Dimethylformamid
- Emulgator:: 1 Gewichtsteil Alkylarylpolyglykolether
Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigen z. B. die folgenden Verbindungen der Herstellungsbeispiele überlegene Wirksamkeit gegenüber dem Stand der Technik: 5, 38, 44, 47, 52, 53, 54 und 73.

### Beispiel C

### Tetranychus-Test (OP-resistent) (OP= organische Phosphorester)

- Lösungsmittel:: 3 Gewichtsteile Dimethylformamid
- Emulgator:: 1 Gewichtsteil Alkylarylpolyglykolether
Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigen z. B. die folgenden Verbindungen der Herstellungsbeispiele überlegene Wirksamkeit gegenüber dem Stand der Technik: 1, 2, 32, 72 und 74.

### Beispiel D

### Erysiphe-Test (Gerste) / protektiv

- Lösungsmittel:: 100 Gewichtsteile Dimethylformamid
- Emulgator:: 0,25 Gewichtsteile Alkylarylpolyglykolether
Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp. hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z. B. die Verbindungen gemäß folgender Herstellungsbeispiele: 38 und 54.

### Beispiel E

### Antimykotische in-vitro-Wirksamkeit

### Versuchsbeschreibung:

Die in-vitro-Prüfungen wurden mit Keiminokula von durchschnittlich 1 x 10⁴ Keimen/ml Substrat durchgeführt. Als Nährmedium diente Yeast Nitrogen Base-Medium für Hefen und Kimmig-Medium für Schimmelpilze und Dermatophyten.

Die Bebrütungstemperatur betrug 37 °C bei Hefen und 28 °C bei Schimmelpilzen und Dermatophyten, die Bebrütungsdauer lag bei 24 bis 96 Stunden bei Hefen und 96 bis 120 Stunden bei Dermatophyten und Schimmelpilzen.

Die Beurteilung der Fungizide erfolgt durch Ausplattieren und erneutes Bebrüten voll gehemmter Ansätze, wobei fungizide Konzentrationen weniger als 100 Keime c.f.n. (colony forming unit) pro ml enthalten.

In diesem Test zeigen die erfindungsgemäßen Verbindungen der Formel (I) gemäß den Herstellungsbeispielen 21, 26, 28, 30, 39, 42, 44, 45, 50, 56, 65, 69 eine stark ausgeprägte antimykotische Wirksamkeit.

## Patentansprüche (Patentansprüche für folgende(n) Vertragsstaat(en): BE, CH, DE, FR, GB, IT, LI, NL)

1. Substituierte 3-Aryl-pyrrolidin-2,4-dione der allgemeinen Formel (I) 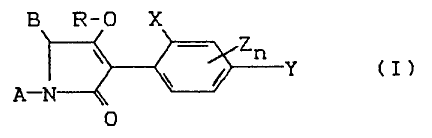 in welcher
X für Wasserstoff, C₁-C₆-Alkyl, Halogen, C₁-C₆-Alkoxy steht,
Y für Wasserstoff, C₁-C₆-Alkyl, Halogen, C₁-C₆-Alkoxy, C₁-C₃-Halogenalkyl steht,
Z für C₁-C₆-Alkyl, Halogen, C₁-C₆-Alkoxy steht,
n für eine Zahl von 0-3 steht,
R für Wasserstoff (Ia) oder für die Gruppen der Formel
-CO-R¹ (Ib) oder
-CO-O-R² (Ic) (Ic)
steht, in welchen
R¹ für gegebenenfalls durch Halogen substituiertes C₁-C₂₀-Alkyl, C₂-C₂₀-Alkenyl, C₁-C₈-Alkoxy-C₂-C₈-alkyl, C₁-C₈-Alkylthio-C₂-C₈-alkyl, C₁-C₈-Polyalkoxy-C₂-C₈-alkyl und Cycloalkyl mit 3-8 Ringatomen, das durch Sauerstoff und/oder Schwefel unterbrochen sein kann, steht,
für gegebenenfalls durch Halogen, Nitro, C₁-C₆-Alkyl, C₁-C₆-Alkoxy, C₁-C₆-Halogenalkyl, C₁-C₆-Halogenalkoxy substituiertes Phenyl;
für gegebenenfalls durch Halogen, C₁-C₆-Alkyl, C₁-C₆-Alkoxy, C₁-C₆-Halogenalkyl, C₁-C₆-Halogenalkoxy substituiertes Phenyl-C₁-C₆-alkyl steht,
für gegebenenfalls durch Fluor, Chlor, Brom und Methyl, Ethyl substituiertes Pyridyl, Pyrimidyl, Thiazolyl und Pyrazolyl steht,
für gegebenenfalls durch Halogen und C₁-C₆-Alkyl substituiertes Phenoxy-C₁-C₆-alkyl steht,
für gegebenenfalls durch Fluor, Chlor, Amino, Methyl, Ethyl substituiertes Pyrodyloxy-C₁-C₄-alkyl, Pyrimidyloxy-C₁-C₄-alkyl und Thiazolyloxy-C₁-C₄-alkyl steht,
R² für gegebenenfalls durch Halogen substituiertes C₁-C₂₀-Alkyl, C₂-C₂₀-Alkenyl, C₁-C₈-Alkoxy-C₂-C₈-alkyl, C₁-C₈-Polyalkoxy-C₂-C₈-alkyl steht,
für gegebenenfalls durch Halogen, Nitro, C₁-C₆-Alkyl, C₁-C₆-Alkoxy, C₁-C₆-Halogenalkyl substituiertes Phenyl steht,
A für gegebenenfalls durch Halogen substituiertes geradkettiges oder verzweigtes C₁-C₁₂-Alkyl, C₃-C₈-Alkenyl, C₃-C₈-Alkinyl, C₁-C₁₀-Alkoxy-C₂-C₈-alkyl, C₁-C₈-Polyalkoxy-C₂-C₈-alkyl, C₁-C₁₀-Alkylthio-C₂-C₈-alkyl, Cycloalkyl mit 3-8 Ringatomen, das durch Sauerstoff und/oder Schwefel unterbrochen sein kann,
steht,
B für gegebenenfalls einfach oder zweifach, gleich oder verschieden durch Nitro, Fluor, Chlor, Brom, gegebenenfalls durch Fluor oder Chlor substituiertes C₁-C₄-Alkyl oder C₁-C₄-Alkoxy substituiertes Phenyl oder Benzyl steht
sowie die enantiomerenreinen Formen von Verbindungen der Formel (I).

2. Substituierte 3-Aryl-pyrrolidin-2,4-dione der Formel (I) gemaß Anspruch 1, in welcher
X für Wasserstoff, C₁-C₄-Alkyl, Halogen, C₁-C₄-Alkoxy steht,
Y für Wasserstoff, C₁-C₆-Alkyl, Halogen, C₁-C₄-Alkoxy, C₁-C₂-Halogenalkyl steht,
Z für C₁-C₄-Alkyl, Halogen, C₁-C₄-Alkoxy steht,
n für eine Zahl von 0-3 steht,
R für Wasserstoff (Ia) oder für die Gruppen der Formel
-CO-R¹ (Ib) oder
-CO-O-R² (Ic)
steht, in welchen
R¹ für gegebenenfalls durch Halogen substituiertes C₁-C₁₆-Alkyl, C₂-C₁₆-Alkenyl, C₁-C₆-Alkoxy-C₂-C₆-alkyl, C₁-C₆-Alkylthio-C₂-C₆-alkyl, C₁-C₆-Polyalkoxy-C₂-C₆-alkyl und Cycloalkyl mit 3-7 Ringatomen, das durch 1-2 Sauerstoff-und/oder Schwefelatome unterbrochen sein kann steht,
für gegebenenfalls durch Halogen, Nitro, C₁-C₄-Alkyl, C₁-C₄-Alkoxy, C₁-C₃-Halogenalkyl, C₁-C₃-Halogenalkoxy substituiertes Phenyl steht,
für gegebenenfalls durch Halogen, C₁-C₄-Alkyl, C₁-C₄-Alkoxy, C₁-C₃-Halogenalkyl, C₁-C₃-Halogenalkoxy substituiertes Phenyl-C₁-C₄-alkyl steht,
für gegebenenfalls durch Fluor, Chlor, Brom und Methyl, Ethyl substituiertes Pyridyl, Pyrimidyl, Thiazolyl und Pyrazolyl steht,
gegebenenfalls für durch Halogen und C₁-C₄-Alkyl substituiertes Phenoxy-C₁-C₅-alkyl steht,
für gegebenfalls durch Fluor, Chlor, Amino, Methyl, Ethyl substituiertes Pyridyloxy-C₁-C₄-alkyl, Pyrimidyloxy-C₁-C₄-alkyl und Thiazolyloxy-C₁-C₄-alkyl steht,
R² für gegebenenfalls durch Halogen substituiertes C₁-C₁₆-Alkyl, C₂-C₁₆-Alkenyl, C₁-C₁₆-Alkoxy-C₂-C₆-alkyl, C₁-C₆-Polyalkoxy-C₂-C₆-alkyl steht,
für gegebenenfalls durch Halogen, Nitro, C₁-C₄-Alkyl, C₁-C₃-Alkoxy, C₁-C₃-Halogenalkyl substituiertes Phenyl steht,
A für gegebenenfalls durch Halogen substituiertes geradkettiges oder verzweigtes C₁-C₁₀-Alkyl, C₃-C₆-Alkenyl, C₃-C₆-Alkinyl, C₁-C₈-Alkoxy-C₂-C₆-alkyl, C₁-C₆-Polyalkoxy-C₂-C₆-alkyl, C₁-C₈-Alkylthio-C₂-C₆-alkyl, Cycloalkyl mit 3-7 Ringatomen, das durch 1-2 Sauerstoff- und/oder Schwefelatomen unterbrochen sein kann
steht,
B für gegebenenfalls einfach oder zweifach, gleich oder verschieden durch Nitro, Fluor, Chlor, Brom, gegebenenfalls durch Fluor oder Chlor substituiertes C₁-C₄-Alkyl oder C₁-C₄-Alkoxy substituiertes Phenyl oder Benzyl steht
sowie die enantiomerenreinen Formen von Verbindungen der Formel (I).

3. Substituierte 3-Aryl-pyrrolidin-2,4-dione der Formel (I) gemaß Anspruch 1, in welcher
X für Wasserstoff, Methyl, Ethyl, Propyl, i-Propyl, Fluor, Chlor, Brom, Methoxy und Ethoxy steht,
Y für Wasserstoff, Methyl, Ethyl, Propyl, i-Propyl, Butyl, i-Butyl, tert.-Butyl, Fluor, Chlor, Brom, Methoxy, Ethoxy und Trifluormethyl steht,
Z für Methyl, Ethyl, i-Propyl, Butyl, i-Butyl, tert.-Butyl, Fluor, Chlor, Brom, Methoxy und Ethoxy steht,
n für eine Zahl von 0-3 steht,
R für Wasserstoff (Ia) oder für die Gruppen der Formel
-CO-R¹ (Ib) oder
-CO-O-R² (Ic)
steht, in welcher
R¹ für gegebenenfalls durch Fluor oder Chlor substituiertes C₁-C₁₄-Alkyl, C₂-C₁₄-Alkenyl, C₁-C₄-Alkoxy-C₂-C₆-alkyl, C₁-C₄-Alkylthio-C₂-C₆-alkyl, C₁-C₄-Polyalkoxyl-C₂-C₄-alkyl und Cycloalkyl mit 3-6 Ringatomen, das durch 1-2 Sauerstoff- und/oder Schwefelatome unterbrochen sein kann steht,
für gegebenenfalls durch Fluor, Chlor, Brom, Methyl, Ethyl, Propyl, i-Propyl, Methoxy, Ethoxy, Trifluormethyl, Trifluormethoxy, Nitro substituiertes Phenyl steht,
für gegebenenfalls durch Fluor, Chlor, Brom, Methyl, Ethyl, Propyl, i-Propyl, Methoxy, Ethoxy, Trifluormethyl, Trifluormethoxy substituiertes Phenyl-C₁-C₃-alkyl steht,
für gegebenenfalls durch Fluor, Chlor, Brom, Methyl, Ethyl substituiertes Pyridyl, Pyrimidyl, Thiazolyl und Pyrazolyl steht,
für gegebenenfalls durch Fluor, Chlor, Methyl, Ethyl substituiertes Phenoxy-C₁-C₄-alkyl steht,
für gegebenenfalls durch Fluor, Chlor, Amino, Methyl, Ethyl substituiertes Pyridyloxy-C₁-C₄-alkyl, Pyrimidyloxy-C₁-C₄-alkyl und Thiazolyloxy-C₁-C₄-alkyl steht,
R² für gegebenenfalls durch Fluor oder Chlor substituiertes C₁-C₁₄-Alkyl, C₂-C₁₄-Alkenyl, C₁-C₄-Alkoxy-C₂-C₆-alkyl, C₁-C₄-Polyalkoxy-C₂-C₆-alkyl steht,
oder für gegebenenfalls durch Fluor, Chlor, Nitro, Methyl, Ethyl, Propyl, i-Propyl, Methoxy, Ethoxy, Trifluormethyl substituiertes Phenyl steht,
A für gegebenenfalls durch Halogen substituiertes geradkettiges oder verzweigtes C₁-C₈-Alkyl, C₃-C₄-Alkenyl, C₃-C₄-Alkinyl, C₁-C₆-Alkoxy-C₂-C₄-alkyl, C₁-C₄-Polyalkoxy-C₂-C₄-alkyl, C₁-C₆-Alkylthio-C₂-C₄-alkyl, Cycloalkyl mit 3-6 Ringatomen, das durch 1-2 Sauerstoff- und/oder Schwefelatomen unterbrochen sein kann steht,
B für unsubstituiertes Phenyl oder Benzyl oder für durch Nitro, Fluor, Chlor, gegebenenfalls durch Fluor oder Chlor substituiertes C₁-C₂-Alkyl oder C₁-C₂-Alkoxy substituiertes Phenyl oder Benzyl steht
sowie die enantiomerenreinen Formen von Verbindungen der Formel I.

4. Verfahren zur Herstellung von substituierten 3-Aryl-pyrrolidin-2,4-dionen der Formel (I) gemaß Anspruch 1, dadurch gekennzeichnet, daß man (A) zum Erhalt von substituierten 3-Phenyl-4-hydroxy-3-pyrrolin-2-onen bzw. deren Dionen der Formel (Ia) 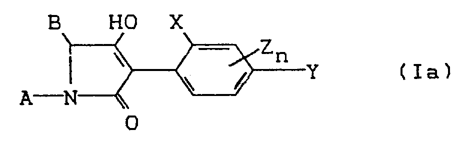 in welcher A, B, X, Y, Z und n die oben angegebene Bedeutung haben,
N-Acylaminosäureester der Formel (II) 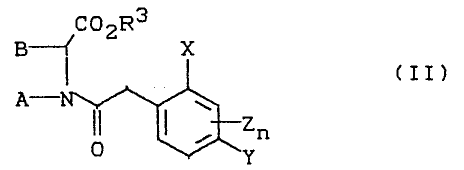 in welcher
A, B, X, Y, Z und n die oben angegebene Bedeutung haben
und
R³ für Alkyl steht,
in Gegenwart eines Verdünnungsmittels und in Gegenwart einer Base intramolekular kondensiert,
(B) oder daß man zum Erhalt von Verbindungen der Formel (Ib) 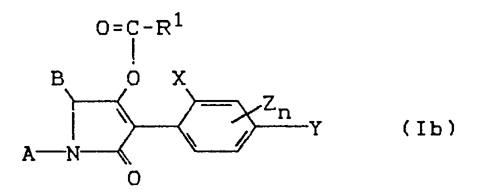 in welcher A, B, X, Y, Z, R¹ und n die oben angegebene Bedeutung haben,
Verbindungen der Formel (Ia), 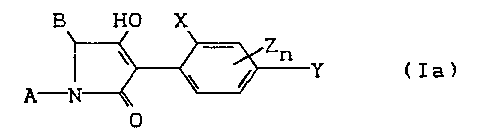 in welcher
A, B, X, Y, Z und n die oben angegebene Bedeutung haben,
α) mit Säurehalogeniden der allgemeinen Formel (III) 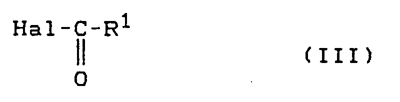 in welcher
R¹ die oben angegebene Bedeutung hat
und
Hal für Halogen steht,
gegebenenfalls in Gegenwart eines Verdünnungsmittels und gegebenenfalls in Gegenwart eines Säurebindemittels,
oder
β) mit Carbonsäureanhydriden der allgemeinen Formel (IV)
R¹-CO-O-CO-R¹ (IV)
in welcher
R¹ die oben angegebene Bedeutung hat,
gegebenenfalls in Gegenwart eines Verdünnungsmittels und gegebenenfalls in Gegenwart eines Säurebindemittels,
umsetzt,
(C) oder daß man zum Erhalt von Verbindungen der Formel (Ic) 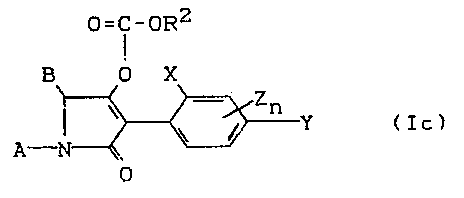 in welcher
A, B, X, Y, Z, R² und n die oben angegebene Bedeutung haben,
Verbindungen der Formel (Ia) 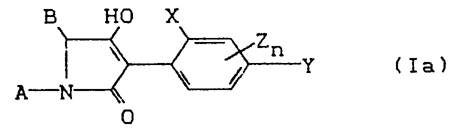 in welcher
A, B, X, Y, Z und n die oben angegebene Bedeutung haben,
mit Chlorameisensäureestern der allgemeinen Formel (V)
R²-O-CO-Cl (V)
in welcher
R² die oben angegebene Bedeutung hat,
gegebenenfalls in Gegenwart eines Verdünnungsmittel und gegebenenfalls in Gegenwart eines Säurebindemittels umsetzt.

5. Insektizide, akarizide und fungizide Mittel, gekennzeichnet durch einen Gehalt an mindestens einem substituierten 3-Aryl-pyrrolidin-2,4-dion-Derivat der allgemeinen Formel (I) gemäß Anspruch 1.

6. Verfahren zur Bekämpfung von Insekten, Spinnentieren und Pilzen, dadurch gekennzeichnet, daß man substituierte 3-Aryl-pyrrolidin-2,4-dion-Derivate der allgemeinen Formel (I) gemäß Anspruch 1 auf Insekten und/oder Spinnentiere und/oder Pilze und/oder deren Lebensraum einwirken läßt.

7. Verwendung von substituierten 3-Aryl-pyrrolidin-2,4-dion-Derivaten der Formel (I) gemäß Anspruch 1 zur Bekämpfung von Insekten und/oder Spinnentieren und/oder Pilzen.

8. Verfahren zur Herstellung von insektiziden, akariziden und fungiziden Mitteln, dadurch gekennzeichnet, daß man 3-Aryl-pyrrolidin-2,4-dion-Derivate der Formel (I) gemaß Anspruch 1 mit Streckmitteln und/oder oberflachenaktiven Mitteln mischt.

9. 3-Aryl-pyrrolidin-2,4-dion-Derivate gemäß Anspruch 1 zur Bekämpfung von Mykosen.

10. Antimykotische Mittel enthaltende 3-Aryl-pyrrolidin-2,4-dion-Derivate gemäß Anspruch 1.

11. Verwendung von 3-Aryl-pyrrolidin-2,4-dion-Derivaten gemäß Anspruch 1 bei der Bekämpfung von Mykosen.

12. Verwendung von 3-Aryl-pyrrolidin-2,4-dion-Derivaten gemäß Anspruch 1 bei der Herstellung von Arzneimitteln zur Bekämpfung von Mykosen.

## Patentansprüche (Patentansprüche für folgende(n) Vertragsstaat(en): ES)

1. Verfahren zur Herstellung von substituierten 3-Aryl-pyrrolidin-2,4-dionen der Formel (I) 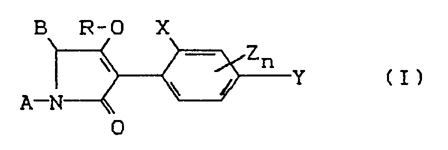 in welcher
X für Wasserstoff, C₁-C₆-Alkyl, Halogen, C₁-C₆-Alkoxy steht,
Y für Wasserstoff, C₁-C₆-Alkyl, Halogen, C₁-C₆-Alkoxy, C₁-C₃-Halogenalkyl steht,
Z für C₁-C₆-Alkyl, Halogen, C₁-C₆-Alkoxy steht,
n für eine Zahl von 0-3 steht,
R für Wasserstoff (Ia) oder für die Gruppen der Formel
-CO-R¹ (Ib) oder
-CO-O-R² (Ic)
steht, in welchen
R¹ für gegebenenfalls durch Halogen substituiertes C₁-C₂₀-Alkyl, C₂-C₂₀-Alkenyl, C₁-C₈-Alkoxy-C₂-C₈-alkyl, C₁-C₈-Alkylthio-C₂-C₈-alkyl, C₁-C₈-Polyalkoxy-C₂-C₈-alkyl und Cycloalkyl mit 3-8 Ringatomen, das durch Sauerstoff und/oder Schwefel unterbrochen sein kann, steht,
für gegebenenfalls durch Halogen, Nitro, C₁-C₆-Alkyl, C₁-C₆-Alkoxy, C₁-C₆-Halogenalkyl, C₁-C₆-Halogenalkoxy substituiertes Phenyl;
für gegebenenfalls durch Halogen, C₁-C₆-Alkyl, C₁-C₆-Alkoxy, C₁-C₆-Halogenalkyl, C₁-C₆-Halogenalkoxy substituiertes Phenyl-C₁-C₆-alkyl steht,
für gegebenenfalls durch Fluor, Chlor, Brom und Methyl, Ethyl substituiertes Pyridyl, Pyrimidyl, Thiazolyl und Pyrazolyl steht,
für gegebenenfalls durch Halogen und C₁-C₆-Alkyl substituiertes Phenoxy-C₁-C₆-alkyl steht,
für gegebenenfalls durch Fluor, Chlor, Amino, Methyl, Ethyl substituiertes Pyridyloxy-C₁-C₄-alkyl, Pyrimidyloxy-C₁-C₄-alkyl und Thiazolyloxy-C₁-C₄-alkyl steht,
R² für gegebenenfalls durch Halogen substituiertes C₁-C₂₀-Alkyl, C₂-C₂₀-Alkenyl, C₁-C₈-Alkoxy-C₂-C₈-alkyl, C₁-C₈-Polyalkoxy-C₂-C₈-alkyl steht,
für gegebenenfalls durch Halogen, Nitro, C₁-C₆-Alkyl, C₁-C₆-Alkoxy, C₁-C₆-Halogenalkyl substituiertes Phenyl steht,
A für gegebenenfalls durch Halogen substituiertes geradkettiges oder verzweigtes C₁-C₁₂-Alkyl, C₃-C₈-Alkenyl, C₃-C₈-Alkinyl, C₁-C₁₀-Alkoxy-C₂-C₈-alkyl, C₁-C₈-Polyalkoxy-C₂-C₈-alkyl, C₁-C₁₀-Alkylthio-C₂-C₈-alkyl, Cycloalkyl mit 3-8 Ringatomen, das durch Sauerstoff und/oder Schwefel unterbrochen sein kann,
steht,
B für gegebenenfalls einfach oder zweifach, gleich oder verschieden durch Nitro, Fluor, Chlor, Brom, gegebenenfalls durch Fluor oder Chlor substituiertes C₁-C₄-Alkyl oder C₁-C₄-Alkoxy substituiertes Phenyl oder Benzyl steht
sowie die enantiomerenreinen Formen von Verbindungen der Formel (I),
dadurch gekennzeichnet, daß man (A) zum Erhalt von substituierten 3-Aryl-pyrrolidin-2,4-dionen bzw. deren Enolen der Formel (Ia) 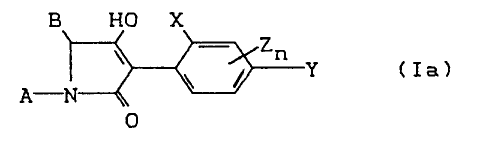 in welcher A, B, X, Y, Z und n die oben angegebene Bedeutung haben,
N-Acylaminosäureester der Formel (II) 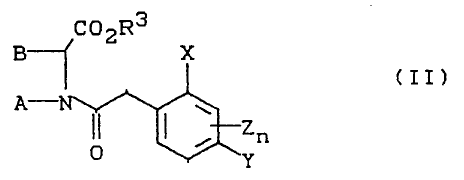 in welcher
A, B, X, Y, Z und n die oben angegebene Bedeutung haben
und
R³ für Alkyl steht,
in Gegenwart eines Verdünnungsmittels und in Gegenwart einer Base intramolekular kondensiert,
(B) oder daß man zum Erhalt von Verbindungen der Formel (Ib) 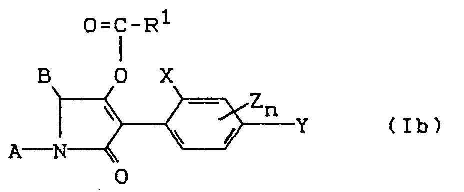 in welcher A, B, X, Y, Z, R¹ und n die oben angegebene Bedeutung haben,
Verbindungen der Formel (Ia), 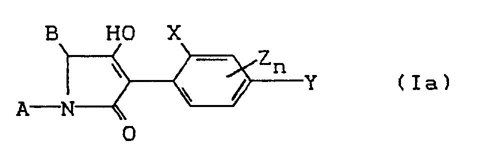 in welcher
A, B, X, Y, Z und n die oben angegebene Bedeutung haben,
α) mit Säurehalogeniden der allgemeinen Formel (III) 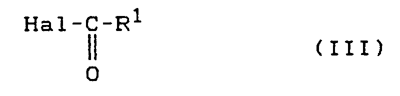 in welcher
R¹ die oben angegebene Bedeutung hat und
Hal für Halogen steht,
gegebenenfalls in Gegenwart eines Verdünnungsmittels und gegebenenfalls in Gegenwart eines Säurebindemittels,
oder
β) mit Carbonsäureanhydriden der allgemeinen Formel (IV)
R¹-CO-O-CO-R¹ (IV)
in welcher
R¹ die oben angegebene Bedeutung hat,
gegebenenfalls in Gegenwart eines Verdünnungsmittels und gegebenenfalls in Gegenwart eines Säurebindemittels,
umsetzt,
(C) oder daß man zum Erhalt von Verbindungen der Formel (Ic) 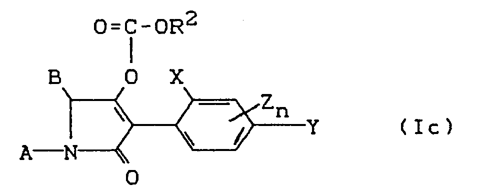 in welcher
A, B, X, Y, Z, R² und n die oben angegebene Bedeutung haben,
Verbindungen der Formel (Ia) 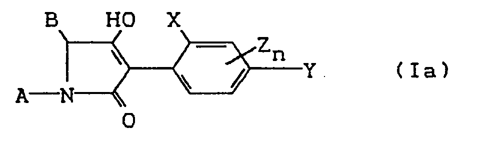 in welcher
A, B, X, Y, Z und n die oben angegebene Bedeutung haben,
mit Chlorameisensäureestern der allgemeinen Formel (V)
R²-O-CO-Cl (V)
in welcher
R² die oben angegebene Bedeutung hat,
gegebenenfalls in Gegenwart eines Verdünnungsmittel und gegebenenfalls in Gegenwart eines Säurebindemittels umsetzt.

2. Verfahren gemäß Anspruch 1 zum Erhalt von Verbindungen der Formel (I), in welcher
X für Wasserstoff, C₁-C₄-Alkyl, Halogen, C₁-C₄-Alkoxy steht,
Y für Wasserstoff, C₁-C₆-Alkyl, Halogen, C₁-C₄-Alkoxy, C₁-C₂-Halogenalkyl steht,
Z für C₁-C₄-Alkyl, Halogen, C₁-C₄-Alkoxy steht,
n für eine Zahl von 0-3 steht,
R für Wasserstoff (Ia) oder für die Gruppen der Formel
-CO-R¹ (Ib) oder
-CO-O-R² (Ic)
steht, in welchen
R¹ für gegebenenfalls durch Halogen substituiertes C₁-C₁₆-Alkyl, C₂-C₁₆-Alkenyl, C₁-C₆-Alkoxy-C₂-C₆-alkyl, C₁-C₆-Alkylthio-C₂-C₆-alkyl, C₁-C₆-Polyalkoxy-C₂-C₆-alkyl und Cycloalkyl mit 3-7 Ringatomen, das durch 1-2 Sauerstoff-und/oder Schwefelatome unterbrochen sein kann steht,
für gegebenenfalls durch Halogen, Nitro, C₁-C₄-Alkyl, C₁-C₄-Alkoxy, C₁-C₃-Halogenalkyl, C₁-C₃-Halogenalkoxy substituiertes Phenyl steht,
für gegebenenfalls durch Halogen, C₁-C₄-Alkyl, C₁-C₄-Alkoxy, C₁-C₃-Halogenalkyl, C₁-C₃-Halogenalkoxy substituiertes Phenyl-C₁-C₄-alkyl steht,
für gegebenenfalls durch Fluor, Chlor, Brom und Methyl, Ethyl substituiertes Pyridyl, Pyrimidyl, Thiazolyl und Pyrazolyl steht,
gegebenenfalls für durch Halogen und C₁-C₄-Alkyl substituiertes Phenoxy-C₁-C₅-alkyl steht,
für gegebenfalls durch Fluor, Chlor, Amino, Methyl, Ethyl substituiertes Pyridyloxy-C₁-C₄-alkyl, Pyrimidyloxy-C₁-C₄-Alkyl und Thiazolyloxy-C₁-C₄-alkyl steht,
R² für gegebenenfalls durch Halogen substituiertes C₁-C₁₆-Alkyl, C₂-C₁₆-Alkenyl, C₁-C₁₆-Alkoxy-C₂-C₆-alkyl, C₁-C₆-Polyalkoxy-C₂-C₆-alkyl steht,
für gegebenenfalls durch Halogen, Nitro, C₁-C₄-Alkyl, C₁-C₃-Alkoxy, C₁-C₃-Halogenalkyl substituiertes Phenyl steht,
A für gegebenenfalls durch Halogen substituiertes geradkettiges oder verzweigtes C₁-C₁₀-Alkyl, C₃-C₆-Alkenyl, C₃-C₆-Alkinyl, C₁-C₈-Alkoxy-C₂-C₆-alkyl, C₁-C₆-Polyalkoxy-C₂-C₆-alkyl, C₁-C₈-Alkylthio-C₂-C₆-alkyl, Cycloalkyl mit 3-7 Ringatomen, das durch 1-2 Sauerstoff- und/oder Schwefelatomen unterbrochen sein kann
steht,
B für gegebenenfalls einfach oder zweifach, gleich oder verschieden durch Nitro, Fluor, Chlor, Brom, gegebenenfalls durch Fluor oder Chlor substituiertes C₁-C₄-Alkyl oder C₁-C₄-Alkoxy substituiertes Phenyl oder Benzyl steht
sowie die enantiomerenreinen Formen von Verbindungen der Formel (I).

3. Insektizide, akarizide und fungizide Mittel, gekennzeichnet durch einen Gehalt an mindestens einem substituierten 3-Aryl-pyrrolidin-2,4-dion-Derivat der allgemeinen Formel (I) gemäß Anspruch 1.

4. Verfahren zur Bekämpfung von Insekten, Spinnentieren und Pilzen, dadurch gekennzeichnet, daß man substituierte 3-Aryl-pyrrolidin-2,4-dion-Derivate der allgemeinen Formel (I) gemäß Anspruch 1 auf Insekten und/oder Spinnentiere und/oder Pilze und/oder deren Lebensraum einwirken läßt.

5. Verwendung von substituierten 3-Aryl-pyrrolidin-2,4-dion-Derivaten der Formel (I) gemäß Anspruch 1 zur Bekämpfung von Insekten und/oder Spinnentieren und/oder Pilzen.

6. Verfahren zur Herstellung von insektiziden, akariziden und fungiziden Mitteln, dadurch gekennzeichnet, daß man 3-Aryl-pyrrolidin-2,4-dion-Derivate der Formel (I) gemäß Anspruch 1 mit Streckmitteln und/oder oberflächenaktiven Mitteln mischt.

7. Antimykotische Mittel, enthaltend 3-Aryl-pyrrolidin-2,4-dion-Derivate der allgemeinen Formel (I) gemäß Anspruch 1.

8. Verwendung von 3-Aryl-pyrrolidin-2,4-dion-Derivaten gemäß Anspruch 1 bei der Bekämpfung von Mykosen.

9. Verwendung von 3-Aryl-pyrrolidin-2,4-dion-Derivaten gemäß Anspruch 1 bei der Herstellung von Arzneimitteln zur Bekämpfung von Mykosen.

## Claims (Claims for the following Contracting State(s): BE, CH, DE, FR, GB, IT, LI, NL)

1. Substituted 3-aryl-pyrrolidine-2,4-diones of the general formula (I) 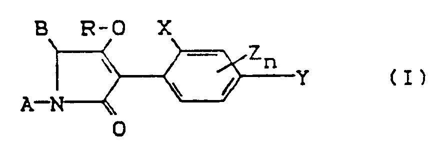 in which
X represents hydrogen, C₁-C₆-alkyl, halogen or C₁-C₆-alkoxy,
Y represents hydrogen, C₁-C₆-alkyl, halogen, C₁-C₆-alkoxy or C₁-C₃-halogenoalkyl,
Z represents C₁-C₆-alkyl, halogen or C₁-C₆-alkoxy,
n represents a number from 0-3,
R represents hydrogen (Ia) or the groups of the formula
-CO-R¹ (Ib) or
-CO-O-R² (Ic)
in which
R¹ represents optionally halogen-substituted C₁-C₂₀-alkyl, C₂-C₂₀-alkenyl, C₁-C₈-alkoxy-C₂-C₈-alkyl, C₁-C₈-alkylthio-C₂-C₈-alkyl, C₁-C₈-polyalkoxy-C₂-C₈-alkyl and cycloalkyl which has 3-8 ring atoms and which can be interrupted by oxygen and/or sulphur,
or represents phenyl which is optionally substituted by halogen, nitro, C₁-C₆-alkyl, C₁-C₆-alkoxy, C₁-C₆-halogenoalkyl or C₁-C₆-halogenoalkoxy;
or represents phenyl-C₁-C₆-alkyl which is optionally substituted by halogen, C₁-C₆-alkyl, C₁-C₆-alkoxy, C₁-C₆-halogenoalkyl or C₁-C₆-halogenoalkoxy,
or represents pyridyl, pyrimidyl, thiazolyl and pyrazolyl which are optionally substituted by fluorine, chlorine, bromine and methyl or ethyl,
or represents phenoxy-C₁-C₆-alkyl which is optionally substituted by halogen and C₁-C₆-alkyl,
or represents pyridyloxy-C₁-C₄-alkyl, pyrimidyloxy-C₁-C₄-alkyl and thiazolyloxy-C₁-C₄-alkyl which are optionally substituted by fluorine, chlorine, amino, methyl or ethyl,
R² represents optionally halogen-substituted C₁-C₂₀-alkyl, C₂-C₂₀-alkenyl, C₁-C₈-alkoxy-C₂-C₈-alkyl or C₁-C₈-polyalkoxy-C₂-C₈-alkyl, or represents phenyl which is optionally substituted by halogen, nitro, C₁-C₆-alkyl, C₁-C₆-alkoxy or C₁-C₆-halogenoalkyl,
A represents optionally halogen-substituted straight-chain or branched C₁-C₁₂-alkyl, C₃-C₈-alkenyl, C₃-C₈-alkinyl, C₁-C₁₀-alkoxy-C₂-C₈-alkyl, C₁-C₈-polyalkoxy-C₂-C₈-alkyl, C₁-C₁₀-alkylthio-C₂-C₈-alkyl, cycloalkyl which has 3-8 ring atoms and which can be interrupted by oxygen and/or sulphur,
B represents phenyl or benzyl, each of which is optionally monosubstituted or disubstituted by identical or different substituents from the series comprising nitro, fluorine, chlorine, bromine or in each case optionally fluorine- or chlorine-substituted C₁-C₄-alkyl or C₁-C₄-alkoxy,
and the pure enantiomeric forms of compounds of the formula (I).

2. Substituted 3-aryl-pyrrolidine-2,4-diones of the formula (I) according to Claim 1, in which
X represents hydrogen, C₁-C₄-alkyl, halogen or C₁-C₄-alkoxy,
Y represents hydrogen, C₁-C₆-alkyl, halogen, C₁-C₄-alkoxy or C₁-C₂-halogenoalkyl,
Z represents C₁-C₄-alkyl, halogen or C₁-C₄-alkoxy,
n represents a number from 0-3,
R represents hydrogen (Ia) or the groups of the formula
-CO-R¹ (Ib) or
-CO-O-R² (Ic)
in which
R¹ represents optionally halogen-substituted C₁-C₁₆-alkyl, C₂-C₁₆-alkenyl, C₁-C₆-alkoxy-C₂-C₆-alkyl, C₁-C₆-alkylthio-C₂-C₆-alkyl, C₁-C₆-polyalkoxy-C₂-C₆-alkyl and cycloalkyl which has 3-7 ring atoms and which can be interrupted by 1-2 oxygen and/or sulphur atoms,
or represents phenyl which is optionally substituted by halogen, nitro, C₁-C₄-alkyl, C₁-C₄-alkoxy, C₁-C₃-halogenoalkyl or C₁-C₃-halogenoalkoxy,
or represents phenyl-C₁-C₄-alkyl which is optionally substituted by halogen, C₁-C₄-alkyl, C₁-C₄-alkoxy, C₁-C₃-halogenoalkyl or C₁-C₃-halogenoalkoxy,
or represents pyridyl, pyrimidyl, thiazolyl and pyrazolyl which are optionally substituted by fluorine, chlorine, bromine and methyl or ethyl,
or represents phenoxy-C₁-C₅-alkyl which is optionally substituted by halogen and C₁-C₄-alkyl,
or represents pyridyloxy-C₁-C₄-alkyl, pyrimidyloxy-C₁-C₄-alkyl and thiazolyloxy-C₁-C₄-alkyl which are optionally substituted by fluorine, chlorine, amino, methyl or ethyl,
R² represents optionally halogen-substituted C₁-C₁₆-alkyl, C₂-C₁₆-alkenyl, C₁-C₁₆-alkoxy-C₂-C₆-alkyl or C₁-C₆-polyalkoxy-C₂-C₆-alkyl,
or represents phenyl which is optionally substituted by halogen, nitro, C₁-C₄-alkyl, C₁-C₃-alkoxy or C₁-C₃-halogenoalkyl,
A represents optionally halogen-substituted straight-chain or branched C₁-C₁₀-alkyl, C₃-C₆-alkenyl, C₃-C₆-alkinyl, C₁-C₈-alkoxy-C₂-C₆-alkyl, C₁-C₆-polyalkoxy-C₂-C₆-alkyl, C₁-C₈-alkylthio-C₂-C₆-alkyl, cycloalkyl which has 3-7 ring atoms and which can be interrupted by 1-2 oxygen and/or sulphur atoms,
B represents phenyl or benzyl, each of which is optionally monosubstituted or disubstituted by identical or different substituents from the series comprising nitro, fluorine, chlorine, bromine or in each case optionally fluorine- or chlorine- substituted C₁-C₄-alkyl or C₁-C₄-alkoxy,
and the pure enantiomeric forms of compounds of the formula (I).

3. Substituted 3-aryl-pyrrolidine-2,4-diones of the formula (I) according to Claim 1, in which
X represents hydrogen, methyl, ethyl, propyl, i-propyl, fluorine, chlorine, bromine, methoxy and ethoxy,
Y represents hydrogen, methyl, ethyl, propyl, i-propyl, butyl, i-butyl, tert.-butyl, fluorine, chlorine, bromine, methoxy, ethoxy and trifluoromethyl,
Z represents methyl, ethyl, i-propyl, butyl, i-butyl, tert.-butyl, fluorine, chlorine, bromine, methoxy and ethoxy,
n represents a number from 0-3,
R represents hydrogen (Ia) or the groups of the formula
-CO-R¹ (Ib) or
-CO-O-R² (Ic)
in which
R¹ represents optionally fluorine- or chlorine-substituted C₁-C₁₄-alkyl, C₂-C₁₄-alkenyl, C₁-C₄-alkoxy-C₂-C₆-alkyl, C₁-C₄-alkylthio-C₂-C₆-alkyl, C₁-C₄-polyalkoxy-C₂-C₄-alkyl and cycloalkyl which has 3-6 ring atoms and which can be interrupted by 1-2 oxygen and/or sulphur atoms,
or represents phenyl which is optionally substituted by fluorine, chlorine, bromine, methyl, ethyl, propyl, i-propyl, methoxy, ethoxy, trifluoromethyl, trifluoromethoxy or nitro,
or represents phenyl-C₁-C₃-alkyl which is optionally substituted by fluorine, chlorine, bromine, methyl, ethyl, propyl, i-propyl, methoxy, ethoxy, trifluoromethyl or trifluoromethoxy,
or represents pyridyl, pyrimidyl, thiazolyl and pyrazolyl which are optionally substituted by fluorine, chlorine, bromine, methyl or ethyl,
or represents phenoxy-C₁-C₄-alkyl which is optionally substituted by fluorine, chlorine, methyl or ethyl,
or represents pyridyloxy-C₁-C₄-alkyl, pyrimidyloxy-C₁-C₄-alkyl and thiazolyloxy-C₁-C₄-alkyl which are optionally substituted by fluorine, chlorine, amino, methyl or ethyl,
R² represents optionally fluorine- or chlorine-substituted C₁-C₁₄-alkyl, C₂-C₁₄-alkenyl, C₁-C₄-alkoxy-C₂-C₆-alkyl or C₁-C₄-polyalkoxy-C₂-C₆-alkyl,
or represents phenyl which is optionally substituted by fluorine, chlorine, nitro, methyl, ethyl, propyl, i-propyl, methoxy, ethoxy or trifluoromethyl,
A represents optionally halogen-substituted straight-chain or branched C₁-C₈-alkyl, C₃-C₄-alkenyl, C₃-C₄-alkinyl, C₁-C₆-alkoxy-C₂-C₄-alkyl, C₁-C₄-polyalkoxy-C₂-C₄-alkyl, C₁-C₆-alkylthio-C₂-C₄-alkyl or cycloalkyl which has 3-6 ring atoms and which can be interrupted by 1-2 oxygen and/or sulphur atoms,
B represents unsubstituted phenyl or benzyl, or represents phenyl or benzyl, each of which is substituted by nitro, fluorine, chlorine or in each case optionally fluorine- or chlorine-substituted C₁-C₂-alkyl or C₁-C₂-alkoxy,
and the pure enantiomeric forms of compounds of the formula I.

4. Process for the preparation of substituted 3-aryl-pyrrolidine-2,4-diones of the formula (I) according to Claim 1, characterised in that (A) in order to obtain substituted 3-phenyl-4-hydroxy-3-pyrrolin-2-ones or their diones of the formula (Ia) 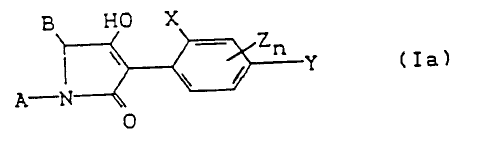 in which A, B, X, Y, Z and n have the abovementioned meaning,
N-acylamino acid esters of the formula (II) 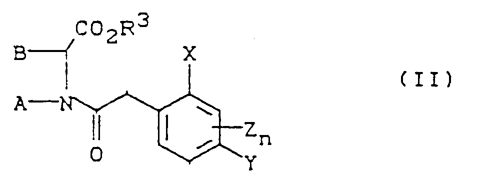 in which
A, B, X, Y, Z and n have the abovementioned meaning
and
R³ represents alkyl,
are subjected to an intramolecular condensation reaction in the presence of a diluent and in the presence of a base,
(B) or in that in order to obtain compounds of the formula (Ib) 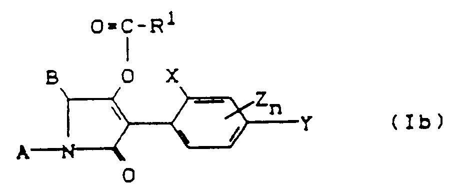 in which A, B, X, Y, Z, R¹ and n have the abovementioned meaning,
compounds of the formula (Ia) 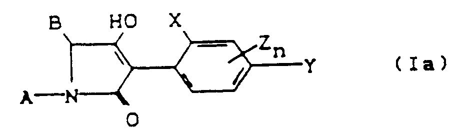 in which
A, B, X, Y, Z and n have the abovementioned meaning are reacted
α) with acid halides of the general formula (III) 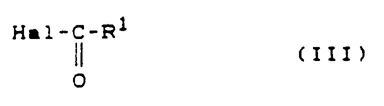 in which
R¹ has the abovementioned meaning
and
Hal represents halogen,
if appropriate in the presence of a diluent and if appropriate in the presence of an acid-binding agent,
or
β) with carboxylic anhydrides of the general formula (IV)
R¹-CO-O-CO-R¹ (IV)
in which
R¹ has the abovementioned meaning,
if appropriate in the presence of a diluent and if appropriate in the presence of an acid-binding agent,
(C) or in that in order to obtain compounds of the formula (Ic) 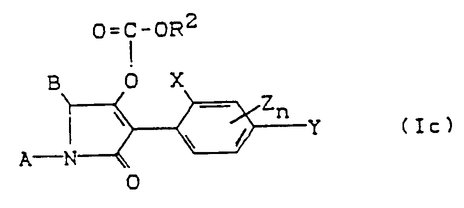 in which
A, B, X, Y, Z, R² and n have the abovementioned meaning,
compounds of the formula (Ia) 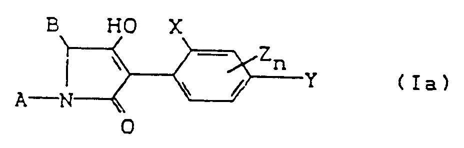 in which
A, B, X, Y, Z and n have the abovementioned meaning, are reacted with chloroformic esters of the general formula (V)
R²-O-CO-Cl (V)
in which
R² has the abovementioned meaning,
if appropriate in the presence of a diluent and if appropriate in the presence of an acid-binding agent.

5. Insecticidal, acaricidal and fungicidal agents, characterised in that they contain at least one substituted 3-aryl-pyrrolidine-2,4-dione derivative of the general formula (I) according to Claim 1.

6. Method of combating insects, arachnids and fungi, characterised in that substituted 3-aryl-pyrrolidine-2,4-dione derivatives of the general formula (I) according to Claim 1 are allowed to act on insects and/or arachnids and/or fungi and/or their habitat.

7. Use of substituted 3-aryl-pyrrolidine-2,4-dione derivatives of the formula (I) according to Claim 1 for combating insects and/or arachnids and/or fungi.

8. Process for the preparation of insecticidal, acaricidal and fungicidal agents, characterised in that 3-aryl-pyrrolidine-2,4-dione derivatives of the formula (I) according to Claim 1 are mixed with extenders and/or surface-active agents.

9. 3-Aryl-pyrrolidine-2,4-dione derivatives according to Claim 1 for combating mycoses.

10. 3-Aryl-pyrrolidine-2,4-dione derivatives according to Claim 1 comprising antimycotic agents.

11. Use of 3-aryl-pyrrolidine-2,4-dione derivatives according to Claim 1 for combating mycoses.

12. Use of 3-aryl-pyrrolidine-2,4-dione derivatives according to Claim 1 for the preparation of medicaments for combating mycoses.

## Claims (Claims for the following Contracting State(s): ES)

1. Process for the preparation of substituted 3-aryl-pyrrolidine-2,4-diones of the formula (I) 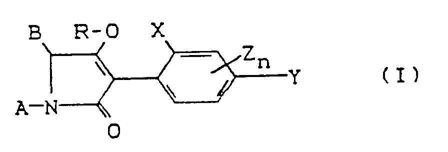 in which
X represents hydrogen, C₁-C₆-alkyl, halogen or C₁-C₆-alkoxy,
Y represents hydrogen, C₁-C₆-alkyl, halogen, C₁-C₆-alkoxy or C₁-C₃-halogenoalkyl,
Z represents C₁-C₆-alkyl, halogen or C₁-C₆-alkoxy,
n represents a number from 0-3,
R represents hydrogen (Ia) or the groups of the formula
-CO-R¹ (Ib) or
-CO-O-R² (Ic)
in which
R¹ represents optionally halogen-substituted C₁-C₂₀-alkyl, C₂-C₂₀-alkenyl, C₁-C₈-alkoxy-C₂-C₈-alkyl, C₁-C₈-alkylthio-C₂-C₈-alkyl, C₁-C₈-polyalkoxy-C₂-C₈-alkyl and cycloalkyl which has 3-8 ring atoms and which can be interrupted by oxygen and/or sulphur,
or represents phenyl which is optionally substituted by halogen, nitro, C₁-C₆-alkyl, C₁-C₆-alkoxy, C₁-C₆-halogenoalkyl or C₁-C₆-halogenoalkoxy;
or represents phenyl-C₁-C₆-alkyl which is optionally substituted by halogen, C₁-C₆-alkyl, C₁-C₆-alkoxy, C₁-C₆-halogenoalkyl or C₁-C₆-halogenoalkoxy,
or represents pyridyl, pyrimidyl, thiazolyl and pyrazolyl which are optionally substituted by fluorine, chlorine, bromine and methyl or ethyl,
or represents phenoxy-C₁-C₆-alkyl which is optionally substituted by halogen and C₁-C₆-alkyl,
or represents pyridyloxy-C₁-C₄-alkyl, pyrimidyloxy-C₁-C₄-alkyl and thiazolyloxy-C₁-C₄-alkyl which are optionally substituted by fluorine, chlorine, amino, methyl or ethyl,
R² represents optionally halogen-substituted C₁-C₂₀-alkyl, C₂-C₂₀-alkenyl, C₁-C₈-alkoxy-C₂-C₈-alkyl or C₁-C₈-polyalkoxy-C₂-C₈-alkyl,
or represents phenyl which is optionally substituted by halogen, nitro, C₁-C₆-alkyl, C₁-C₆-alkoxy or C₁-C₆-halogenoalkyl,
A represents optionally halogen-substituted straight-chain or branched C₁-C₁₂-alkyl, C₃-C₈-alkenyl, C₃-C₈-alkinyl, C₁-C₁₀-alkoxy-C₂-C₈-alkyl, C₁-C₈-polyalkoxy-C₂-C₈-alkyl, C₁-C₁₀-alkylthio-C₂-C₈-alkyl, cycloalkyl which has 3-8 ring atoms and which can be interrupted by oxygen and/or sulphur,
B represents phenyl or benzyl, each of which is optionally monosubstituted or disubstituted by identical or different substituents from the series comprising nitro, fluorine, chlorine, bromine or in each case optionally fluorine- or chlorine-substituted C₁-C₄-alkyl or C₁-C₄-alkoxy,
and the pure enantiomeric forms of compounds of the formula (I), characterised in that (A) in order to obtain substituted 3-phenyl-4-hydroxy-3-pyrrolin-2-ones or their diones of the formula (Ia) 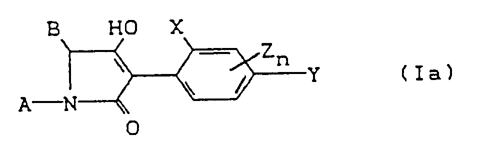 in which A, B, X, Y, Z and n have the abovementioned meaning,
N-acylamino acid esters of the formula (II) 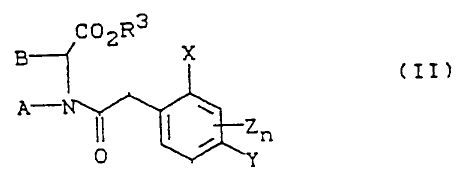 in which
A, B, X, Y, Z and n have the abovementioned meaning
and
R³ represents alkyl,
are subjected to an intramolecular condensation reaction in the presence of a diluent and in the presence of a base,
(B) or in that in order to obtain compounds of the formula (Ib) 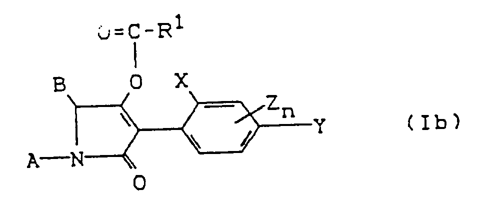 in which A, B, X, Y, Z, R¹ and n have the abovementioned meaning,
compounds of the formula (Ia) 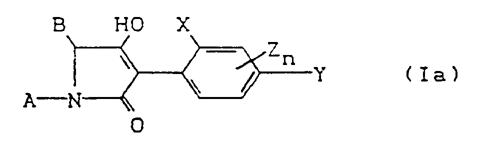 in which
A, B, X, Y, Z and n have the abovementioned meaning are reacted
α) with acid halides of the general formula (III) 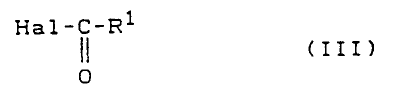 in which
R¹ has the abovementioned meaning
and
Hal represents halogen,
if appropriate in the presence of a diluent and if appropriate in the presence of an acid-binding agent,
or
β) with carboxylic anhydrides of the general formula (IV)
R¹-CO-O-CO-R¹ (IV)
in which
R¹ has the abovementioned meaning,
if appropriate in the presence of a diluent and if appropriate in the presence of an acid-binding agent,
(C) or in that in order to obtain compounds of the formula (Ic) 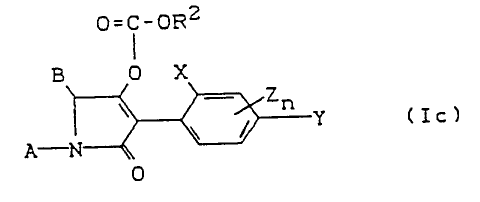 in which
A, B, X, Y, Z, R² and n have the abovementioned meaning,
compounds of the formula (Ia) 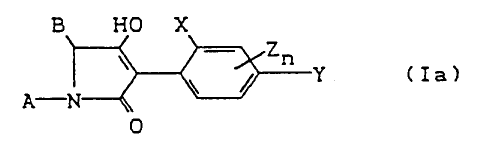 in which
A, B, X, Y, Z and n have the abovementioned meaning, are reacted with chloroformic esters of the general formula (V)
R²-O-CO-Cl (V)
in which
R² has the abovementioned meaning,
if appropriate in the presence of a diluent and if appropriate in the presence of an acid-binding agent.

2. Process according to Claim 1 for obtaining compounds of the formula (I), in which
X represents hydrogen, C₁-C₄-alkyl, halogen or C₁-C₄-alkoxy,
Y represents hydrogen, C₁-C₆-alkyl, halogen, C₁-C₄-alkoxy or C₁-C₂-halogenoalkyl,
Z represents C₁-C₄-alkyl, halogen or C₁-C₄-alkoxy,
n represents a number from 0-3,
R represents hydrogen (Ia) or the groups of the formula
-CO-R¹ (Ib) or
-CO-O-R² (Ic)
in which
R¹ represents optionally halogen-substituted C₁-C₁₆-alkyl, C₂-C₁₆-alkenyl, C₁-C₆-alkoxy-C₂-C₆-alkyl, C₁-C₆-alkylthio-C₂-C₆-alkyl, C₁-C₆-polyalkoxy-C₂-C₆-alkyl and cycloalkyl which has 3-7 ring atoms and which can be interrupted by 1-2 oxygen and/or sulphur atoms,
or represents phenyl which is optionally substituted by halogen, nitro, C₁-C₄-alkyl, C₁-C₄-alkoxy, C₁-C₃-halogenoalkyl or C₁-C₃-halogenoalkoxy,
or represents phenyl-C₁-C₄-alkyl which is optionally substituted by halogen, C₁-C₄-alkyl, C₁-C₄-alkoxy, C₁-C₃-halogenoalkyl or C₁-C₃-halogenoalkoxy,
or represents pyridyl, pyrimidyl, thiazolyl and pyrazolyl which are optionally substituted by fluorine, chlorine, bromine and methyl or ethyl,
or represents phenoxy-C₁-C₅-alkyl which is optionally substituted by halogen and C₁-C₄-alkyl,
or represents pyridyloxy-C₁-C₄-alkyl, pyrimidyloxy-C₁-C₄-alkyl and thiazolyloxy-C₁-C₄-alkyl which are optionally substituted by fluorine, chlorine, amino, methyl or ethyl,
R² represents optionally halogen-substituted C₁-C₁₆-alkyl, C₂-C₁₆-alkenyl, C₁-C₁₆-alkoxy-C₂-C₆-alkyl or C₁-C₆-polyalkoxy-C₂-C₆-alkyl,
or represents phenyl which is optionally substituted by halogen, nitro, C₁-C₄-alkyl, C₁-C₃-alkoxy or C₁-C₃-halogenoalkyl,
A represents optionally halogen-substituted straight-chain or branched C₁-C₁₀-alkyl, C₃-C₆-alkenyl, C₃-C₆-alkinyl, C₁-C₈-alkoxy-C₂-C₆-alkyl, C₁-C₆-polyalkoxy-C₂-C₆-alkyl, C₁-C₈-alkylthio-C₂-C₆-alkyl, cycloalkyl which has 3-7 ring atoms and which can be interrupted by 1-2 oxygen and/or sulphur atoms,
B represents phenyl or benzyl, each of which is optionally monosubstituted or disubstituted by identical or different substituents from the series comprising nitro, fluorine, chlorine, bromine or in each case optionally fluorine- or chlorine- substituted C₁-C₄-alkyl or C₁-C₄-alkoxy,
and the pure enantiomeric forms of compounds of the formula (I).

3. Insecticidal, acaricidal and fungicidal agents, characterised in that they contain at least one substituted 3-aryl-pyrrolidine-2,4-dione derivative of the general formula (I) according to Claim 1.

4. Method of combating insects, arachnids and fungi, characterised in that substituted 3-aryl-pyrrolidine-2,4-dione derivatives of the general formula (I) according to Claim 1 are allowed to act on insects and/or arachnids and/or fungi and/or their habitat.

5. Use of substituted 3-aryl-pyrrolidine-2,4-dione derivatives of the formula (I) according to Claim 1 for combating insects and/or arachnids and/or fungi.

6. Process for the preparation of insecticidal, acaricidal and fungicidal agents, characterised in that 3-aryl-pyrrolidine-2,4-dione derivatives of the formula (I) according to Claim 1 are mixed with extenders and/or surface-active agents.

7. Antimycotic agents comprising 3-aryl-pyrrolidine-2,4-dione derivatives of the general formula (I) according to Claim 1.

8. Use of 3-aryl-pyrrolidine-2,4-dione derivatives according to Claim 1 for combating mycoses.

9. Use of 3-aryl-pyrrolidine-2,4-dione derivatives according to Claim 1 for the preparation of medicaments for combating mycoses.

## Revendications (Revendications pour l'(les) Etat(s) contractant(s) suivant(s): BE, CH, DE, FR, GB, IT, LI, NL)

1. 3-aryl-pyrrolidine-2,4-diones substituées de formule générale (I) 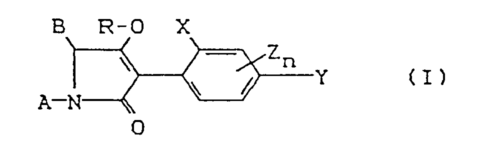 dans laquelle
X est de l'hydrogène, un groupe alkyle en C₁ à C₆, un halogène, un groupe alkoxy en C₁ à C₆,
Y est de l'hydrogène, un groupe alkyle en C₁ à C₆, un halogène, un groupe alkoxy en C₁ à C₆, un groupe halogénalkyle en C₁ à C₃,
Z est un groupe alkyle en C₁ à C₆, un halogène, un groupe alkoxy en C₁ à C₆,
n est un nombre de 0 à 3,
R représente de l'hydrogène (Ia) ou les groupes de formules
-CO-R¹ (Ib) ou
-CO-O-R² (Ic)
dans lesquelles
R¹ représente un groupe alkyle en C₁ à C₂₀ éventuellement substitué par un halogène, un groupe alcényle en C₂ à C₂₀, un groupe (alkoxy en C₁ à C₈)-(alkyle en C₂ à C₈), un groupe (alkylthio en C₁ à C₈)-(alkyle en C₂ à C₈), un groupe poly(alkoxy en C₁ à C₈)-(alkyle en C₂ à C₈), et un groupe cycloalkyle ayant 3 à 8 atomes de carbone dans le noyau, qui peut être interrompu par de l'oxygène et/ou du soufre,
un groupe phényle éventuellement substitué par un radical halogéno, nitro, alkyle en C₁ à C₆, alkoxy en C₁ à C₆, halogénalkyle en C₁ à C₆, halogénalkoxy en C₁ à C₆ ;
un groupe phényl-(alkyle en C₁ à C₆) éventuellement substitué par un radical halogéno, alkyle en C₁ à C₆, alkoxy en C₁ à C₆, halogénalkyle en C₁ à C₆, halogénalkoxy en C₁ à C₆,
un groupe pyridyle, pyrimidyle, thiazolyle ou pyrazolyle éventuellement substitué par du fluor, du chlore, du brome, un radical méthyle ou éthyle,
un groupe phénoxy (alkyle en C₁ à C₆) éventuellement substitué par un halogène et un radical alkyle en C₁ à C₆,
un groupe pyridyloxy-(alkyle en C₁ à C₄), pyrimidyloxy-(alkyle en C₁ à C₄), ou thiazolyloxy-(alkyle en C₁ à C₄) éventuellement substitué par du fluor, du chlore, un radical amino, méthyle ou éthyle,
R² est un groupe alkyle en C₁ à C₂₀ éventuellement substitué par un halogène, un groupe alcényle en C₂ à C₂₀, (alkoxy en C₁ à C₈)-(alkyle en C₂ à C₈), poly(alkoxy en C₁ à C₈)-(alkyle en C₂ à C₈), un groupe phényle éventuellement substitué par un radical halogéno, nitro, alkyle en C₁ à C₆, alkoxy en C₁ à C₆, halogénalkyle en C₁ à C₆,
A est un groupe alkyle en C₁ à C₁₀ linéaire ou ramifié éventuellement substitué par un halogène, un groupe alcényle en C₃ à C₈, alcynyle en C₃ à C₈, (alkoxy en C₁ à C₁₀)-(alkyle en C₂ à C₈), poly(alkoxy en C₁ à C₈)-(alkyle en C₂ à C₈), (alkylthio en C₁ à C₁₀)-(alkyle en C₂ à C₈), un groupe cycloalkyle à noyau à 3 à 8 atomes qui peut être interrompu par de l'oxygène et/ou du soufre,
B est un groupe phényle ou benzyle portant éventuellement un ou deux substituants, identiques ou différents, nitro, fluoro, chloro, bromo, alkyle en C₁ à C₄ ou alkoxy en C₁ à C₄ éventuellement substitué par du fluor ou du chlore, ainsi que les formes de pureté énantiomériques de composés de formule (I).

2. 3-aryl-pyrrolidine-2,4-diones substituées de formule (I) suivant la revendication 1, dans laquelle
X représente de l'hydrogène, un groupe alkyle en C₁ à C₄, un halogène, un groupe alkoxy en C₁ à C₄,
Y est de l'hydrogène, un groupe alkyle en C₁ à C₆, un halogène, un groupe alkoxy en C₁ à C₄, un groupe halogénalkyle en C₁ ou C₂,
Z est un groupe alkyle en C₁ à C₄, un halogène, un groupe alkoxy en C₁ à C₄,
n est un nombre de 0 à 3,
R représente de l'hydrogène (Ia) ou les groupes de formule
-CO-R¹ (Ib) ou
-CO-O-R² (Ic)
dans lesquelles
R¹ est un groupe alkyle en C₁ à C₁₆ éventuellement substitué par un halogène, un groupe alcényle en C₂ à C₆, (alkoxy en C₁ à C₆)-(alkyle en C₂ à C₆), (alkylthio en C₁ à C₆)-(alkyle en C₂ à C₆), poly(alkoxy en C₁ à C₆)-(alkyle en C₂ à C₆), et un groupe cycloalkyle à noyau de 3 à 7 atomes, qui peut être interrompu par 1-2 atomes d'oxygène et/ou de soufre, un groupe phényle éventuellement substitué par un radical halogéno, nitro, alkyle en C₁ à C₄, alkoxy en C₁ à C₄, halogénalkyle en C₁ à C₃, halogénalkoxy en C₁ à C₃,
un groupe phényl-(alkyle en C₁ à C₄) éventuellement substitué par un radical halogéno, alkyle en C₁ à C₄, alkoxy en C₁ à C₄, halogénalkyle en C₁ à C₃, halogénalkoxy en C₁ à C₃,
un groupe pyridyle, pyrimidyle, thiazolyle ou pyrazolyle éventuellement substitué par du fluor, du chlore, du brome et un radical méthyle ou éthyle,
un groupe phénoxy-(alkyle en C₁ à C₅) éventuellement substitué par un halogène ou un radical alkyle en C₁ à C₄,
un groupe pyridyloxy-(alkyle en C₁ à C₄), pyrimidyloxy-(alkyle en C₁ à C₄) ou thiazolyloxy-(alkyle en C₁ à C₄) éventuellement substitué par du fluor, du chlore, un radical amino, méthyle, éthyle,
R² est un groupe alkyle en C₁ à C₁₆ éventuellement substitué par un halogène, un groupe alcényle en C₂ à C₁₆, (alkoxy en C₁ à C₁₆)-(alkyle en C₂ à C₆), poly(alkoxy en C₁ à C₆)-(alkyle en C₂ à C₆), un groupe phényle éventuellement substitué par un halogène, un radical nitro, alkyle en C₁ à C₄, alkoxy en C₁ à C₃, halogénalkyle en C₁ à C₃,
A représente un groupe alkyle à C₁ à C₁₀ linéaire ou ramifié éventuellement substitué par un halogène, un groupe alcényle en C₃ à C₆, alcynyle en C₃ à C₆, (alkoxy en C₁ à C₈)-(alkyle en C₂ à C₆), poly(alkoxy en C₁ à C₆)-(alkyle en C₂ à C₆), (alkylthio en C₁ à C₈)-(alkyle en C₂ à C₆), cycloalkyle à noyau de 3 à 7 atomes qui peut être interrompu par 1 ou 2 atomes d'oxygène et/ou de soufre,
B représente un groupe phényle ou benzyle portant le cas échéant un ou deux substituants, identiques ou différents, nitro, fluoro, chloro, bromo, alkyle en C₁ à C₄ ou alkoxy en C₁ à C₄ éventuellement substitué par du fluor ou du chlore,
ainsi que les formes de pureté énantiomérique de composés de formule (I).

3. 3-aryl-pyrrolidine-2,4-diones substituées de formule (I) suivant la revendication 1, dans laquelle
X représente de l'hydrogène, les radicaux méthyle, éthyle, propyle, isopropyle, fluoro, chloro, bromo, méthoxy et éthoxy,
Y représente de l'hydrogène, les radicaux méthyle, éthyle, propyle, isopropyle, butyle, isobutyle, tertio-butyle, fluoro, chloro, bromo, méthoxy, éthoxy et trifluorométhyle,
Z représente les radicaux méthyle, éthyle, isopropyle, butyle, isobutyle, tertio-butyle, fluoro, chloro, bromo, méthoxy et éthoxy,
n est un nombre de 0 à 3,
R représente de l'hydrogène (Ia) ou les groupes de formule
-CO-R¹ (Ib) ou
-CO-O-R² (Ic)
dans laquelle
R¹ est un groupe alkyle en C₁ à C₁₄ éventuellement substitué par du fluor ou du chlore, un groupe alcényle en C₂ à C₁₄, (alkoxy en C₁ à C₄)-(alkyle en C₂ à C₆), (alkylthio en C₁ à C₄)-(alkyle en C₂ à C₆), poly(alkoxy en C₁ à C₄)-(alkyle en C₂ à C₄) et un groupe cycloalkyle à noyau de 3 à 6 atomes qui peut être interrompu par 1 ou 2 atomes d'oxygène et/ou de soufre,
un groupe phényle éventuellement substitué par un radical fluoro, chloro, bromo, méthyle, éthyle, propyle, isopropyle, méthoxy, éthoxy, trifluorométhyle, trifluorométhoxy, nitro,
un groupe phényl-(alkyle en C₁ à C₃) éventuellement substitué par un radical fluoro, chloro, bromo, méthyle, éthyle, propyle, isopropyle, méthoxy, éthoxy, trifluorométhyle, trifluorométhoxy,
un groupe pyridyle, pyrimidyle, thiazolyle ou pyrazolyle éventuellement substitué par un radical fluoro, chloro, bromo, méthyle, éthyle,
un groupe phénoxy (alkyle en C₁ à C₄) éventuellement substitué par un radical fluoro, chloro, méthyle, éthyle,
un groupe pyridyloxy-(alkyle en C₁ à C₄), pyrimidyloxy-(alkyle en C₁ à C₄) ou thiazolyloxy-(alkyle en C₁ à C₄) éventuellement substitué par un radical fluoro, chloro, amino, méthyle, éthyle,
R² est un groupe alkyle en C₁ à C₁₄ éventuellement substitué par du fluor ou du chlore, un groupe alcényle en C₁ à C₁₄, (alkoxy en C₁ à C₄)-(alkyle en C₂ à C₆), poly(alkoxy en C₁ à C₄)-(alkyle en C₂ à C₆),
ou bien un groupe phényle substitué le cas échéant par un radical fluoro, chloro, nitro, méthyle, éthyle, propyle, isopropyle, méthoxy, éthoxy, trifluorométhyle,
A est un groupe alkyle en C₁ à C₈ linéaire ou ramifié éventuellement substitué par un halogène, un groupe alcényle en C₃ ou C₄, alcynyle en C₃ ou C₄, (alkoxy en C₁ à C₆)-(alkyle en C₂ à C₄), poly(alkoxy en C₁ à C₄)-(alkyle en C₂ à C₄), (alkylthio en C₁ à C₆)-(alkyle en C₂ à C₄), cycloalkyle à noyau de 3 à 6 atomes qui peut être interrompu par 1-2 atomes d'oxygène et/ou de soufre,
B est un groupe phényle ou benzyle non substitué ou un groupe phényle ou benzyle substitué par un radical nitro, fluoro, chloro, alkyle en C₁-C₂ ou alkoxy en C₁-C₂ éventuellement substitué par du fluor ou du chlore,
ainsi que les formes de pureté énantiomérique de composés de formule I.

4. Procédé de production de 3-aryl-pyrrolidine-2,4-diones substituées de formule (I) suivant la revendication 1, caractérisé en ce que (A) pour obtenir des 3-phényl-4-hydroxy-3-pyrroline-2-ones substituées ou leurs diones de formule (Ia) 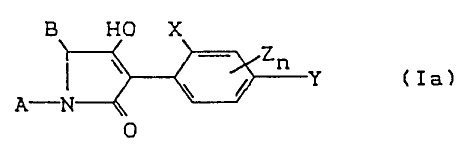 dans laquelle A, B, X, Y, Z et n ont la définition indiquée ci-dessus,
On effectue la condensation intramoléculaire d'un ester de N-acylaminoacide de formule (II) 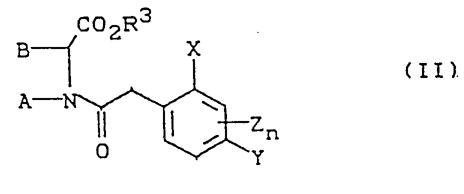 dans laquelle
A, B, X, Y, Z et n ont la définition indiquée ci-dessus, et
R³ est un groupe alkyle,
en présence d'un diluant et en présence d'une base,
ou bien
(B) pour obtenir des composés de formule (Ib) 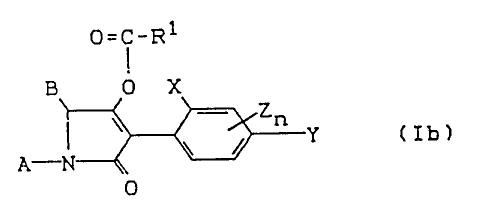 dans laquelle
A, B, X, Y, Z, R¹ et n ont la définition indiquée ci-dessus,
on fait réagir des composés de formule (Ia), 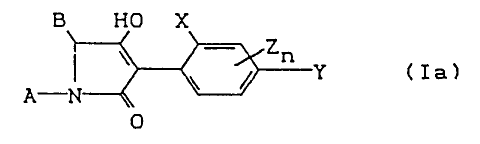 dans laquelle
A, B, X, Y, Z et n ont la définition indiquée ci-dessus,
α) avec des halogénures d'acides de formule générale (III) 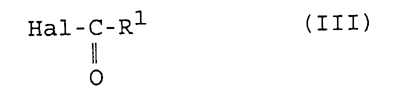 dans laquelle
R¹ a la définition indiquée ci-dessus et
Hal représente un halogène,
le cas échéant en présence d'un diluant et en la présence éventuelle d'un accepteur d'acide, ou bien
β) avec des anhydrides d'acide carboxylique de formule générale (IV)
R¹-CO-O-CO-R¹ (IV)
dans laquelle
R¹ a la définition indiquée ci-dessus, le cas échéant en présence d'un diluant et en la présence éventuelle d'un accepteur d'acide,
ou bien
(C) pour obtenir des composés de formule (Ic) 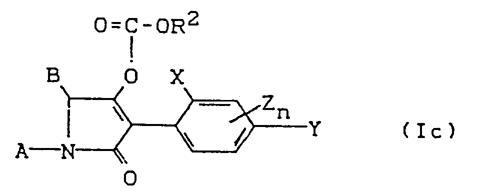 dans laquelle
A, B, X, Y, Z, R₂ et n ont la définition indiquée ci-dessus,
On fait réagir des composés de formule (Ia) 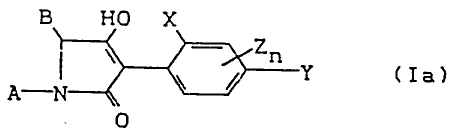 dans laquelle
A, B, X, Y, Z et n ont la définition indiquée ci-dessus, avec des esters d'acides chloroformiques de formule générale (V)
R²-O-CO-Cl (V)
dans laquelle
R² a la définition indiquée ci-dessus,
le cas échéant en présence d'un diluant et en la présence éventuelle d'un accepteur d'acide.

5. Compositions insecticides, acaricides et fongicides, caractérisées par une teneur en au moins un dérivé substitué de 3-aryl-pyrrolidine-2,4-diones de formule générale (I) suivant la revendication 1.

6. Procédé pour combattre des insectes, des acariens et des champignons, caractérisé en ce qu'on fait agir sur les insectes et/ou les acariens et/ou les champignons et/ou sur leur milieu, des dérivés substitués de 3-aryl-pyrroline-2,4-diones de formule générale (I) suivant la revendication 1.

7. Utilisation de dérivés substitués de 3-aryl-pyrrolidine-2,4-diones de formule (I) suivant la revendication 1 pour combattre des insectes et/ou des acariens et/ou des champignons.

8. Procédé de préparation de compositions insecticides, acaricides et fongicides, caractérisé en ce qu'on mélange des dérivés de 3-aryl-pyrrolidine-4-diones de formule (I) suivant la revendication 1 avec des diluants et/ou des agents tensio-actifs.

9. Dérivés de 3-aryl-pyrrolidine-2,4-diones suivant la revendication 1, destinés à combattre des mycoses.

10. Compositions antimycosiques contenant des dérivés de 3-aryl-pyrrolidine-2,4-diones suivant la revendication 1.

11. Utilisation de dérivés de 3-aryl-pyrrolidine-2,4-diones suivant la revendication 1 pour combattre des mycoses.

12. Utilisation de dérivés de 3-aryl-pyrrolidine-2,4-diones suivant la revendication 1 dans la préparation de médicaments destinés à combattre des mycoses.

## Revendications (Revendications pour l'(les) Etat(s) contractant(s) suivant(s): ES)

1. Procédé de production de 3-aryl-pyrrolidine-2,4-diones substituées de formule (I) 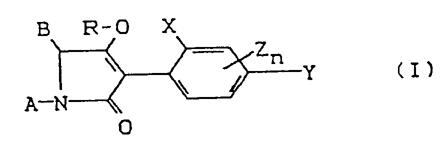 dans laquelle
X est de l'hydrogène, un groupe alkyle en C₁ à C₆, un halogène, un groupe alkoxy en C₁ à C₆,
Y est de l'hydrogène, un groupe alkyle en C₁ à C₆, un halogène, un groupe alkoxy en C₁ à C₆, un groupe halogénalkyle en C₁ à C₃,
Z est un groupe alkyle en C₁ à C₆, un halogène, un groupe alkoxy en C₁ à C₆,
n est un nombre de 0 à 3,
R représente de l'hydrogène (Ia) ou les groupes de formules
-CO-R¹ (Ib) ou
-CO-O-R² (Ic)
dans lesquelles
R¹ représente un groupe alkyle en C₁ à C₂₀ éventuellement substitué par un halogène, un groupe alcényle en C₂ à C₂₀, un groupe (alkoxy en C₁ à C₈)-(alkyle en C₂ à C₈), un groupe (alkylthio en C₁ à C₈)-(alkyle en C₂ à C₈), un groupe poly(alkoxy en C₁ à C₈)-(alkyle en C₂ à C₈), et un groupe cycloalkyle ayant 3 à 8 atomes de carbone dans le noyau, qui peut être interrompu par de l'oxygène et/ou du soufre,
un groupe phényle éventuellement substitué par un radical halogéno, nitro, alkyle en C₁ à C₆, alkoxy en C₁ à C₆, halogénalkyle en C₁ à C₆, halogénalkoxy en C₁ à C₆ ;
un groupe phényl-(alkyle en C₁ à C₆) éventuellement substitué par un radical halogéno, alkyle en C₁ à C₆, alkoxy en C₁ à C₆, halogénalkyle en C₁ à C₆, halogénalkoxy en C₁ à C₆,
un groupe pyridyle, pyrimidyle, thiazolyle ou pyrazolyle éventuellement substitué par du fluor, du chlore, du brome, un radical méthyle ou éthyle, un groupe phénoxy (alkyle en C₁ à C₆) éventuellement substitué par un halogène et un radical alkyle en C₁ à C₆,
un groupe pyridyloxy-(alkyle en C₁ à C₄), pyrimidyloxy-(alkyle en C₁ à C₄), ou thiazolyloxy-(alkyle en C₁ à C₄) éventuellement substitué par du fluor, du chlore, un radical amino, méthyle ou éthyle,
R² est un groupe alkyle en C₁ à C₂₀ éventuellement substitué par un halogène, un groupe alcényle en C₂ à C₂₀, (alkoxy en C₁ à C₈)-(alkyle en C₂ à C₈), poly(alkoxy en C₁ à C₈)-(alkyle en C₂ à C₈), un groupe phényle éventuellement substitué par un radical halogéno, nitro, alkyle en C₁ à C₆, alkoxy en C₁ à C₆, halogénalkyle en C₁ à C₆,
A est un groupe alkyle en C₁ à C₁₂ linéaire ou ramifié éventuellement substitué par un halogène, un groupe alcényle en C₃ à C₈, alcynyle en C₃ à C₈, (alkoxy en C₁ à C₁₀)-(alkyle en C₂ à C₈), poly(alkoxy en C₁ à C₈)-(alkyle en C₂ à C₈), (alkylthio en C₁ à C₁₀)-(alkyle en C₂ à C₈), un groupe cycloalkyle à noyau à 3 à 8 atomes qui peut être interrompu par de l'oxygène et/ou du soufre,
B est un groupe phényle ou benzyle portant éventuellement un ou deux substituants, identiques ou différents, nitro, fluoro, chloro, bromo, alkyle en C₁ à C₄ ou alkoxy en C₁ à C₄ éventuellement substitué par du fluor ou du chlore, ainsi que les formes de pureté énantiomériques de composés de formule (I)
caractérisé en ce que (A) pour obtenir des 3-aryl-pyrrolidine-2,4-diones substituées ou leurs énols de formule (Ia) 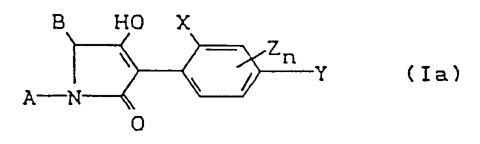 dans laquelle A, B, X, Y, Z et n ont la définition indiquée ci-dessus,
On effectue la condensation intramoléculaire d'un ester de N-acylaminoacide de formule (II) 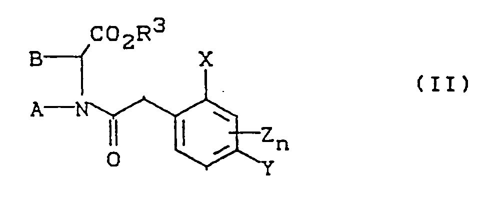 dans laquelle
A, B, X, Y, Z et n ont la définition indiquée ci-dessus, et
R³ est un groupe alkyle,
en présence d'un diluant et en présence d'une base, ou bien
(B) pour obtenir des composés de formule (Ib) 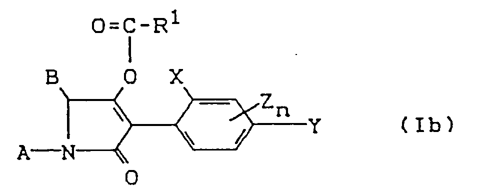 dans laquelle
A, B, X, Y, Z, R¹ et n ont la définition indiquée ci-dessus,
on fait réagir des composés de formule (Ia), 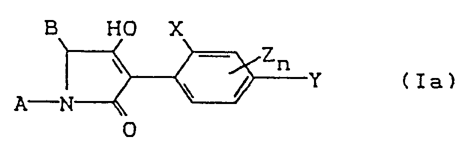 dans laquelle
A, B, X, Y, Z et n ont la définition indiquée ci-dessus,
α) avec des halogénures d'acides de formule générale (III) 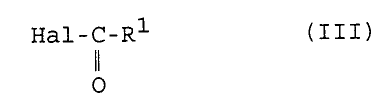 dans laquelle
R¹ a la définition indiquée ci-dessus
et
Hal représente un halogène,
le cas échéant en présence d'un diluant et en la présence éventuelle d'un accepteur d'acide, ou bien
β) avec des anhydrides d'acide carboxylique de formule générale (IV)
R¹-CO-O-CO-R¹ (IV)
dans laquelle
R¹ a la définition indiquée ci-dessus, le cas échéant en présence d'un diluant et en la présence éventuelle d'un accepteur d'acide,
ou bien
(C) pour obtenir des composés de formule (Ic) 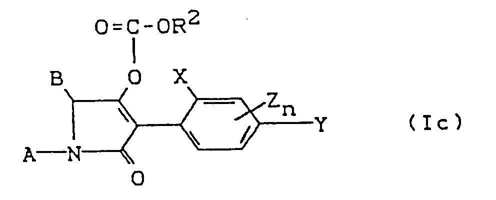 dans laquelle
A, B, X, Y, Z, R₂ et n ont la définition indiquée ci-dessus,
On fait réagir des composés de formule (Ia) 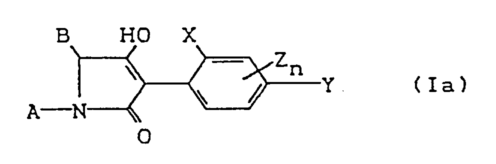 dans laquelle
A, B, X, Y, Z et n ont la définition indiquée ci-dessus, avec des esters d'acides chloroformiques de formule générale (V)
R²-O-CO-Cl (V)
dans laquelle
R² a la définition indiquée ci-dessus,
le cas échéant en présence d'un diluant et en la présence éventuelle d'un accepteur d'acide.

2. Procédé suivant la revendication 1, pour l'obtention de composés de formule (I) dans laquelle
X représente de l'hydrogène, un groupe alkyle en C₁ à C₄, un halogène, un groupe alkoxy en C₁ à C₄,
Y est de l'hydrogène, un groupe alkyle en C₁ à C₆, un halogène, un groupe alkoxy en C₁ à C₄, un groupe halogénalkyle en C₁ ou C₂,
Z est un groupe alkyle en C₁ à C₄, un halogène, un groupe alkoxy en C₁ à C₄,
n est un nombre de 0 à 3,
R représente de l'hydrogène (Ia) ou les groupes de formule
-CO-R¹ (Ib) ou
-CO-O-R² (Ic)
dans lesquelles
R¹ est un groupe alkyle en C₁ à C₁₆ éventuellement substitué par un halogène, un groupe alcényle en C₂ à C₁₆, (alkoxy en C₁ à C₆)-(alkyle en C₂ à C₆), (alkylthio en C₁ à C₆)-(alkyle en C₂ à C₆), poly(alkoxy en C₁ à C₆)-(alkyle en C₂ à C₆), et un groupe cycloalkyle à noyau de 3 à 7 atomes, qui peut être interrompu par 1-2 atomes d'oxygène et/ou de soufre, un groupe phényle éventuellement substitué par un radical halogéno, nitro, alkyle en C₁ à C₄, alkoxy en C₁ à C₄, halogénalkyle en C₁ à C₃, halogénalkoxy en C₁ à C₃,
un groupe phényl-(alkyle en C₁ à C₄) éventuellement substitué par un radical halogéno, alkyle en C₁ à C₄, alkoxy en C₁ à C₄, halogénalkyle en C₁ à C₃, halogénalkoxy en C₁ à C₃,
un groupe pyridyle, pyrimidyle, thiazolyle ou pyrazolyle éventuellement substitué par du fluor, du chlore, du brome et un radical méthyle ou éthyle, un groupe phénoxy-(alkyle en C₁ à C₅) éventuellement substitué par un halogène ou un radical alkyle en C₁ à C₄,
un groupe pyridyloxy-(alkyle en C₁ à C₄), pyrimidyloxy-(alkyle en C₁ à C₄) ou thiazolyloxy-(alkyle en C₁ à C₄) éventuellement substitué par du fluor, du chlore, un radical amino, méthyle, éthyle,
R² est un groupe alkyle en C₁ à C₁₆ éventuellement substitué par un halogène, un groupe alcényle en C₂ à C₁₆, (alkoxy en C₁ à C₁₆)-(alkyle en C₂ à C₆), poly(alkoxy en C₁ à C₆)-(alkyle en C₂ à C₆), un groupe phényle éventuellement substitué par un halogène, un radical nitro, alkyle en C₁ à C₄, alkoxy en C₁ à C₃, halogénalkyle en C₁ à C₃,
A représente un groupe alkyle à C₁ à C₁₀ linéaire ou ramifié éventuellement substitué par un halogène, un groupe alcényle en C₃ à C₆, alcynyle en C₃ à C₆, (alkoxy en C₁ à C₈)-(alkyle en C₂ à C₆), poly(alkoxy en C₁ à C₆)-(alkyle en C₂ à C₆), (alkylthio en C₁ à C₈)-(alkyle en C₂ à C₆), cycloalkyle à noyau de 3 à 7 atomes qui peut être interrompu par 1 ou 2 atomes d'oxygène et/ou de soufre,
B représente un groupe phényle ou benzyle portant le cas échéant un ou deux substituants, identiques ou différents, nitro, fluoro, chloro, bromo, alkyle en C₁ à C₄ ou alkoxy en C₁ à C₄ éventuellement substitué par du fluor ou du chlore,
ainsi que les formes de pureté énantiomérique de composés de formule (I).

3. Compositions insecticides, acaricides et fongicides, caractérisées par une teneur en au moins un dérivé substitué de 3-aryl-pyrrolidine-2,4-diones de formule générale (I) suivant la revendication 1.

4. Procédé pour combattre des insectes, des acariens et des champignons, caractérisé en ce qu'on fait agir sur les insectes et/ou les acariens et/ou les champignons et/ou sur leur milieu, des dérivés substitués de 3-aryl-pyrroline-2,4-diones de formule générale (I) suivant la revendication 1.

5. Utilisation de dérivés substitués de 3-aryl-pyrrolidine-2,4-diones de formule (I) suivant la revendication 1 pour combattre des insectes et/ou des acariens et/ou des champignons.

6. Procédé de préparation de compositions insecticides, acaricides et fongicides, caractérisé en ce qu'on mélange des dérivés de 3-aryl-pyrrolidine-4-diones de formule (I) suivant la revendication 1 avec des diluants et/ou des agents tensio-actifs.

7. Compositions anti-mycosiques, contenant des dérivés de 3-aryl-pyrrolidine-2,4-diones de formule (I) suivant la revendication 1.

8. Utilisation de dérivés de 3-aryl-pyrrolidine-2,4-diones suivant la revendication 1 pour combattre des mycoses.

9. Utilisation de dérivés de 3-aryl-pyrrolidine-2,4-diones suivant la revendication 1 dans la préparation de médicaments destinés à combattre des mycoses.
